# EUROPEAN PATENT APPLICATION

(11) **EP 3 495 354 A1**
(43) Date of publication of application: **12.06.2019**
(21) Application number: 17836390.9
(22) Date of filing: 02.08.2017
(51) Int. Cl.: C07D 271/08, C07D 413/12, A61K 31/4245, A61P 25/00, A61P 27/12, A61P 37/00

(54) **IDO1 INHIBITOR AND PREPARATION METHOD AND APPLICATION THEREOF**

(30) Priority: 02.08.2016 CN 201610631523; 30.08.2016 CN 201610797622
(71) Applicant: Shandong Luye Pharmaceutical Co., Ltd., Shandong 264670 (CN)
(72) Inventor: ZHANG, Yang, Shanghai 200131 (CN); FU, Zhifei, Shanghai 200131 (CN); LUO, Miaorong, Shanghai 200131 (CN); LI, Jian, Shanghai 200131 (CN); CHEN, Shuhui, Shanghai 200131 (CN)
(74) Representative: Rössler, Matthias
(86) International application number: PCT/CN2017/095571
(87) International publication number: WO 2018/024208

(57) **Abstract**

A compound as an indoleamine-2,3-dioxygenase 1 (IDO1) inhibitor, and an application thereof in the field of IDO1-related diseases, and in particular a compound as shown in formula (I) and a pharmaceutically acceptable salts thereof.

## Description

### Technical Field

The present disclosure relates to a class of compounds as indoleamine-2,3-dioxygenase 1 (IDO1) inhibitors, and use thereof in the field of IDO1-related diseases, particularly to a compound represented by formula (I) and a pharmaceutically acceptable salt thereof.

### Background Art

Indoleamine-2,3-dioxygenase (IDO) is a ferroheme-containing monomeric enzyme first discovered by Hayaishi team in cells in 1967, with cDNA encoded protein of 403 amino acids with a molecular weight of 455 kDa. It is a rate-limiting enzyme of catabolism in a tryptophan-kynurenine pathway, and is widely expressed in tissues of a variety of mammals (Hayaishi O. et al., Science, 1969, 164, 389-396). In cells of tumor patients, IDO usually plays an important physiological role in inducing immune tolerance in tumor microenvironment. A tryptophan (Trp)-kynurenine (Kyn) metabolic pathway mediated by IDO takes part in tumor immune escape. IDO also plays an important role in inducing immune tolerance in tumor microenvironment.

Tryptophan (Trp) is an essential amino acid required by biosynthetic proteins, niacin, and neurotransmitter 5-hydroxytryptamine (serotonin). In recent years, immunoregulatory effect of Trp depletion attracts a lot of attention. IDO degrades the indole moieties of tryptophan, 5-hydroxytryptamine and melatonin, inducing generation of neuroactive and immunoregulatory metabolites collectively referred to as kynurenine. By partially consuming tryptophan and increasing pro-apoptotic kynurenine, IDO expressed by dendritic cell (DC) may greatly affect proliferation and survival of T cells. Induced expression of IDO in DC may be a general mechanism underlying consumption tolerance driven by regulatory T cells. Because it is contemplated that such type of tolerogenic reaction plays a role in a variety of physiological and pathological diseases, tryptophan metabolism and kynurenine generation may represent a key interface between immune and nervous systems (Grohmann et al., 2003, Trends Immunol., 24: 242-8). In a state of continuous immune activation, available free serum Trp is decreased, and as production of 5-hydroxytryptamine is decreased, the functions of 5-hydroxytryptamine also may be affected (Wirleitner et al., 2003, Curr. Med. Chem., 10: 1581-91).

IDO inhibitors for treating or preventing IDO-related diseases are under development. Facing a huge unfulfilled market, there is a need in the art for an IDO inhibitor with better activity so as to satisfy requirement for treatment.

### Summary

The present disclosure provides a compound represented by formula (I) or a pharmaceutically acceptable salt thereof, 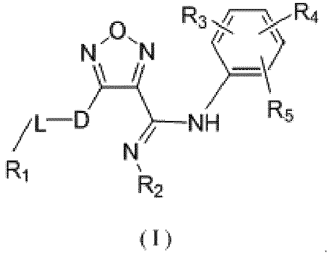
Wherein, D is O, S or -S(=O)-;
L is a single bond, or selected from -C₁₋₁₀ alkyl-, -C₃₋₆ cycloalkyl-, -C₃₋₆ cycloalkyl-C₁₋₃ alkyl-, -phenyl-, -3- to 6-membered heterocycloalkyl-, or -3- to 6-membered heterocycloalkyl-C₁₋₃ alkyl- groups, one or more of which are optionally substituted by 1, 2, or 3 R groups;
R₁ is selected from the group consisting of H, F, Cl, Br, I, OH, and NH₂, or selected from the group consisting of C₁₋₆ alkyl group, C₃₋₆ cycloalkyl group, C₁₋₆ heteroalkyl group, *N,N*-bis(C₁₋₆ alkyl)amino group, 3- to 6-membered heterocycloalkyl group, C₂₋₆ alkenyl, phenyl group, 5- to 9-membered heteroaryl group, 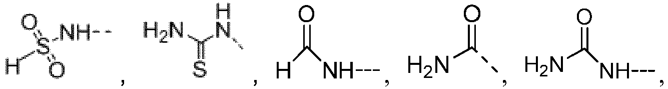 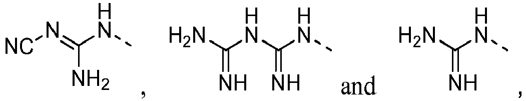 one or more of which are optionally substituted by 1, 2, or 3 R groups;
R₂ is OH or CN;
R₃, R₄, and R₅ are each independently selected from the group consisting of H, F, Cl, Br, I, OH, CN, and NH₂, or selected from the group consisting of C₁₋₆ alkyl, C₃₋₆ cycloalkyl, C₁₋₆ heteroalkyl, *N,N*-bis(C₁₋₆ alkyl)amino, and 3- to 6-membered heterocycloalkyl groups, one or more of which are optionally substituted by 1, 2, or 3 R groups;
R is selected from the group consisting of H, F, Cl, Br, I, OH, CN, and NH₂, or selected from the group consisting of C₁₋₆ alkyl, C₁₋₆ heteroalkyl, *N,N*-bis(C₁₋₆ alkyl)amino, C₃₋₆ cycloalkyl, C₂₋₆ alkenyl, phenyl, and thienyl groups, one or more of which are optionally substituted by 1, 2, or 3 R' groups;
R' is selected from the group consisting of F, Cl, Br, I, OH, CN, and NH₂;
the "hetero" moieties in the -3- to 6-membered heterocycloalkyl, the -3- to 6-membered heterocycloalkyl-C₁₋₃ alkyl-, the C₁₋₆ heteroalkyl, the 3- to 6-membered heterocycloalkyl, or the 5- to 9-membered heteroaryl groups are each independently selected from -C(=O)NH-, -NH-, -S(=O)₂NH-, -S(=O)NH-, N, -O-, -S-, =O, =S, -C(=O)O-, -C(=O)-, -C(=S)-, -S(=O)-, -S(=O)₂-, -NHC(=O)NH-, -NHC(=S)NH-, and -H₂P(=O)-NH-;
in any of above cases, the number of heteroatom or heteroatom group is each independently selected from 1, 2 or 3.

In some embodiments of the present disclosure, the above-mentioned R is selected from the group consisting of H, F, Cl, Br, I, OH, CN, and NH₂, or selected from the group consisting of C₁₋₆ alkyl, C₁₋₆ alkoxy, C₁₋₆ alkylamino, *N,N*-bis(C₁₋₆ alkyl)amino, C₂₋₆ alkenyl, C₃₋₆ cycloalkyl, phenyl, and thienyl groups, optionally one or more of which are substituted by 1, 2, or 3 R' groups.

In some embodiments of the present disclosure, the above-mentioned R is selected from the group consisting of H, F, Cl, Br, I, OH, CN, and NH₂, or selected from Me, Et, 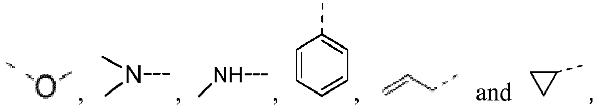 one or more of which are optionally substituted by 1, 2, or 3 R' groups.

In some embodiments of the present disclosure, the above-mentioned R is selected form H, F, Cl, Br, I, OH, CN, NH₂, Me, Et, CF₃, CHF₂, CH₂F, 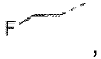 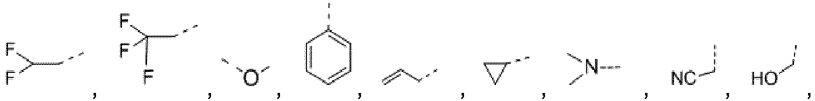 and 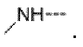 .

In some embodiments of the present disclosure, the above-mentioned L is selected from a single bond, or selected from -C₁₋₅ alkyl-, -C₃₋₆ cycloalkyl-, -C₃₋₆ cycloalkyl-C₁₋₃ alkyl-, and -3- to 6-membered azacycloalkyl-C₁₋₃ alkyl-, one or more of which are optionally substituted by 1, 2 or 3 R groups.

In some embodiments of the present disclosure, the above-mentioned L is selected from the group consisting of a single bond, or selected from -CH₂-, 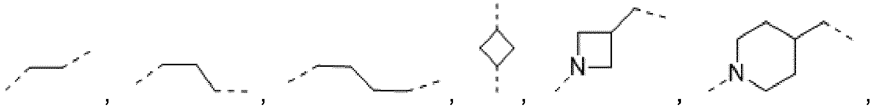 and 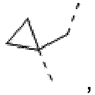 one or more of which are optionally substituted by 1, 2 or 3 R groups.

In some embodiments of the present disclosure, the above-mentioned L is selected from the group consisting of a single bond, -CH₂-,

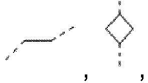

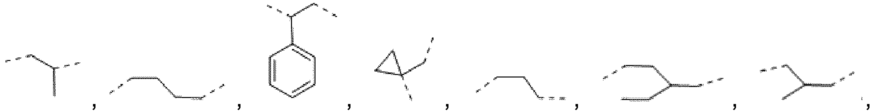

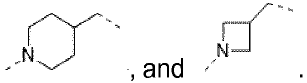

In some embodiments of the present disclosure, the above-mentioned R₁ is selected from the group consisting of H, F, Cl, Br, I, OH, and NH₂, or selected from the group consisting of C₁₋₆ alkyl, C₁₋₆ alkoxy, C₁₋₆ alkylthio, C₁₋₆ alkylamino, *N,N*'-bis(C₁₋₃ alkyl) amino, C₃₋₆ cycloalkyl, tetrahydrofuryl, oxetanyl, C₂₋₆ alkenyl, phenyl, thienyl, pyridyl, imidazolyl, thiazolyl, 2-oxo-imidazolidinyl, NH₂C(=S)-NH-, C₁₋₆ alkyl S(=O)-, C₁₋₆ alkyl-S(=O)₂-, C₁₋₆ alkoxy-C(=O)-NH-, NH₂-S(=O)-NH-, -NH₂-C(=O)-, NH₂-C(=O)-NH-, H-C(=O)-NH-, H-S(=O)₂-NH-, 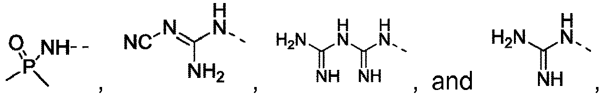 one or more of which are optionally substituted by 1, 2 or 3 R groups.

In some embodiments of the present disclosure, the above-mentioned R₁ is selected from H, F, Cl, Br, I, OH, and NH₂, or selected from Me, Et, BOC-NH-, CH₂ = CH-, 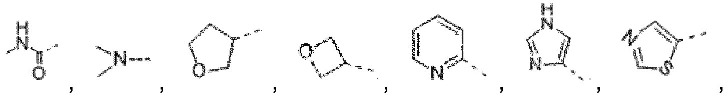 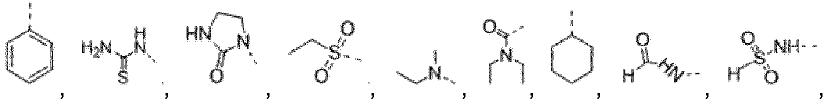 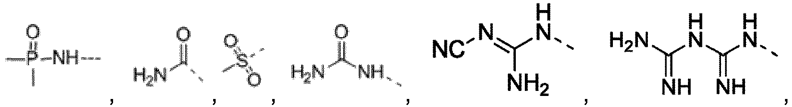 and 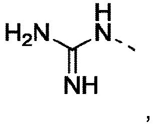 optionally one or more of which are substituted by 1, 2 or 3 R groups:.

In some embodiments of the present disclosure, the above-mentioned R₁ is selected from the group consisting of H, F, Cl, Br, I, OH, NH₂, Me, CF₃, BOC-NH-, CH₂=CH-,

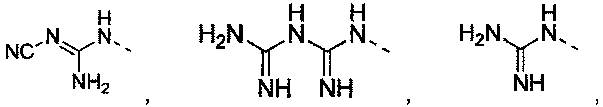

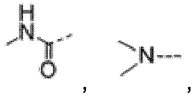

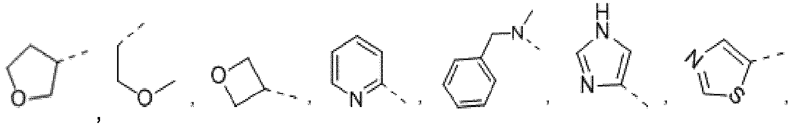

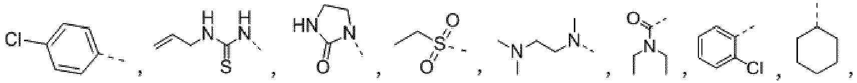

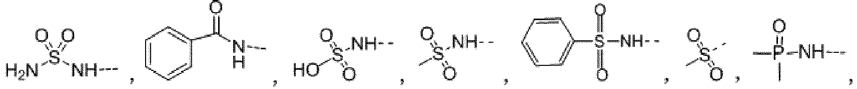

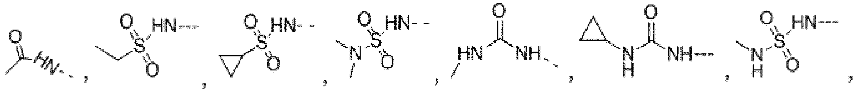

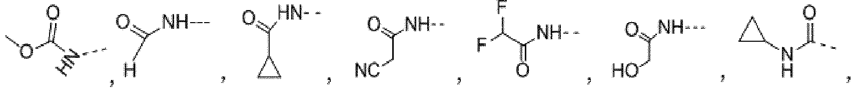

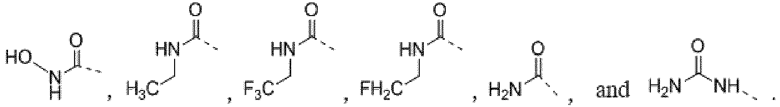

In some embodiments of the present disclosure, the above structural unit R₁-L- is H. NH₂. Me, BOC-NH-, 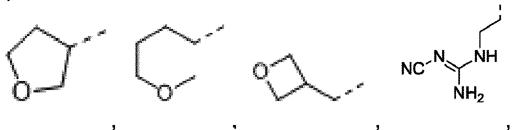 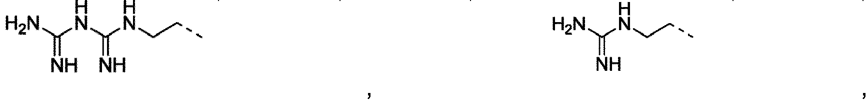 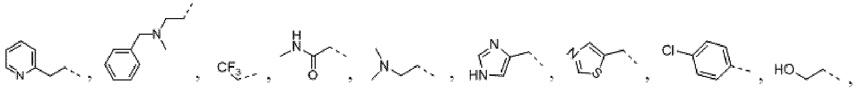 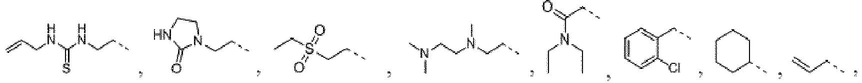 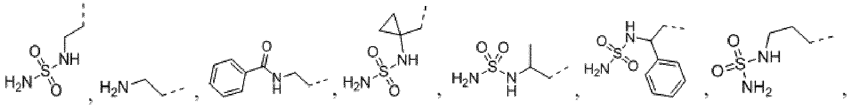 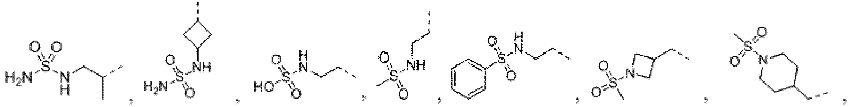 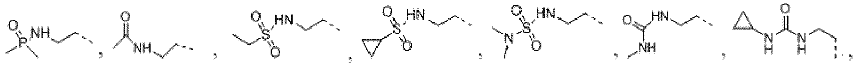 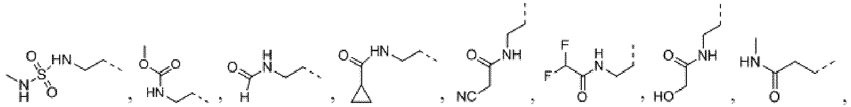 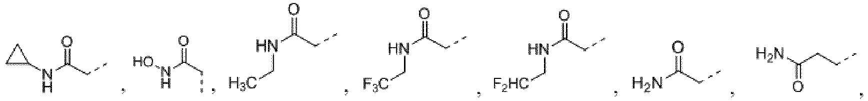 and

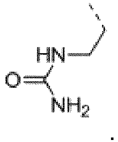

In some embodiments of the present disclosure, the above-mentioned R₃, R₄, and R₅ are each independently selected from H, F, Cl, Br, I, OH, CN, and NH₂, or selected from Me, Et, C₃₋₆ cycloalkyl, and C₁₋₃ alkoxy, one or more of which are optionally substituted by 1, 2, or 3 R groups.

In some embodiments of the present disclosure, the above-mentioned R₃, R₄, and R₅ are each independently selected from H, F, Cl, Br, I, OH, CN, and NH₂, or selected from Me, Et, 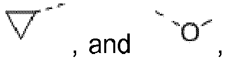 one or more of which are optionally substituted by 1, 2, or 3 R groups.

In some embodiments of the present disclosure, the above-mentioned R₃, R₄, and R₅ are each independently selected from H, F, Cl, Br, I, CN, CH₂F, CHF₂, CF₃,

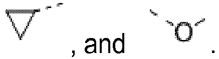

In some embodiments of the present disclosure, the above structural unit 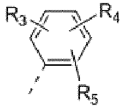 is

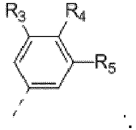

In some embodiments of the present disclosure, the above structural unit 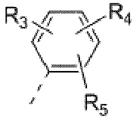 is selected from the group consisting of

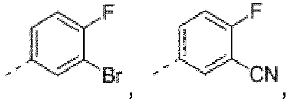

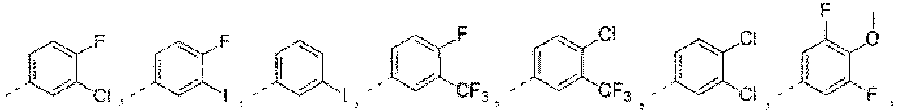

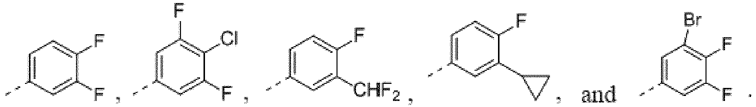

In some embodiments of the present disclosure, the above compound or the pharmaceutically acceptable salt thereof is selected from 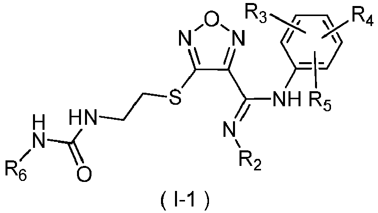 wherein
R₂, R₃, R₄, and R₅ are as defined in the above;
R₆ is selected from H, or selected from C₁₋₃ alkyl group and C₃₋₆ cycloalkyl group, one or more of which are optionally substituted by 1, 2, or 3 R' groups; and
provided that R₆ is not Me.

In some embodiments of the present disclosure, the above-mentioned R₆ is selected from the group consisting of H, CF₃, Et, 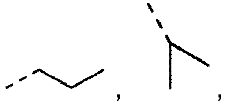 and

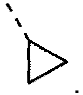

In some embodiments of the present disclosure, the above compound or the pharmaceutically acceptable salt thereof is selected from the group consisting of 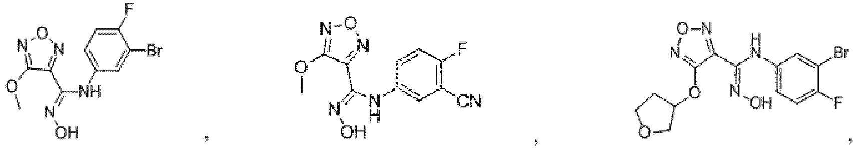 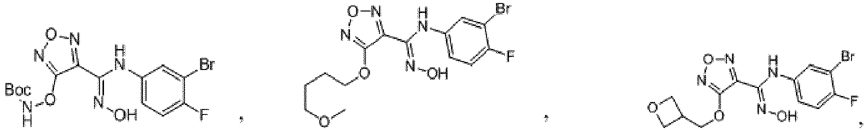 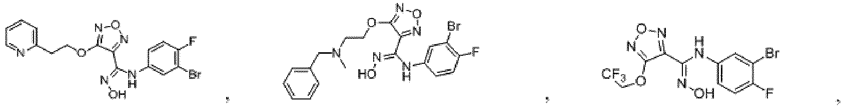 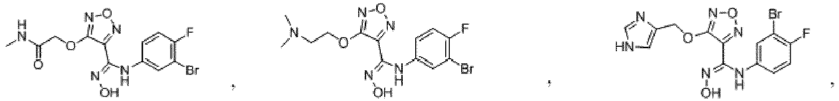 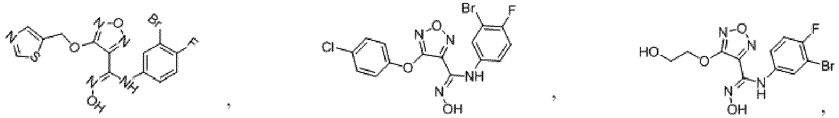 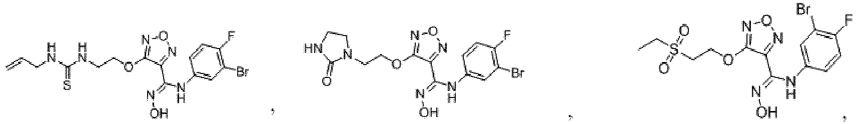 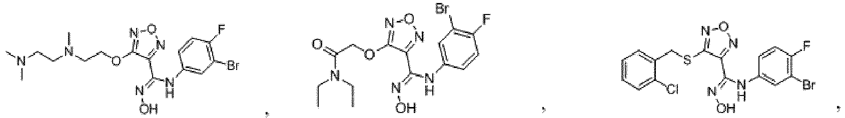 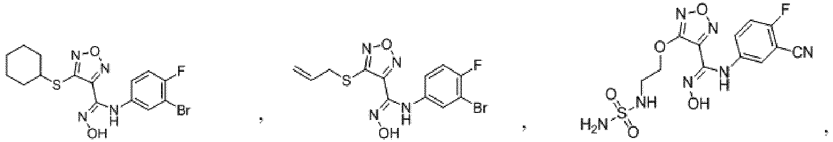 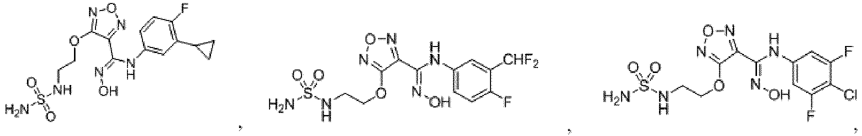 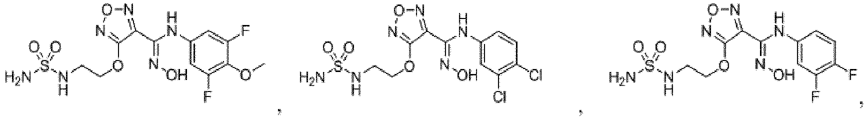 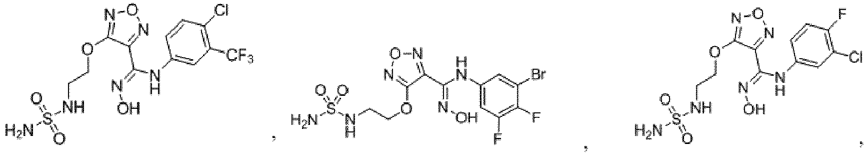 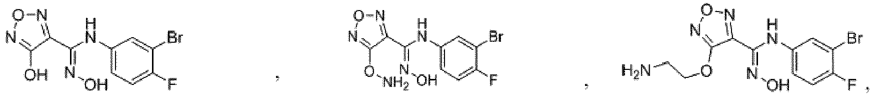 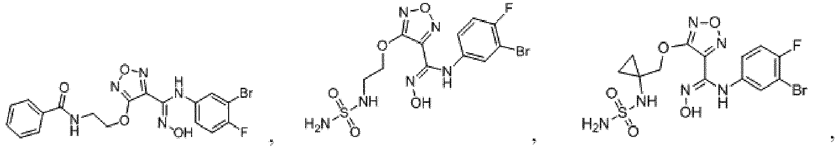 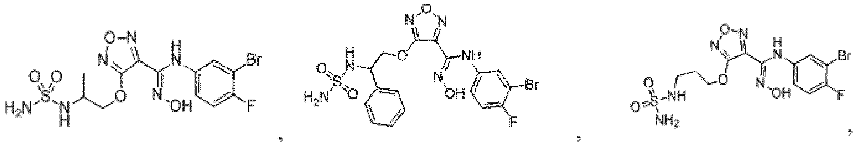 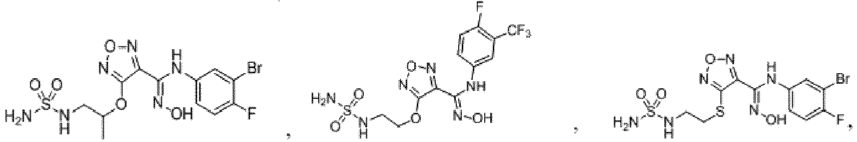 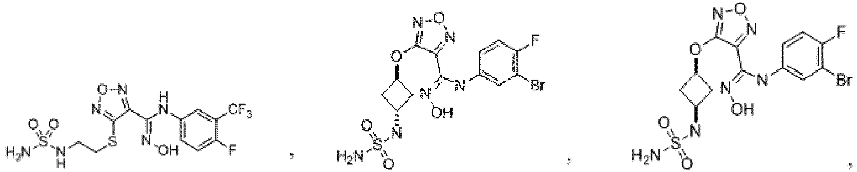 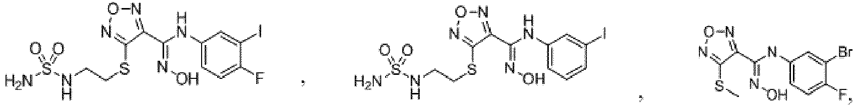 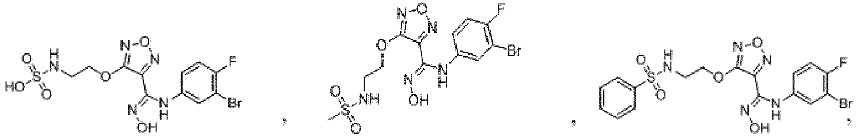 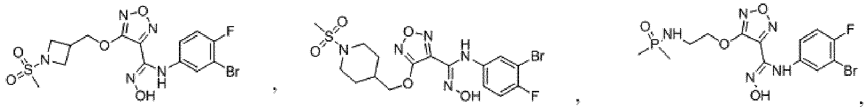 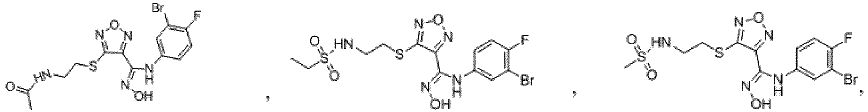 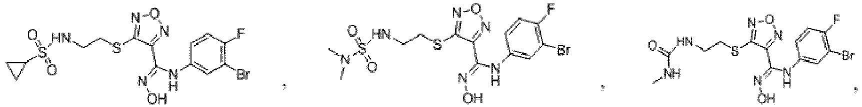 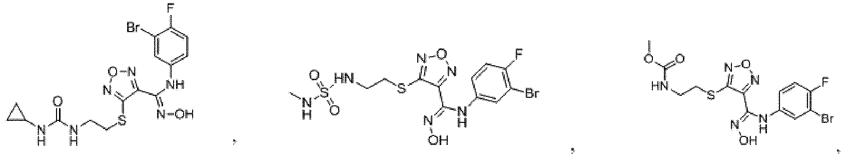 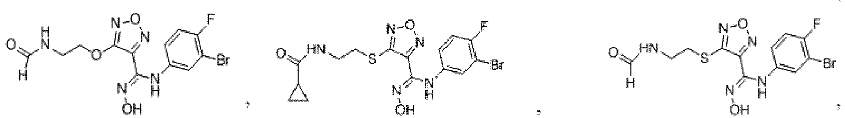 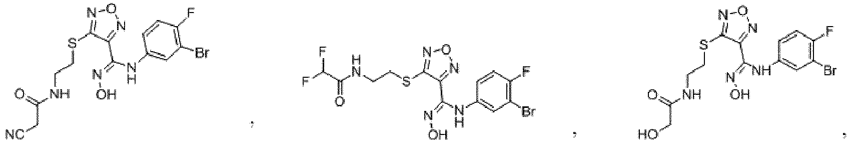 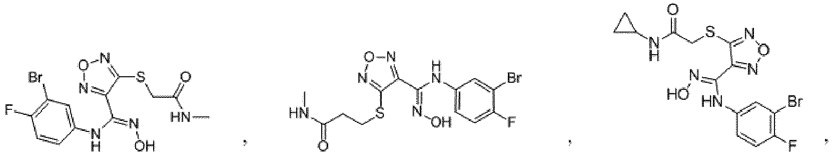 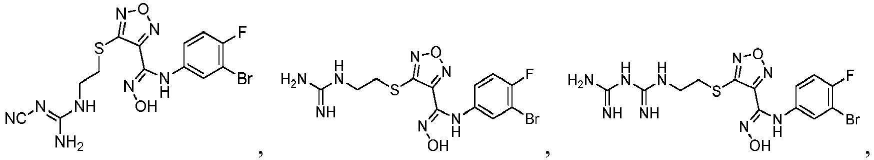 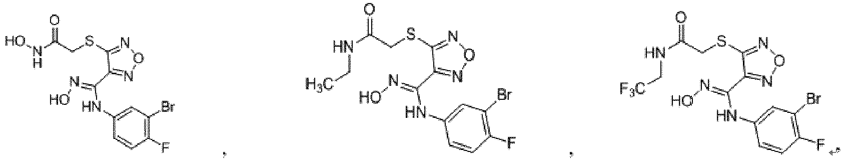 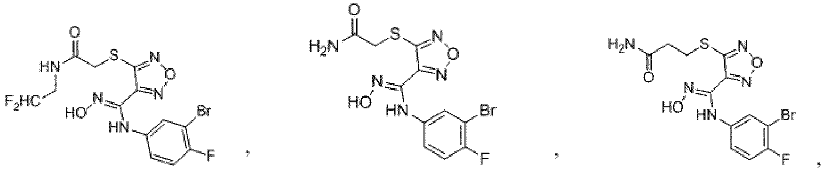 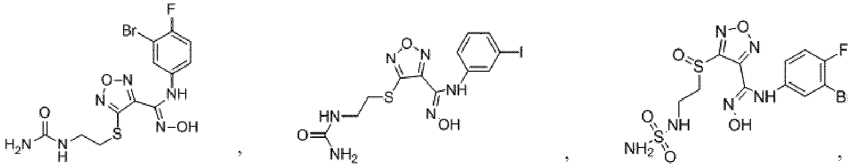 and

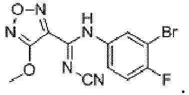

The present disclosure further provides a pharmaceutical composition including the above compound or a pharmaceutically acceptable salt thereof as an active ingredient and a pharmaceutical acceptable carrier.

The present disclosure further provides use of the above compound or the pharmaceutically acceptable salt thereof or a composition containing the compound or the pharmaceutically acceptable salt thereof in preparation of a medicament for treatment of IDO1-related diseases.

### Definition and Explanation

Unless otherwise stated, the following terms and phrases used herein are intended to have the following meanings. A specific term or phrase without being specifically defined should not be regarded as being uncertain or unclear, but should be understood by its plain meaning. When a brand name appears herein, it is intended to refer to a commercial product corresponding thereto or an active component thereof.

A term "pharmaceutically acceptable" refers to those compounds, materials, compositions, and/or dosage forms which are, within the scope of sound medical judgment, suitable for use in contact with tissues of human beings and animals without excessive toxicity, or causing irritation or allergic reactions, or other problems or complications, and commensurate with a reasonable benefit/risk ratio.

The term "pharmaceutically acceptable salt" refers to a salt of a compound of the present disclosure which is prepared from the compound with specific substituents discovered in the present disclosure and a relatively non-toxic acid or base. When the compound of the present disclosure contains a relatively acidic functional group, a base addition salt can be obtained by contacting such compounds in a neutral form with a sufficient amount of base in a pure solution or in a suitable inert solvent. Examples of the pharmaceutically acceptable base addition salt include salt of sodium, potassium, calcium, ammonium, organic amine, or magnesium, or the like. When the compound of the present disclosure contains a relatively basic functional group, an acid addition salt can be obtained by contacting such compounds in a neutral form with a sufficient amount of acid in a pure solution or in a suitable inert solvent. Examples of the pharmaceutically acceptable acid addition salt include a salt of an inorganic acid, where the inorganic acid includes, for example, hydrochloric acid, hydrobromic acid, nitric acid, carbonic acid, bicarbonate, sulfuric acid, bisulfate, hydriodic acid, phosphoric acid, hydrogen phosphate, dihydrogen phosphate, and phosphorous acid; as well as a salt of an organic acid, where the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluene sulfonic acid, citric acid, tartaric acid, methanesulfonic acid and the like; and also a salt of an amino acid (such as arginate), and a salt of an organic acid like glucuronic acid (see Berge et al., "Pharmaceutical Salts", Journal of Pharmaceutical Science 66: 1-19 (1977)). Certain specific compounds of the present disclosure contain basic and acidic functional groups, such that the compounds can be transformed to any of the base or acid addition salts.

Preferably, the neutral form of the compound is regenerated by contacting the salt with a base or an acid in a conventional manner and then separating a parent compound. A parent form of a compound and various salt forms thereof differ in certain physical properties, such as solubility in a polar solvent.

"Pharmaceutically acceptable salt" used herein belongs to a derivative of the compound of the present disclosure, wherein the parent compound is modified by salifying with an acid or a base. Examples of the pharmaceutically acceptable salt include but are not limited to: an inorganic acid or organic acid salt of a basic group such as amine, an alkali metal or an organic salt of an acid radical such as carboxylic acid and so on. The pharmaceutically acceptable salt includes conventional non-toxic salts or quaternary ammonium salts of the parent compound, such as a salt formed by a non-toxic inorganic acid or organic acid. The conventional non-toxic salt includes but is not limited to those salts derived from an inorganic acid and an organic acid, where the inorganic acid or the organic acid is selected from 2-acetoxybenzoic acid, 2-isethionic acid, acetic acid, ascorbic acid, benzenesulfonic acid, benzoic acid, bicarbonate, carbonic acid, citric acid, edetic acid, ethanedisulfonic acid, ethanesulfonic acid, fumaric acid, glucoheptose, gluconic acid, glutamic acid, glycolic acid, hydrobromic acid, hydrochloric acid, hydriodate, hydroxyl, hydroxynaphthoic acid, isethionic acid, lactic acid, lactose, dodecanesulfonic acid, maleic acid, malic acid, mandelic acid, methanesulfonic acid, nitric acid, oxalic acid, pamoic acid, pantothenic acid, phenylacetic acid, phosphoric acid, polygalacturonic acid, propionic acid, salicylic acid, stearic acid, folinic acid, succinic acid, aminosulfonic acid, p-aminobenzenesulfonic acid, sulfuric acid, tannic acid, tartaric acid, and p-toluene sulfonic acid.

The pharmaceutically acceptable salt of the present disclosure can be synthesized from the parent compound containing an acidic radical or a basic group by a conventional chemical method. Generally, a preparation method of such salt is: in water or an organic solvent or a mixture of both, reacting these compounds which are in a form of free acids or bases with a stoichiometric amount of proper base or acid. In general, a non-aqueous medium such as ether, ethyl acetate, ethanol, isopropanol or acetonitrile is preferred.

Some compounds of the present disclosure may contain an asymmetric carbon atom (optical center) or double bond. Racemates, diastereomers, geometric isomers, and individual isomers are all encompassed within the scope of the present disclosure.

Unless otherwise stated, an absolute configuration of a stereocenter is represented by wedge and dashed lines ( 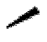 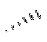 ), and a relative configuration of a stereocenter is represented by 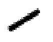 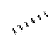 . When the compound described herein contains an olefinic double bond or other geometrically asymmetric center, unless otherwise specified, *E* and *Z* geometric isomers are included. Similarly, all tautomeric forms are all included within the scope of the present disclosure.

The compound of the present disclosure may be present in a specific geometric isomer form or stereoisomeric form. It is contemplated in the present disclosure that all of this class of compounds, including cis- and trans-isomers, (-)- and (+)- enantiomers, (*R*)- and (*S*)- enantiomers, diastereomers, (*D*)-isomers, (*L*)-isomers, as well as racemic mixtures thereof and other mixtures, for example, enantiomer- or diastereomer-enriched mixtures, and all of these mixtures are within the scope of the present disclosure. Additional asymmetric carbon atoms may be present in a substituent such as an alkyl. All of these isomers and their mixtures are included within the scope of the present disclosure.

Optically active (*R*)- and (*S*)-isomers and *D* and *L* isomers can be prepared by chiral synthesis or chiral reagents or other conventional techniques. If an enantiomer of a certain compound of the present disclosure is desired, it can be prepared by asymmetric synthesis or derivatization reaction in the presence of a chiral auxiliary agent, separating the resultant diastereomeric mixture, and performing cleavage of auxiliary groups to provide a pure desired enantiomer. Alternatively, when a molecule contains a basic functional group (such as an amino group) or an acidic functional group (such as a carboxyl group), a diastereomeric salt is formed by reaction of it with an appropriate optically active acid or base, then followed by diastereomeric resolution by a conventional method known in the art, and then the pure enantiomer is obtained by recovery. In addition, separation of an enantiomer and a diastereomer is usually accomplished by chromatography, where the chromatography employs a chiral stationary phase, and is optionally combined with a chemical derivatization method (e.g., generating a carbamate from an amine).

The term "pharmaceutically acceptable carrier" refers to any formulation or carrier medium which is capable of delivering an effective amount of an active substance of the present disclosure, will not interfere with the biological activity of the active substance and has no toxic or side effect on a host or a patient. Typical examples of carriers include water, oil (vegetable and mineral), cream matrix, lotion matrix, ointment matrix and so on. These matrixes include a suspending agent, a viscosity enhancer, a transdermal enhancer and so on. These formulations are well known to those in the art of cosmetics or in the art of topical drugs. As to other information about the carrier, reference can be made to Remington: The Science and Practice of Pharmacy, 21st Ed., Lippincott, Williams & Wilkins (2005), the content of which document is incorporated herein by reference.

In terms of pharmaceutical or pharmacological active agent, a term "effective amount" or "therapeutically effective amount" refers to an amount of a drug or formulation sufficient to achieve desired effects with minimal toxicity. For an oral formulation of the present disclosure, an "effective amount" of an active substance in a composition refers to an amount required to achieve desired effects in combination with another active substance in the composition. The determination of the effective amount varies from person to person, and depends on the age, the general condition of a recipient, as well as the specific active substance. On a patient-by-patient basis, an appropriate effective amount can be determined by a person skilled in the art according to conventional tests.

The term "active ingredient," "therapeutic agent," "active substance" or "active agent" refers to a chemical entity, which can effectively treat a disorder, disease or condition of a target subject.

"Optional" or "optionally" means that an event or situation subsequently described may occur but not necessarily, and such description includes a case where the event or situation occurs and a case where the event or situation does not occur.

The term "substituted" refers to any one or more hydrogen atoms on a specific atom being replaced by substituent(s), wherein the one or more hydrogen atoms may include a hydrogen and a variant of hydrogen provided that a valence state of the specific atom is normal and the substituted compound is stable. When the substituent is a keto group (i.e. =O), it means that two hydrogen atoms are replaced. Keto substitution will not occur on an aryl group. The term "optionally substituted" means that it may be substituted or not be substituted, unless otherwise specified, a given type and number of substituents may be arbitrary, provided that they may be achieved in chemistry.

When any variable (e.g. R) occurs more than once in the composition or structure of a compound, its definition at each occurrence is independent. Therefore, for example, if a group is substituted by 0-2 R groups, the group may optionally be substituted by at most two R groups, and R has an independent option at each occurrence. In addition, a combination of substituents and/or variants thereof is allowed only if such a combination will lead to a stable compound.

When a linking group is 0 in number, for example, -(CRR)₀-, it means that this linking group is a single bond.

When one variable thereof is selected from a single bond, it means that two groups connected thereby are directly linked, for example, when L in A-L-Z represents a single bond, it means that this structure is equivalent to A-Z.

When a substituent is absent, it means that this substituent does not exist, for example, when X in A-X is absent, it means that this structure actually is A. When a bond of a substituent can be cross-connected to two atoms on a ring, such substituent can be bonded to any atom on the ring. If the atom in the exemplified substituent connected to a particular compound included, but not specifically mentioned, in the general chemical structure is not specified, such substituent can be bonded via any of its atoms. A combination of a substituent and/or variants thereof is allowed only if such a combination will lead to a stable compound. For example, a structural unit 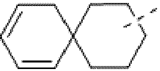 or 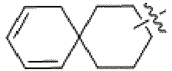 indicates that substitution may occur on any position of the cyclohexyl or cyclohexadiene.

Unless otherwise specified, the term "hetero" refers to a heteroatom or a heteroatom group (i.e. an atomic group containing a heteroatom), including atoms other than carbon (C) and hydrogen (H) and atomic groups containing these heteroatoms, such as oxygen (O), phosphorus (P), nitrogen (N), sulfur (S), silicon (Si), germanium (Ge), aluminum (Al), boron (B), -O-, -S-, =O, =S, -C(=O)O-, -C(=O)-, -C(=S)-, -S(=O), -S(=O)₂-, 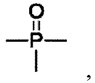 and optionally substituted -C(=O)N(H)-, -N(H)-, -C(=NH)-, -S(=O)₂N(H)-, or -S(=O)N(H)-.

Unless otherwise specified, "ring" refers to substituted or unsubstituted cycloalkyl, heterocycloalkyl, cycloalkenyl, heterocycloalkenyl, cycloalkynyl, heterocycloalkynyl, aryl or heteroaryl. The so-called ring includes a single ring, a joint ring, a spiro ring, a fused ring or a bridged ring. The number of atoms on the ring is usually defined as the number of members of the ring. For example, "5- to 7-membered ring" is a ring looped with 5∼7 atoms. Unless otherwise specified, the ring optionally contains 1∼3 heteroatoms. Therefore, "5- to 7-membered ring" includes, for example, phenyl, pyridine, and piperidinyl. On the other hand, a term "5- to 7-membered heterocycloalkyl ring" includes pyridyl and piperidinyl, but does not include phenyl. The term "ring" also includes a ring system containing at least one ring, wherein each "ring" is independently in line with the above definition.

Unless otherwise specified, a term "heterocycle" or "heterocyclic group" refers to a stable monocyclic, bicyclic or tricyclic ring containing heteroatom(s) or heteroatom group(s), which may be saturated, partially unsaturated or unsaturated (aromatic), and contain carbon atoms and 1, 2, 3 or 4 ring heteroatoms independently selected from N, O, and S, wherein any of the above-mentioned heterocycles can be fused to a benzene ring to form a bicyclic ring. Nitrogen and sulfur heteroatoms can be optionally oxidized (i.e., NO and S(O)p, where p is 1 or 2). The nitrogen atom can be substituted or unsubstituted (i.e. N or NR, wherein R is H or other substituent that has been defined herein). The heterocycle can be attached to a side group of any heteroatom or carbon atom so as to form a stable structure. If a formed compound is stable, the heterocycle described herein can be substituted on a carbon site or a nitrogen site. The nitrogen atom in the heterocycle is optionally quaternized. In a preferred embodiment, when a total number of S and O atoms in the heterocycle exceeds 1, these heteroatoms are not adjacent to each other. In another preferred embodiment, a total number of S and O atoms in the heterocycle is no more than 1. As used herein, the term "aromatic heterocyclic group" or "heteroaryl" refers to a stable aromatic ring of a 5-, 6-, 7-membered monocyclic or bicyclic or 7-, 8-, 9- or 10-membered bicyclic heterocyclyl, which contains carbon atoms and 1, 2, 3 or 4 ring heteroatoms independently selected from N, O, and S. The nitrogen atom can be substituted or unsubstituted (i.e. N or NR, wherein R is H or other substituent that has been defined herein). Nitrogen and sulfur heteroatoms can be optionally oxidized (i.e., NO and S(O)ₚ, where p is 1 or 2). It is worth noting that the total number of S and O atoms on the aromatic heterocycle is no more than 1. Bridged rings are also included in the definition of the heterocycle. When one or more atoms (i.e. C, O, N, or S) are connected to two nonadjacent carbon atoms or nitrogen atoms, a bridged ring is formed. A preferred bridged ring includes but is not limited to: one carbon atom, two carbon atoms, one nitrogen atom, two nitrogen atoms, and one carbon-nitrogen group. It is worth noting that a bridge always converts a monocyclic ring into a tricyclic ring. In the bridged ring, the substituent on the ring also can be present on the bridge.

Examples of heterocyclic compound include, but are not limited to: acridinyl, azocinyl, benzimidazolyl, benzofuryl, benzomercaptofuryl, benzomercaptophenyl, benzoxazolyl, benzoxazolinyl, benzothiazolyl, benzotriazolyl, benzotetrazolyl, benzoisoxazolyl, benzoisothiazolyl, benzoimidazolinyl, carbazolyl, 4a*H*-carbazolyl, carbolinyl, chromanyl, chromene, cinnolinyl decahydroquinolinyl, 2*H*,6*H*-1,5,2-dithiazinyl, dihydrofuro[2,3-b]tetrahydrofuryl, furyl, furazanyl, imidazolidinyl, imidazolinyl, imidazolyl, 1*H*-indazolyl, indoalkenyl, indolinyl, indolizinyl, indolyl, 3*H*-indolyl, isobenzofuryl, isoindolyl, isoindolinyl, isoquinolinyl, isothiazolyl, isoxazolyl, methylenedioxyphenyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxindolyl, pyrimidyl, phenanthridinyl, phenanthrolinyl, phenazine, phenothiazine, benzoxanthinyl, phenoxazinyl, phthalazinyl, piperazinyl, piperidyl, oxopiperidinyl, 4-oxopiperidinyl, piperonyl, pteridyl, purinyl, pyranyl, pyrazinyl, pyrazolidinyl, pyrazolinyl, pyrazolyl, pyridazinyl, pyridinooxazole, pyridinoimidazole, pyridinothiazole, pyridyl, pyrrolidinyl, pyrrolinyl, 2*H*-pyrrolyl, pyrrolyl, quinazolinyl, quinolinyl, 4*H*-quinolizinyl, quinoxalinyl, quinuclidinyl, tetrahydrofuryl, tetrahydroisoquinolinyl, tetrahydroquinolinyl, tetrazolyl, 6*H*-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, isothiazolylthienyl, thiophenoxazolyl, thiophenothiazolyl, thiophenoimidazolyl, thienyl, triazinyl, 1,2,3-triazolyl, 1,2,4-triazolyl, 1,2,5-triazolyl, 1,3,4-triazolyl, and xanthenyl. Fused ring and spiro ring compounds are also included.

Unless otherwise specified, the term "hydrocarbon group" or a specific embodiment thereof (such as alkyl, alkenyl, alkynyl, and aryl) itself or as a part of another substituent represents a straight-chain, branched or cyclic hydrocarbon atomic group or a combination thereof, which can be completely saturated (e.g. alkyl), mono- unsaturated or poly-unsaturated (e.g. alkenyl, alkynyl, and aryl), can be monosubstituted or polysubstituted, can be univalent (such as methyl), bivalent (such as methylene) or multivalent (such as methine), can include bivalent or multivalent atomic groups, with a specified number of carbon atoms (for example, C₁-C₁₂ represents 1 to 12 carbon atoms, C₁₋₁₂ is selected from C₁, C₂, C₃, C₄, C₅, C₆, C₇, C₈, C₉, C₁₀, C₁₁, and C₁₂; and C₃₋₁₂ is selected from C₃, C₄, C₅, C₆, C₇, C₈, C₉, C₁₀, C₁₁, and C₁₂). The term "hydrocarbon group" includes but is not limited to an aliphatic hydrocarbon group and an aromatic hydrocarbon group, wherein the aliphatic hydrocarbon group includes chains and cycles, specifically including but not limited to alkyl, alkenyl, and alkynyl, the aromatic hydrocarbon group includes but is not limited to 6- to 12-membered aromatic hydrocarbon group such as benzene and naphthalene. In some examples, the term "hydrocarbon group" refers to a straight-chain or branched atomic group or their combination, which can be completely saturated, monounsaturated or polyunsaturated, can include divalent and polyvalent atomic groups. Examples of saturated hydrocarbon atomic groups include but are not limited to homologues or isomers of methyl, ethyl, n-propyl, iso-propyl, n-butyl, tert-butyl, iso-butyl, sec-butyl, iso-butyl, cyclohexyl, (cyclohexyl)methyl, cyclopropylmethyl, and n-pentyl, n-hexyl, n-heptyl, n-octyl and the like. An unsaturated hydrocarbon group has one or more double bond or triple bond, examples of which include but are not limited to ethenyl, 2-propenyl, butenyl, crotyl, 2-isopentenyl, 2-(butadienyl), 2,4-pentadienyl, 3-(1,4-pentadienyl), acetenyl, 1- and 3-propynyl, 3-butynyl, and higher homologues and isomers.

Unless otherwise specified, the term "heterohydrocarbon group" or a specific embodiment thereof (such as heteroalkyl, heteroalkenyl, heteroalkynyl, and heteroaryl) itself or combined with another term refers to a stable straight-chain, branched or cyclic hydrocarbon group or combinations thereof, consisting of a certain number of carbon atoms and at least one heteroatom. In some examples, the term "heteroalkyl" itself or combined with another term refers to a stable straight-chain, branched hydrocarbon group or combinations thereof, consisting of a certain number of carbon atoms and at least one heteroatom. In one typical example, the heteroatom is selected from B, O, N, and S, in which the nitrogen and sulfur atoms are optionally oxidized, and the nitrogen heteroatom is optionally quaternized. Heteroatoms or heteroatom groups can be located in any internal position of the heterohydrocarbon group, including a position where the hydrocarbon group is attached to the rest part of the molecule. But terms "alkoxy", "alkylamino", and "alkylthio" (or thioalkoxy) are conventional expressions, and refer to those alkyl groups connected to the rest part of the molecule through an oxygen atom, an amino group, or a sulfur atom, respectively. Examples include but are not limited to -CH₂-CH₂-O-CH₃, -CH₂-CH₂-NH-CH₃, -CH₂-CH₂-N(CH₃)-CH₃, -CH₂-S-CH₂-CH₃, -CH₂-CH₂, -S(O)-CH₃, -CH₂-CH₂-S(O)₂-CH₃ -CH=CH-O-CH₃, -CH₂-CH=N-OCH₃, and -CH=CH-N(CH₃)-CH₃. At most two heteroatoms may be consecutive, for example, -CH₂-NH-OCH₃

Unless otherwise specified, a term "cyclohydrocarbon group", "heterocyclic hydrocarbon group" or a specific embodiment thereof (such as aryl, heteroaryl, cycloalkyl, heterocycloalkyl, cycloalkenyl, heterocyclic alkenyl, cycloalkynyl, and heterocyclic alkynyl) itself or combined with other terms respectively refers to a cyclized "hydrocarbon group", "heterohydrocarbon group". In addition, in terms of heterohydrocarbon group or heterocyclic hydrocarbon group (such as heteroalkyl and heterocyclic alkyl), heteroatoms can occupy a position where the heterocyclic ring is attached to the rest part of the molecule. Examples of the cyclohydrocarbon group include but are not limited to cyclopentyl, cyclohexyl, 1-cyclohexenyl, 3-cyclohexenyl, cycloheptyl and so on. Non-limiting examples of the heterocyclic group include 1-(1,2,5,6-tetrahydropyridyl), 1-piperidyl, 2-piperidyl, 3-piperidyl, 4-morpholinyl, 3-morpholinyl, tetrahydrofuran-2-yl, tetrahydrofurylindol-3-yl, tetrahydrothiophen-2-yl, tetrahydrothiophen-3-yl, 1-piperazinyl, and 2-piperazinyl.

Unless otherwise specified, the term "alkyl group" is used to represent a straight-chain or branched saturated hydrocarbon group, can be monosubstituted (such as -CH₂F) or polysubstituted (such as -CF₃), and can be univalent (such as methyl), bivalent (such as methylene) or multivalent (such as methine). Examples of the alkyl group include methyl (Me), ethyl (Et), propyl (such as n-propyl and isopropyl), butyl (such as n-butyl, isobutyl, s-butyl, and t-butyl), pentyl (such as n-pentyl, isopentyl, and neopentyl) and the like.

Unless otherwise specified, "alkenyl" refers to an alkyl having one or more C=C double bonds on any site of a chain, can be monosubstituted or polysubstituted, and can be univalent, bivalent or multivalent. Examples of alkenyl include ethenyl, propenyl, butenyl, pentenyl, hexenyl, 1,3-butadienyl, 1,3-pentadienyl, 1,3-hexadienyl etc.

Unless otherwise specified, cycloalkyl includes any stable cyclic or polycyclic hydrocarbon group, can be saturated for any carbon atom, can be monosubstituted or polysubstituted, and can be univalent, bivalent or multivalent. Examples of these cycloalkyls include but are not limited to cyclopropyl, norbornyl, [2.2.2]bicyclooctane, [4.4.0]bicyclodecane etc.

Unless otherwise specified, cycloalkenyl includes any stable cyclic or polycyclic hydrocarbon group, wherein the hydrocarbon group contains one or more unsaturated C=C double bonds at any site of the ring, can be monosubstituted or polysubstituted, and can be univalent, bivalent or multivalent. Examples of these cycloalkenyls include but are not limited to cyclopentenyl, cyclohexenyl etc.

Unless otherwise specified, the term "halo" or "halogen" itself or as part of another substituent refers to fluorine, chlorine, bromine, or iodine atom. Additionally, the term "haloalkyl" is intended to include monohaloalkyl and polyhaloalkyl. For example, the term "halo(C₁-C₄)alkyl" is intended to include but is not be limited to trifluoromethyl, 2,2,2-trifluoroethyl, 4-chlorobutyl, 3-bromopropyl etc. Unless otherwise specified, examples of haloalkyl include but are not limited to trifluoromethyl, trichloromethyl, pentafluoroethyl, and pentachloroethyl.

"Alkoxy" represents the above alkyl group with a specific number of carbon atoms connected by an oxygen bridge. Unless otherwise specified, C₁₋₆ alkoxy includes C₁, C₂, C₃, C₄, C₅, and C₆ alkoxy. Examples of alkoxy include but are not limited to: methoxy, ethoxy, n-propoxy, iso-propoxy, n-butoxy, sec-butoxy, t-butoxy, n-pentoxy, and sec-pentoxy. Unless otherwise specified,the term "aryl" refers to a polyunsaturated aromatic hydrocarbon substituent, which can be monosubstituted or polysubstituted, can be monovalent, divalent, or polyvalent, and/or can be monocyclic or polycyclic (for example 1 to 3 rings, wherein at least one ring is aromatic), connected via a fused or covalent manner. Then term "heteroaryl" refers to aryl (or ring) containing one to four heteroatoms. In an exemplary example, the heteroatom is selected from B, N, O, and S, in which nitrogen and sulfur atoms are optionally oxidized, and nitrogen atom is optionally quaternized. The heteroaryl can be connected to the rest part of the molecule through a heteroatom. Non-limiting examples of aryl or heteroaryl include phenyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolinyl, 5-isoquinolinyl, 2-quinoxalinyl, 5-quinoxalinyl, 3-quinolinyl, and 6-quinolinyl. A substituent of any of the above aryl and heteroaryl ring systems is selected from acceptable substituents described below.

Unless otherwise specified, when used in combination with other terms (for example, aryloxy, arylthio, arylalkyl), aryl includes aryl and heteroaryl ring as defined above. Thus, the term "arylalkyl" is intended to include those atomic groups formed by attaching an aryl to an alkyl (for example, benzyl, phenethyl, and pyridylmethyl), including those alkyls in which a carbon atom (such as methylene) has been substituted with for example an oxygen atom, e.g., phenoxymethyl and 2-pyridyloxymethyl 3-(1-naphthyloxy)propyl.

The compounds of the present disclosure can be prepared through multiple synthetic methods which are well-known to a person skilled in the art, including specific embodiments listed below, embodiments formed by combination of them with other chemical synthetic methods, and equivalent alternatives which are well-known to a person skilled in the art. Preferred embodiments include but are not limited to examples of the present disclosure.

Solvents used in the present disclosure are commercially available. Following abbreviations are used in the present disclosure: aq represents aqueous (water); HATU represents O-(7-azabenzotriazol-1-yl)-*N,N*,N',N'-tetramethyluronium hexafluorophosphate; EDC represents N-(3-dimethylaminopropyl)-N'-ethyl carbodiimide hydrochloride; m-CPBA represents 3-chloroperbenzoic acid; eq represents equivalent, equal-quantitative; CDI represents carbonyl diimidazole; DCM represents dichloromethane; PE represents petroleum ether; DIAD represents diisopropyl azodicarboxylate; DMF represents *N,N*-dimethylformamide; DMSO represents dimethyl sulfoxide; EtOAc represents ethyl acetate; EtOH represents ethanol; MeOH represents methanol; CBz represents benzyloxycarbonyl, an amino protecting group; BOC represents tert-butoxycarbonyl, an amino protecting group; HOAc represents acetic acid; NaCNBH₃ represents sodium cyanoborohydride; r.t. represents room temperature; O/N represents overnight; THF represents tetrahydrofuran; Boc₂O represents di-tert-butyl dicarbonate; TFA represents trifluoroacetic acid; DIPEA represents diisopropylethylamine; SOCl₂ represents thionyl chloride; CS₂ represents carbon disulfide; TsOH represents p-toluene sulfonic acid; NFSI represents N-fluorobenzenesulfonimide; NCS represents N-chlorosuccinimide; *n*-Bu₄NF represents tetrabutylammonium fluoride; iPrOH represents 2-propanol; HCI represents hydrochloric acid; ACN represents acetonitrile; mp represents melting point; LDA represents lithium diisopropylamide; NFK represents N-methylkynurenine; DPBS represents Dulbecco's phosphate buffered saline; LCMS represents liquid chromatography-mass spectrometry; HPLC represents high performance liquid chromatography; TLC represents thin-layer preparative plate (thin-layer chromatography), MS represents mass spectrum, ESI represents a detector of mass spectrum, H NMR represents nuclear magnetic resonance, CDCl₃ represents deuterated chloroform, CD₃OD represents deuterated methanol, P-gp represents P-glycoprotein, an efflux protein expressed on cell membrane; HEPES represents 2-[4-(2-hydroxyethyl)-1-piperazinyl]ethanesulfonic acid; a compound 231 and a compound 0231 represent a same compound; a compound 117 and a compound 0117 represent a same compound; a compound 227 and a compound 0227 represent a same compound; a compound 360 represents INCB024360. Compounds are named by manual work or software ChemDraw®, and commercially available compounds are named in accordance with suppliers' catalogue.

As a novel IDO1 inhibitor, the compound of the present disclosure has remarkable activity *in vitro,* excellent solubility and permeability, and excellent pharmacokinetics and pharmaceutical effect.

### Brief Description of Drawings

FIG. 1 shows the effect of a tested drug on the body weight of tumor-bearing mice;
FIG. 2 shows the effect of the tested drug on the volume of a transplanted tumor; and
FIG. 3 shows the effect of the tested drug on the weight of the transplanted tumor.

### Detailed Description of Embodiments

### Reference Example 1: Segment BB-1

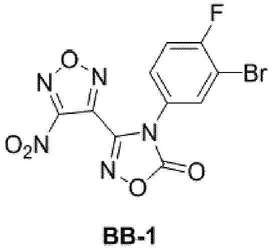

### Synthetic route:

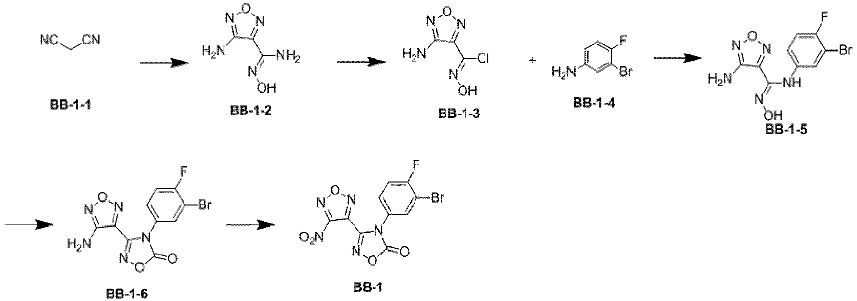

### Step 1: synthesis of compound BB-1-2

BB-1-1 (20.00 g, 302.76 mmol, 19.05 mL, 1.00 *eq*) was dissolved in water (436.00 mL), and stirred for 5 minutes. The reaction solution was cooled in an ice bath to 0 °C. Sodium nitrite (22.98 g, 333.04 mmol, 18.09 mL, 1.10 *eq*) was added, and then hydrochloric acid (6 M, 3.53 mL, 0.07 *eq*) was added. After 15 minutes, the ice bath was removed. After the reaction solution was stirred at 25 °C for 1.5 hours, a 50% hydroxylamine aqueous solution (60.00 g, 908.28 mmol, 3.00 eq) was added all at once. After the reaction solution was stirred continuously at 25 °C for 1 hour, the reaction solution was slowly heated to reflux. The reaction was carried out at reflux for 2 hours before being slowly cooled to 25 °C, and was allowed to further react for 16 hours. At 0 °C, the reaction solution was adjusted to have pH=7.0 with 6 N hydrochloric acid (70 mL, slowly added dropwise for about 30 minutes), and continued to be stirred at 0 °C for 1 hour. A solid precipitate appeared and was filtered and washed with water. A light yellow solid was collected and dried in the air, without the need of further purification. The product BB-1-2 (38.08 g, yield: 87.89%, purity: 100%) was finally obtained as a light yellow solid. MS (ESI) m/z:144 [M+H]⁺.

### Step 2: synthesis of compound BB-1-3

The compound BB-1-2 (38.08 g, 266.11 mmol, 1.00 *eq*) was dissolved in a mixed solution of water (532.00 mL), acetic acid (270.00 mL) and hydrochloric acid (6 M, 133.06 mL, 3.00 *eq*). The resultant mixture was heated to 45 °C and stirred until the solution was completely clear (about 0.5 hours). Sodium chloride (46.65 g, 798.33 mmol, 3.00 *eq*) was added. The mixed reaction solution was cooled to 0 °C. Sodium nitrite (17.99 g, 260.79 mmol, 14.17 mL, 0.98 *eq*) (dissolved in 63 mL of water) was slowly added dropwise to the reaction solution (more than 0.5 hours), while maintaining the temperature at 0 °C during addition. After the addition, the reaction solution continued to stir at 0 °C for 2 hours. With LCMS monitoring showing completion of reaction of raw materials, a precipitated solid was filtered and washed with water (6*60 mL). The solid obtained from the suction filtration was dissolved in ethyl acetate (400 mL), dried over anhydrous sodium sulfate, and filtered. A filtrate was dried by rotary evaporation under reduced-pressure distillation, without the need of purification. A light yellow solid product BB-1-3 (18.83 g, yield: 40.63%, purity: 93.33%) was finally obtained. MS (ESI) m/z: 163 [M+H]⁺

### Step 3: synthesis of compound BB-1-5

The compound BB-1-3 (2.00 g, 12.30 mmol, 1.00 *eq*) was dissolved in ethanol (25.00 mL), and compound BB-1-4 (4.67 g, 24.60 mmol, 2.00 *eq*) was added. After the mixed reaction solution was allowed to react at 85 °C for 16 hours. While being heated the reaction solution gradually turned brown. Complete reaction of raw materials was observed by LCMS monitoring and a desired compound was generated. The reaction solution was dried by rotary evaporation under reduced-pressure distillation. A crude product was separated and purified by flash silica gel column chromatography (petroleum ether:ethyl acetate=2:1), to yield a light gray solid product BB-1-5 (3.60 g, yield: 88.39%, purity: 95.46%). MS (ESI) m/z:316, 318 [M+H]⁺.

### Step 4: synthesis of compound BB-1-6

The compound BB-1-5 (3.60 g, 11.39 mmol, 1.00 *eq*) was dissolved in tetrahydrofuran (30.00 mL), and carbonyl diimidazole (2.03 g, 12.53 mmol, 1.10 *eq*) was added. The mixed reaction solution was allowed to react at 65 °C for 1 hour. With LCMS monitoring showing completion of reaction of raw materials, 20 mL of water was added, followed by extraction with ethyl acetate (25 mL*3). Organic phases were combined, washed with 1 M hydrochloric acid (20 mL*2), washed with brine, dried over anhydrous sodium sulfate, filtered, and dried by rotary evaporation under reduced-pressure distillation, without further purification. A dust-gray solid product BB-1-6 (3.55 g, yield: 91.11%, purity: 100%) was obtained. MS (ESI) m/z:342, 344 [M+H]⁺.

### Step 5: synthesis of compound BB-1

At 0 °C, sulfuric acid (35.00 mL) was slowly added to hydrogen peroxide (41.30 g, 364.30 mmol, 35.00 mL, 30% purity, 41.97 *eq*), then sodium tungstate (2.55 g, 8.68 mmol, 1.00 *eq*) was added followed by the compound BB-1-6 (2.97 g, 8.68 mmol, 1.00 *eq*). The mixture was heated to 25 °C and stirred for 16 hours. With LCMS monitoring showing about half of raw materials remaining, the mixture was diluted by addition of 250 mL of water, and subjected to suction filtration. A resultant white solid was rinsed with water (25 mL*3). The solid was dissolved in ethyl acetate (200 mL), dried over anhydrous sodium sulfate, and filtered. A filtrate was dried by rotary evaporation under reduced-pressure distillation. Purification was performed by flash silica gel column chromatography (petroleum ether:ethyl acetate=10:1) to obtain a light yellow solid product BB-1 (1.29 g, yield: 39.20%, purity: 98.14%). MS (ESI) m/z: 372, 374 [M+H]⁺.

### Reference Example 2: Segment BB-2

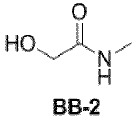

### Synthetic route:

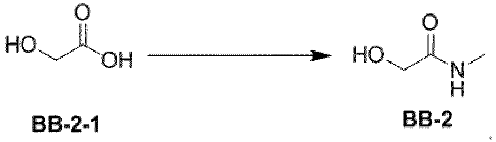

### Step 1: synthesis of compound BB-2

The compound BB-2-1 (25.00 mg, 328.73 umol, 20.00 uL, 1.00 *eq*) and methyl amine (44.39 mg, 657.46 umol, 2.00 *eq,* hydrochloride) were dissolved in *N,N*-dimethyl formamide (1.00 mL). Diisopropylethylamine (254.91 mg, 1.97 mmol, 344.47 uL, 6.00 *eq*) and HATU (187.49 mg, 493.10 umol, 1.50 *eq*) were added. The reaction solution turned yellow from colorless, and the reaction solution was allowed to react at 4 °C for 16 hours. Complete reaction of raw materials was observed by TLC monitoring (petroleum ether:ethyl acetate=1:1). Five mL of water was added to the reaction solution, followed by extraction with ethyl acetate (5 mL*3). Organic phases were combined, dried over anhydrous sodium sulfate, and filtered. A filtrate was dried by rotary evaporation under reduced-pressure distillation to obtain a crude product. The reaction succeeded, and a light yellow liquid product BB-2 (30.00 mg, crude product) was obtained.

### Reference Example 3: Segment BB-3

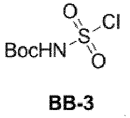

### Synthetic route:

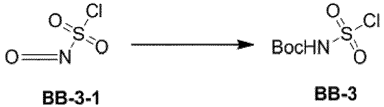

### Step 1: synthesis of compound BB-3

The compound BB-3-1 (571.78 mg, 4.04 mmol, 350.79 uL, 1.00 *eq*) was added to dichloromethane (5 mL), to which a dichloromethane (8 mL) solution containing tertiary butanol (314.42 mg, 4.24 mmol, 403.10 uL, 1.05 *eq*) was added dropwise at 0 °C. The reaction solution was stirred at 0 °C for 1 hour. A dichloromethane solution (13 mL) of a target product BB-3 (871.00 mg, crude product) was obtained and directly used for reaction in the next step.

### Reference Example 4: Segment BB-4

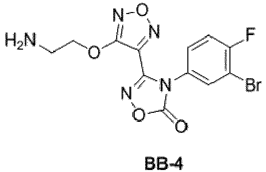

### Synthetic route:

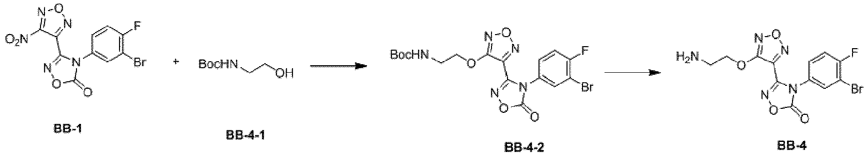

### Step 1: synthesis of compound BB-4-2

The compound BB-1 (1.00 g, 2.69 mmol, 1.00 *eq*) was dissolved in tetrahydrofuran (15.00 mL) and water (500.00 uL), to which the compound BB-4-1 (650.44 mg, 4.04 mmol, 625.42 uL, 1.50 *eq*) and sodium hydroxide (118.36 mg, 2.96 mmol, 1.10 *eq*) were added. The reaction solution was stirred at 25 °C for 16 hours. Complete reaction of raw materials was observed by LCMS monitoring and a desired compound was generated. Five mL of water was added, followed by extraction with ethyl acetate (5 mL*3), drying over anhydrous sodium sulfate, and filtration. A filtrate was dried by rotary evaporation under reduced-pressure distillation, without further purification, to obtain a liquid product BB-4-2 as yellow oil (1.73 g, crude product).

### Step 2: synthesis of compound BB-4

The compound BB-4-2 (1.73 g, 3.56 mmol, 1.00 *eq*) was dissolved in dichloromethane (10.00 mL). Hydrochloric acid/dioxane (4 M, 889.46 uL, 1.00 eq) was added. The reaction solution turned turbid and white from yellow, and reacted at 25 °C for 1 hour. A white solid precipitated. Complete reaction of raw materials was observed by LCMS monitoring, and a main product peak was generated. The reaction solution was dried by rotary evaporation to obtain a crude product, without purification. The reaction succeeded. A white solid product BB-4 (1.48 g, crude product, hydrochloride) was obtained. MS (ESI) m/z:386, 388 [M+H]⁺.

### Reference Example 5: Segment BB-5

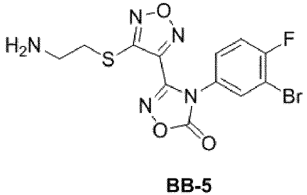

### Synthetic route:

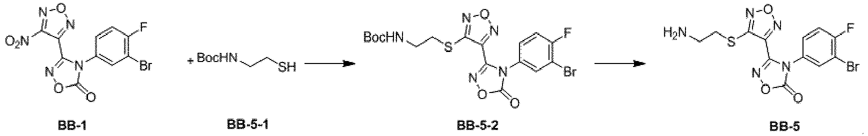

### Step 1: synthesis of compound BB-5-2

The compound BB-1 (2.50 g, 6.72 mmol, 1.00 *eq*) was dissolved in tetrahydrofuran (20.00 mL) and water (1.00 mL). Sodium bicarbonate (846.74 mg, 10.08 mmol, 392.01 uL, 1.50 *eq*) was added. The mixed solution was allowed to react at 14 °C for 16 hours. Complete reaction of raw materials was observed by LCMS monitoring and a main new product peak was generated. Twenty mL of water was added to the reaction, followed by extraction with ethyl acetate (30 mL*3). Organic phases were combined, dried over anhydrous sodium sulfate, and filtered. A filtrate was dried by rotary evaporation under reduced-pressure distillation. Purification was performed by flash silica gel column chromatography (petroleum ether:ethyl acetate=4:1). The reaction succeeded, and a white solid product BB-5-2 (3.29 g, yield: 97.47%) was obtained. MS (ESI) m/z: 502, 504 [M+H]⁺.

### Step 2: synthesis of compound BB-5

The compound BB-5-2 (4.09 g, 8.14 mmol, 1.00 *eq*) was dissolved in dichloromethane (30.00 mL). Hydrochloric acid/dioxane (4 M, 30.00 mL, 14.74 *eq*) was added. The reaction solution was allowed to react at 14 °C for 1 hour. It is observed by LCMS monitoring that 9.4% of raw materials remained, and a target compound was generated. The reaction solution was directly dried by rotary evaporation under reduced-pressure distillation to obtain a crude product. The reaction succeeded, and a white solid product BB-5 (3.57 g, crude product, hydrochloride) was obtained. MS (ESI) m/z: 402, 404 [M+H]⁺.

### Reference Example 6: Segment BB-6

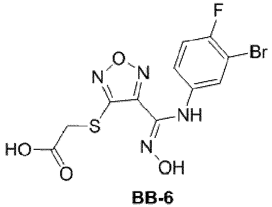

### Synthetic route:

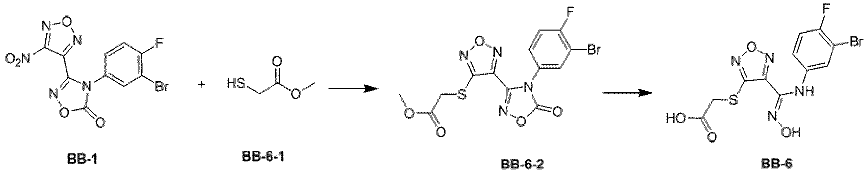

### Step 1: synthesis of compound BB-6-2

The compound BB-1 (200.00 mg, 537.55 umol, 1.00 *eq*) was dissolved in tetrahydrofuran (4.00 mL) and water (800.00 uL), and then sodium bicarbonate (112.90 mg, 1.34 mmol, 52.27 uL, 2.50 *eq*) and the compound BB-6-1 (68.47 mg, 645.06 umol, 58.52 uL, 1.20 *eq*) were added. The reaction solution was allowed to react at 15 °C for 14 hours. The reaction solution was combined with another batch of reaction solution in an amount of 30 mg and the combined reaction solution was concentrated to remove the tetrahydrofuran solvent, then diluted by addition of 5 mL of water, extracted with ethyl acetate (10 mL*3), and combined. Organic phases were dried over anhydrous sodium sulfate, and concentrated to obtain a crude product. The crude product was dissolved by 10 mL of ethyl acetate, mixing with silica gel, and separated by an automatic column chromatography device (petroleum ether:ethyl acetate=1:0∼3:1) to obtain a white solid product BB-6-2 (200.00 mg, yield: 71.90%). MS (ESI) m/z:431, 433 [M+H]⁺. ¹H NMR (400 MHz, CDCl₃) δ 7.66 (dd, J=5.77, 2.51Hz, 1H), 7.29-7.39 (m, 2H), 4.09 (s, 2H), 3.83 (s, 3H).

### Step 2: synthesis of compound BB-6

The compound BB-6-2 (100.00 mg, 231.92 umol, 1.00 *eq*) was dissolved in methanol (2.00 mL) and water (1.00 mL), to which sodium hydroxide (37.11 mg, 927.68 umol, 4.00 *eq*) was added. The reaction solution was allowed to react at 15 °C for 1.5 hours. The reaction solution was concentrated to remove the solvent, diluted by addition of 5 mL of water, extracted with ethyl acetate (5 mL*3), and combined and concentrated to obtain a liquid crude product as light yellow oil. Five mL of dichloromethane was added to the crude product, and the mixture was concentrated to remove the solvent and to obtain a white solid product BB-6 (90.00 mg, crude product) which was directly used for reaction in a next step. MS (ESI) m/z: 413, 415 [M+Na]⁺.

### Reference Example 7: Segment BB-7

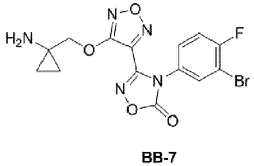

### Synthetic route:

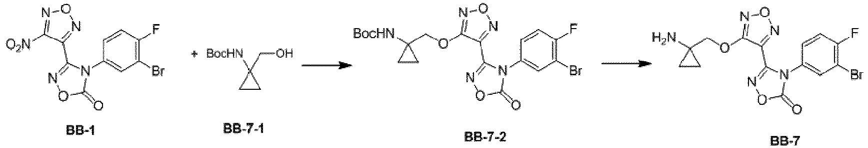

Taking compounds BB-1 and BB-7-1 as raw materials, BB-7 in the reference example was synthesized according to the synthesis steps 1-2 for the segment BB-4 in Reference Example 4. MS (ESI) m/z: 412, 414 [M+H]⁺.

### Reference Example 8: Segment BB-8

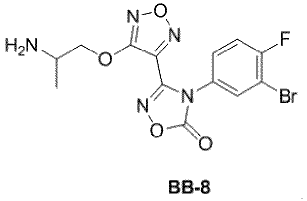

### Synthetic route:

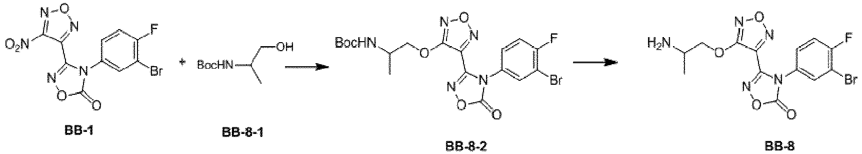

Taking the compounds BB-1 and BB-8-1 as raw materials, BB-8 in the reference example was synthesized according to the synthesis steps 1-2 for the segment BB-4 in Reference Example 4. MS (ESI) m/z: 400, 402 [M+H]⁺.

### Reference Example 9: Segment BB-9

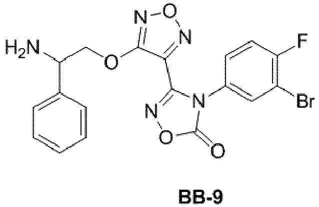

### Synthetic route:

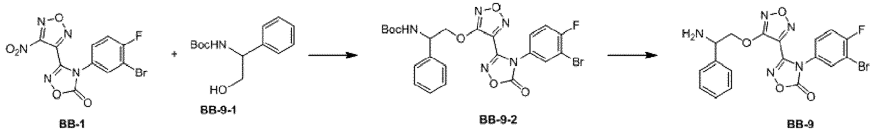

Taking compounds BB-1 and BB-9-1 as raw materials, BB-9 in the reference example was synthesized according to the synthesis steps 1-2 for the segment BB-4 in Reference Example 4. MS (ESI) m/z: 462, 464 [M+H]⁺.

### Reference Example 10: Segment BB-10

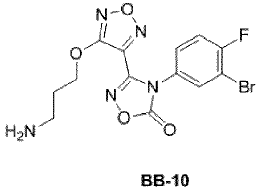

### Synthetic route:

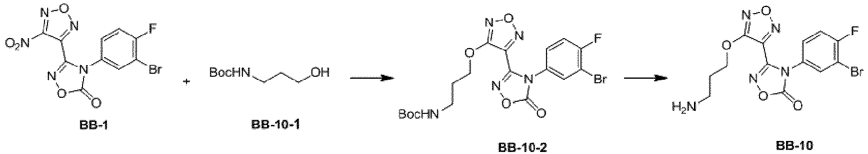

Taking compounds BB-1 and BB-10-1 as raw materials, BB-10 in the reference example was synthesized according to the synthesis steps 1-2 for the segment BB-4 in Reference Example 4. MS (ESI) m/z: 400, 402 [M+H]⁺.

### Reference Example 11: Segment BB-11

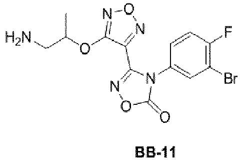

### Synthetic route:

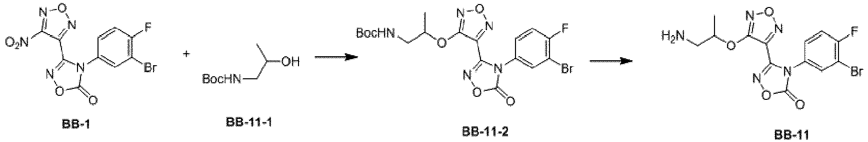

Taking compounds BB-1 and BB-11-1 as raw materials, BB-11 in the reference example was synthesized according to the synthesis steps 1-2 for the segment BB-4 in Reference Example 4. MS (ESI) m/z: 400, 402 [M+H]⁺.

### Reference Example 12: Segment BB-12

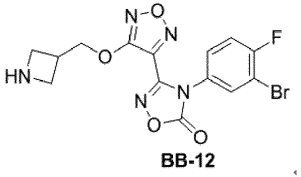

### Synthetic route:

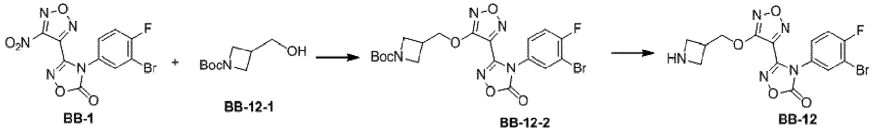

### Step 1: synthesis of compound BB-12-2

Taking compounds BB-1 and BB-11-1 as raw materials, a segment BB-12-2 was synthesized according to the synthesis step 1 for the segment BB-4 in Reference Example 4. MS (ESI) m/z: 534, 536 [M+Na]⁺.

### Step 2: synthesis of compound BB-12

The compound BB-12-2 (110.00 mg, 214.72 umol, 1.00 *eq*) was dissolved in dichloromethane (10.00 mL), to which trifluoroacetic acid (1.54 g, 13.51 mmol, 1.00 mL, 62.90 *eq*) was added, followed by stirring at 25 °C for 1 hour. No remaining raw materials are observed by LCMS monitoring and a desired product was generated. The reaction solution was adjusted to basic with pH of about 8∼9, and subsequently 100 milliliters of dichloromethane wad added. The resultant mixture was washed with water (30 mL*3). Organic phases were dried over anhydrous sodium sulfate, and concentrated under a reduced pressure created by a water pump to obtain a liquid product BB-12 as gray oil (85.00 mg, crude product) which was ready for next step without further purification. MS (ESI) m/z: 412, 414 [M+H]⁺.

### Reference Example 13: Segment BB-13

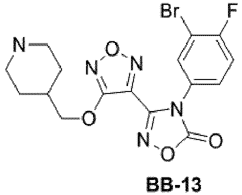

### Synthetic route:

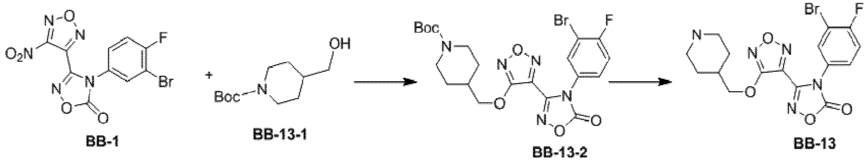

Taking compounds BB-1 and BB-13-1 as raw materials, BB-13 in the reference example was synthesized according to the synthesis steps 1-2 for the segment BB-4 in Reference Example 4. MS (ESI) m/z: 440, 442 [M+H]⁺.

### Reference Example 14: Segment BB-14

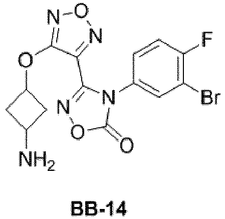

### Synthetic route:

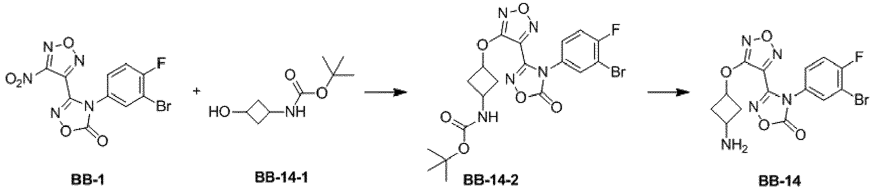

Taking compounds BB-1 and BB-14-1 as raw materials, BB-14 in the reference example was synthesized according to the synthesis steps 1-2 for the segment BB-4 in Reference Example 4. MS (ESI) m/z: 412, 414 [M+H]⁺.

### Reference Example 15: Segment BB-15

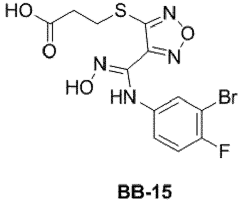

### Synthetic route:

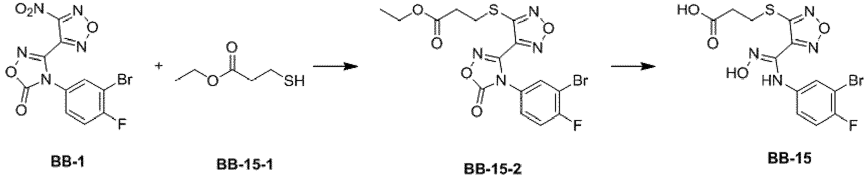

### Step 1: synthesis of compound BB-15-2

Taking compounds BB-1 and BB-15-1 as raw materials, a segment BB-15-2 was synthesized according to the synthesis step 1 for the segment BB-6 in Reference Example 6. MS (ESI) m/z: 459, 461 [M+H]⁺.

### Step 2: synthesis of compound BB-15

The compound BB-15-2 (400.00 mg, 870.99 umol, 1.00 *eq*) was dissolved in water (600.00 uL) and tetrahydrofuran (1.80 mL). Lithium hydroxide hydrate (73.09 mg, 1.74 mmol, 2.00 *eq*) was then added. The resultant mixture was stirred and reacted at 20 °C for 3 hours, whereby a product was generated albeit in a small amount. The reaction continued to stir and react for another 20 hours. The reaction solution was adjusted to pH of 6∼7 by addition of hydrochloric acid (6 M), and dried by rotary evaporation. Methanol (5 mL) was added, followed by filtration, isolation by high performance liquid chromatography (Phenomenex Synergi C18 150*30 mm*4 um, water (0.05% HCI)-ACN), and lyophilization. The target compound BB-15 was obtained as a yellow solid (60.00 mg, yield: 17.00%, purity:100%). MS (ESI) m/z: 405, 407 [M+H]⁺.

### Reference Example 16: Segment BB-16

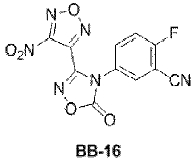

### Synthetic route:

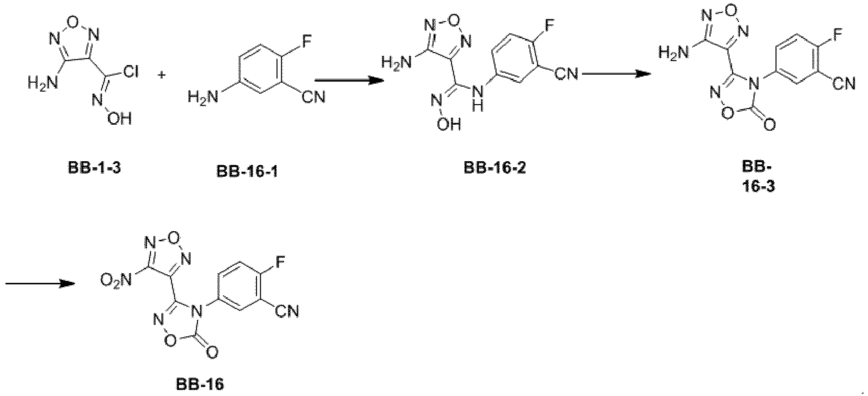

Taking compounds BB-1-3 and BB-16-1 as raw materials, a segment BB-16 was synthesized according to the synthesis steps 3-5 for the segment BB-1 in Reference Example 1. MS (ESI) m/z: 319 [M+H]⁺.

### Reference Example 17: Segment BB-17

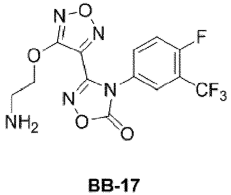

### Synthetic route:

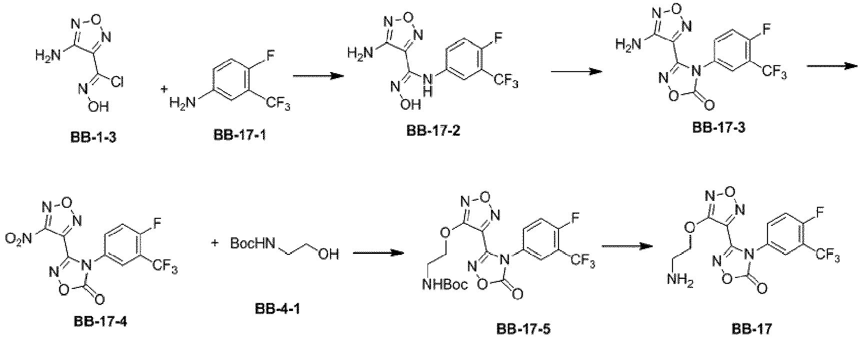

Taking compounds BB-1-3 and BB-17-1 as raw materials, a segment BB-17 was synthesized according to the synthesis steps 3-5 for the segment BB-1 in Reference Example 1, and the synthesis steps 1-2 for the segment BB-4. MS (ESI) m/z: 376 [M+H]⁺.

### Reference Example 18: Segment BB-18

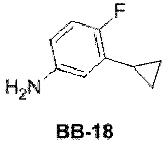

### Synthetic route:

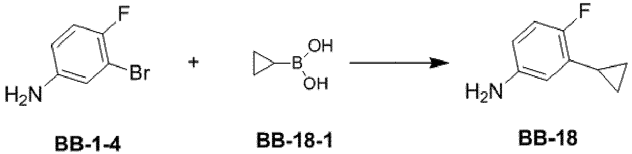

### Step 1: synthesis of compound BB-18

The compounds BB-1-4 (1.00 g, 5.26 mmol, 1.00 *eq*), BB-18-1 (587.38 mg, 6.84 mmol, 1.30 *eq*), potassium phosphate (3.91 g, 18.41 mmol, 3.50 *eq*), triphenylphosphine (137.96 mg, 526.00 umol, 0.10 *eq*), and palladium acetate (59.05 mg, 263.00 umol, 0.05 *eq*) were dissolved in toluene (24.00 mL) and water (2.00 mL), heated to 100 °C in nitrogen ambient and reacted for 16 hours. After being heated, the reaction solution gradually turned dark brown from brown. Complete reaction of raw materials was observed by LCMS monitoring and a target compound was generated. The reaction solution was cooled to 23 °C, followed by addition of 20 mL of water, extraction with ethyl acetate (20 mL*3), drying over anhydrous sodium sulfate, and filtration. A filtrate was dried by rotary evaporation under reduced-pressure distillation, and purified by flash silica gel column chromatography (petroleum ether:ethyl acetate=10:1). The reaction succeeded, and a yellow liquid product BB-18 (790.00 mg, yield: 99.35%) was obtained. MS (ESI) m/z: 152 [M+H]⁺.

### Reference Example 19: Segment BB-19

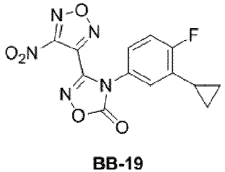

### Synthetic route:

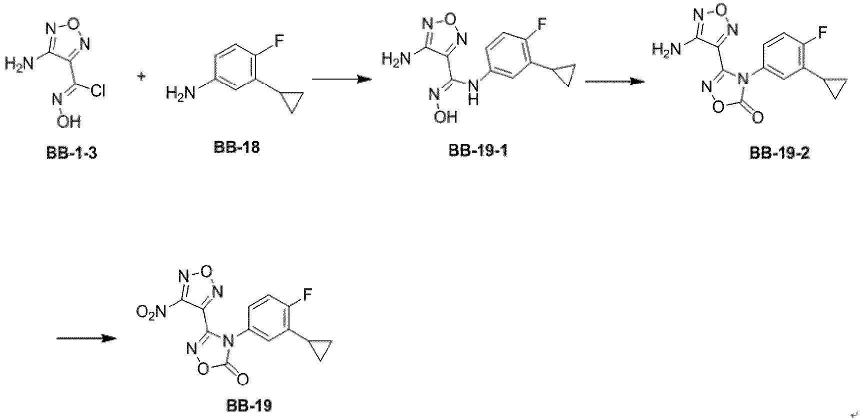

Taking the compounds BB-1-3 and BB-18 as raw materials, a segment BB-19 was synthesized according to the synthesis steps 3-5 for the segment BB-1 in Reference Example 1. MS (ESI) m/z: 334 [M+H]⁺.

### Reference Example 20: Segment BB-20

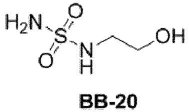

### Synthetic route:

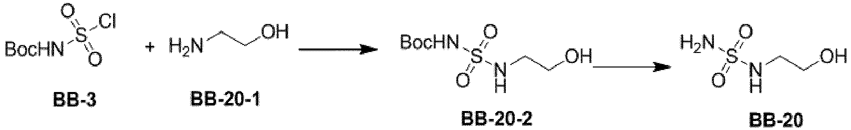

### Step 1: synthesis of compound BB-20-2

At 0 °C, to a dichloromethane solution (20 mL) of the compound BB-3 (5.39 g, 24.99 mmol, 1.00 *eq*), triethylamine (7.59 g, 75.03 mmol, 10.40 mL, 3.00 *eq*) was added. After the mixture was stirred at 0 °C for 30 minutes, the compound BB-20-1 (1.60 g, 26.13 mmol, 1.58 mL, 1.05 *eq*) was added. The mixed solution was heated to 23 °C and reacted for 16 hours. Complete reaction of raw materials was observed by TLC (petroleum ether:ethyl acetate=1:1) monitoring. The reaction solution was then subjected to rotary evaporation under reduced-pressure distillation to remove dichloromethane, adjusted to pH=5 with 1 M hydrochloric acid, and extracted with ethyl acetate (20 mL*3). Organic phases were combined, dried over anhydrous sodium sulfate, and filtered. A filtrate was dried by rotary evaporation under reduced-pressure distillation to obtain a white solid product BB-20-2 (5.44 g, crude product). ¹H NMR (400 MHz, CDCl₃) *δ* 7.83 (s, 1H) 5.88 (t, 1H) 3.73-3.80 (t, 2H) 3.26 (q, 2H) 1.48-1.50 (m, 9H).

### Step 2: synthesis of compound BB-20

The compound BB-20-2 (2.00 g, 8.32 mmol, 1.00 *eq*) was dissolved in dichloromethane (10.00 mL), and hydrochloric acid/dioxane (4 M, 10.00 mL, 4.81 *eq*) was added. The mixed solution was allowed to react at 24 °C for 2 hours. Complete reaction of raw materials was observed by TLC (petroleum ether:ethyl acetate=1:1) monitoring. The reaction solution was then directly dried by rotary evaporation under reduced-pressure distillation to obtain a brown liquid product BB-20 (1.10 g, crude product). ¹H NMR (400 MHz, DMSO-*d*₆) *δ* 6.49 (s, 2H) 3.81-4.19 (s, 1H) 3.43-3.52 (t, 2H) 2.91-2.99 (t, 2H).

### Reference Example 21: Segment BB-21

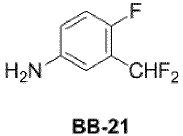

### Synthetic route:

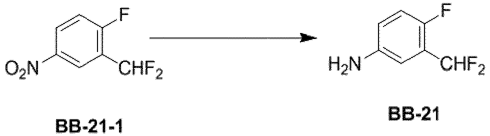

### Step 1: synthesis of compound BB-21

A compound BB-21-1 (2.00 g, 10.47 mmol, 1.00 eq) was dissolved in methanol (20.00 mL), and then 10% carbon-supported palladium (200.00 mg) was added. The reaction solution was allowed to react at 25 °C in 15 Psi hydrogen gas for 16 hours. LCMS showed that reactant 1 was completely consumed and a product peak appeared. The reaction solution was filtered. A filtrate was dried by rotary evaporation to obtain a product BB-21 as yellow oil (1.60 g, crude product) which was directly used for reaction in a next step. MS (ESI) m/z: 162 [M+H]⁺.

### Reference Example 22: Segment BB-22

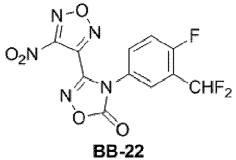

### Synthetic route:

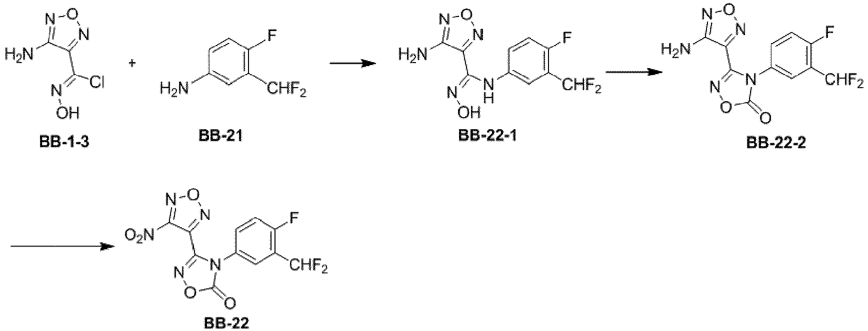

Taking the compounds BB-1-3 and BB-21 as raw materials, a segment BB-22 was synthesized according to the synthesis steps 3-5 for the segment BB-1 in Reference Example 1. MS (ESI) m/z: 344 [M+H]⁺.

### Reference Example 23: Segment BB-23

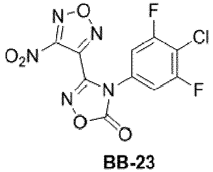

### Synthetic route:

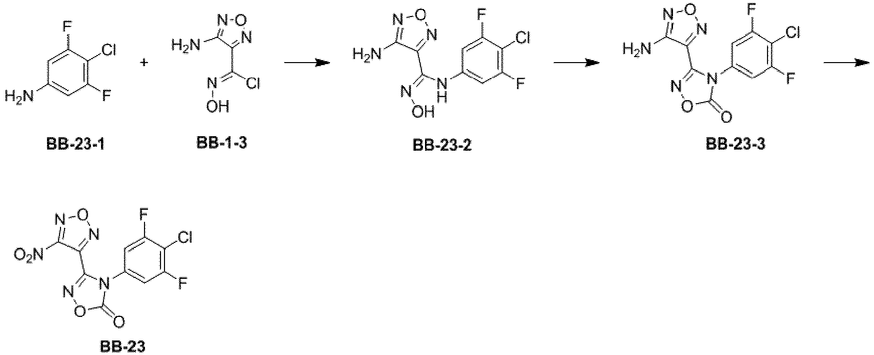

Taking compounds BB-1-3 and BB-23-1 as raw materials, a segment BB-23 was synthesized according to the synthesis steps 3-5 for the segment BB-1 in Reference Example 1. MS (ESI) m/z: 346 [M+H]⁺.

### Reference Example 24: Segment BB-24

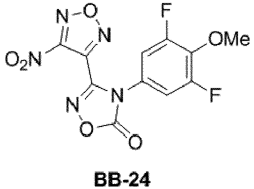

### Synthetic route:

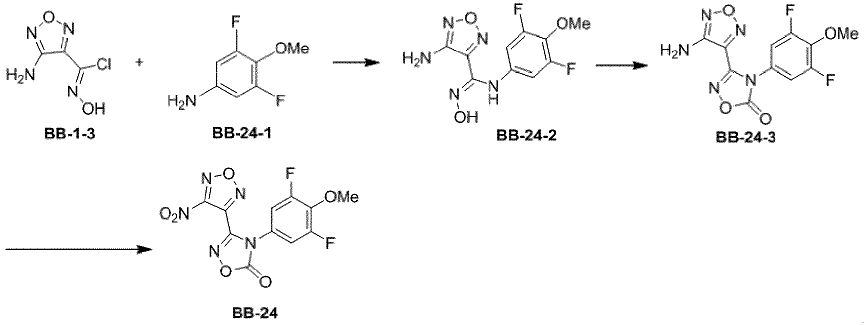

Taking compounds BB-1-3 and BB-24-1 as raw materials, a segment BB-24 was synthesized according to the synthesis steps 3-5 for the segment BB-1 in Reference Example 1. MS (ESI) m/z: 342 [M+H]⁺.

### Reference Example 25: Segment BB-25

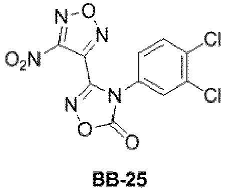

### Synthetic route:

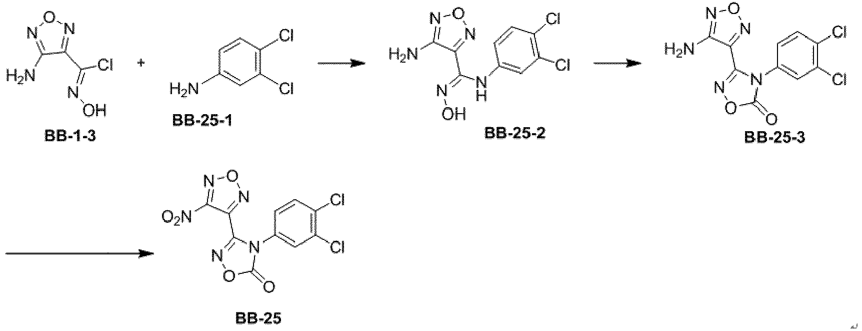

Taking compounds BB-1-3 and BB-25-1 as raw materials, a segment BB-25 was synthesized according to the synthesis steps 3-5 for the segment BB-1 in Reference Example 1. MS (ESI) m/z: 344 [M+H]⁺.

### Reference Example 26: Segment BB-26

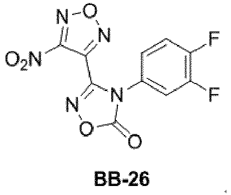

### Synthetic route:

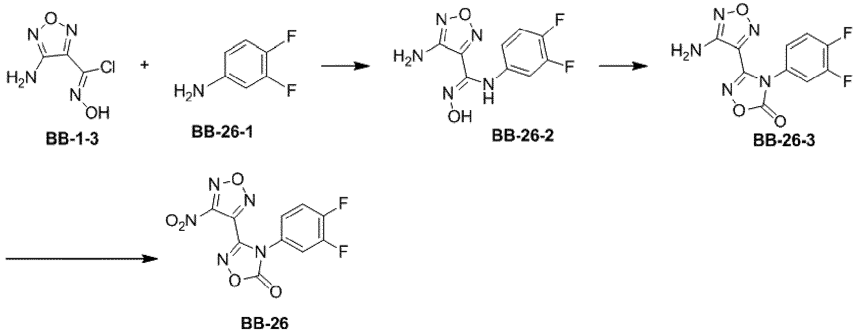

Taking compounds BB-1-3 and BB-26-1 as raw materials, a segment BB-26 was synthesized according to the synthesis steps 3-5 for the segment BB-1 in Reference Example 1. MS (ESI) m/z: 312 [M+H]⁺.

### Reference Example 27: Segment BB-27

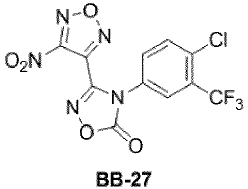

### Synthetic route:

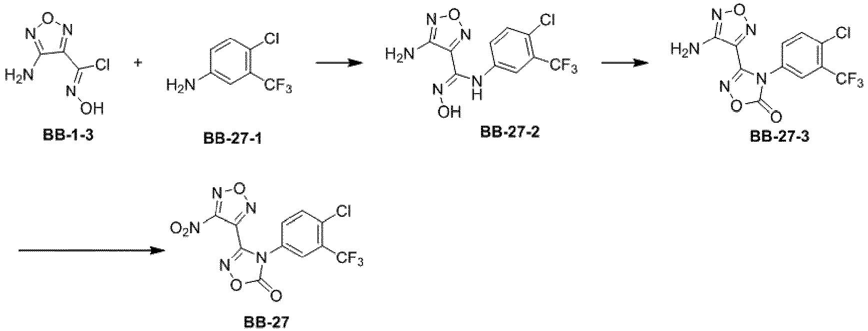

Taking compounds BB-1-3 and BB-27-1 as raw materials, a segment BB-27 was synthesized according to the synthesis steps 3-5 for the segment BB-1 in Reference Example 1. MS (ESI) m/z: 378 [M+H]⁺.

### Reference Example 28: Segment BB-28

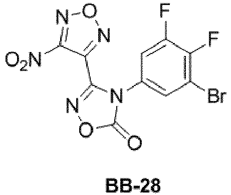

### Synthetic route:

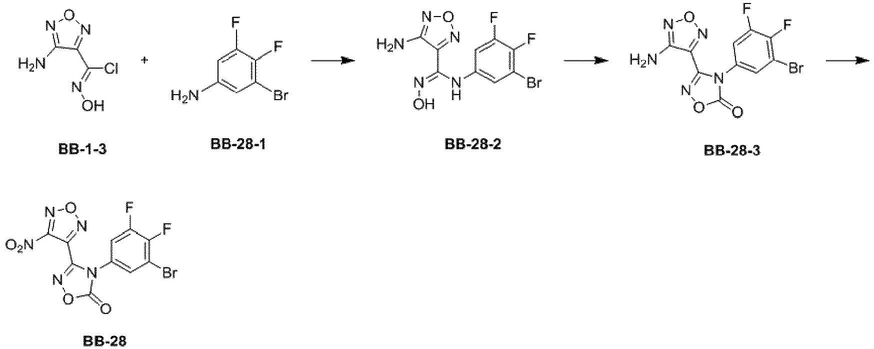

Taking compounds BB-1-3 and BB-28-1 as raw materials, a segment BB-28 was synthesized according to the synthesis steps 3-5 for the segment BB-1 in Reference Example 1. MS (ESI) m/z: 390, 392 [M+H]⁺.

### Reference Example 29: Segment BB-29

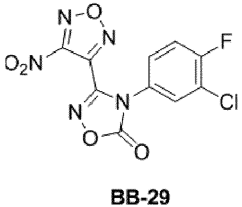

### Synthetic route:

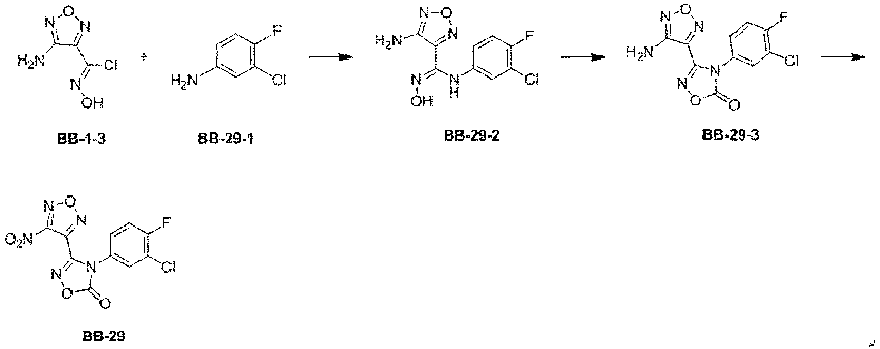

Taking compounds BB-1-3 and BB-29-1 as raw materials, a segment BB-29 was synthesized according to the synthesis steps 3-5 for the segment BB-1 in Reference Example 1. MS (ESI) m/z: 328 [M+H]⁺.

### Reference Example 30: Segment BB-30

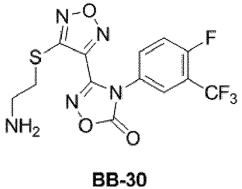

### Synthetic route:

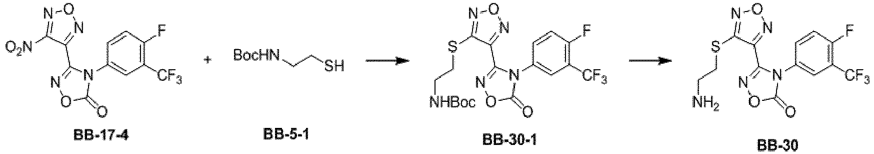

Taking the compounds BB-17-4 and BB-5-1 as raw materials, BB-30 in the reference example was synthesized according to the synthesis steps 1-2 for the segment BB-5 in Reference Example 5. MS (ESI) m/z: 392 [M+H]⁺.

### Reference Example 31: Segment BB-31

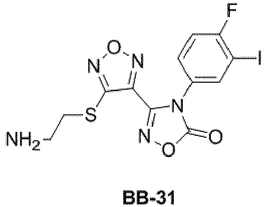

### Synthetic route:

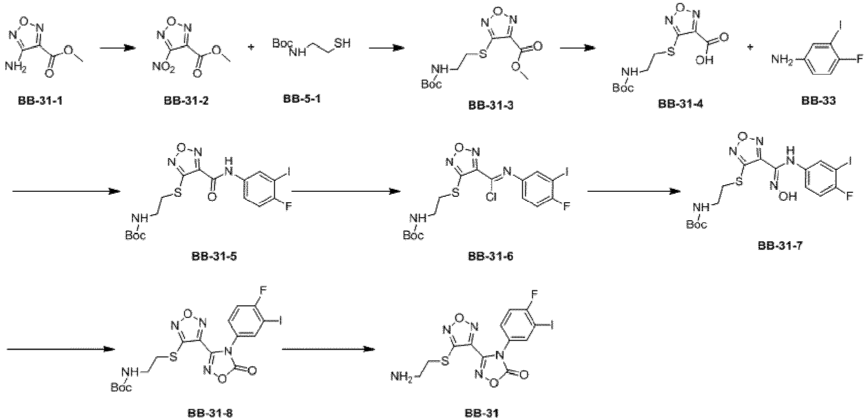

### Step 1: synthesis of compound BB-31-2

At 0 °C, sulfuric acid (30.00 mL) was slowly added to hydrogen peroxide (35.40 g, 312.26 mmol, 30.00 mL, 30% purity, 27.93 *eq*), then sodium tungstate (3.28 g, 11.18 mmol, 1.00 *eq*) was added, to which the compound BB-31-1 (1.60 g, 11.18 mmol, 1.00 *eq*) was added. The resultant mixture was heated to 15 °C and reacted for 3 hours. With LCMS monitoring showing 1.88% of raw materials remaining, 30 mL of water was added to the reaction solution, followed by extraction with ethyl acetate (50 mL*3). Organic phases were combined, washed with water (50 mL*3), washed with a saturated sodium chloride solution (50 mL*3), dried over anhydrous sodium sulfate, and filtered. A filtrate was dried by rotary evaporation under reduced-pressure distillation to obtain a yellow liquid product BB-31-2 (1.40 g, yield: 72.35%, crude product). ¹H NMR (400 MHz, DMSO-*d*₆) δ 4.00-4.02 (s, 3H).

### Step 2: synthesis of compound BB-31-3

The compound BB-31-2 (1.40 g, 8.09 mmol, 1.00 *eq*) was dissolved in tetrahydrofuran (10.00 mL) and water (1.00 mL), the compound BB-5-1 (1.58 g, 8.90 mmol, 1.10 *eq*) was added, and then sodium bicarbonate (1.36 g, 16.18 mmol, 629.63 uL, 2.00 *eq*) was added. The mixed solution was allowed to react at 15 °C for 2 hours. With LCMS monitoring showing completion of reaction of raw materials, 10 mL of water was added, followed by extraction with ethyl acetate (15 mL*3). Organic phases were combined, dried over anhydrous sodium sulfate, and filtered. A filtrate was dried by rotary evaporation under reduced-pressure distillation. Purification was performed by flash silica gel column chromatography (petroleum ether:ethyl acetate=4:1). The reaction succeeded, and a light pink solid product BB-31-3 (2.57 g, yield: 96.28%, purity: 91.93%) was obtained. MS (ESI) m/z: 304 [M+H]⁺.

### Step 3: synthesis of compound BB-31-4

The compound BB-31-3 (2.57 g, 8.47 mmol, 1.00 eq) was dissolved in tetrahydrofuran (10.00 mL) and water (5.00 mL), and lithium hydroxide hydrate (888.50 mg, 21.18 mmol, 2.50 eq) was added. The reaction solution was allowed to react at 15 °C for 1 hour. With LCMS monitoring showing completion of reaction of raw materials, 5 mL of water was added. The resultant mixture was adjusted to have pH=6 with concentrated hydrochloric acid, and extracted with ethyl acetate (15 mL*3). Organic phases were combined, dried over anhydrous sodium sulfate, and filtered. A filtrate was dried by rotary evaporation under reduced-pressure distillation. The reaction succeeded, and a light yellow solid product BB-31-4 (2.70 g, crude product) was obtained. MS (ESI) m/z: 290 [M+H]⁺.

### Step 4: synthesis of compound BB-31-5

The compound BB-31-4 (590.00 mg, 2.04 mmol, 1.00 *eq*) was added to *N,N*-dimethylformamide (10.00 mL), then the compound BB-33 (531.68 mg, 2.24 mmol, 1.10 *eq*), HATU (930.50 mg, 2.45 mmol, 1.20 *eq*), and diisopropylethylamine (527.13 mg, 4.08 mmol, 712.33 uL, 2.00 *eq*) were added. The reaction solution was allowed to react at 15 °C for 2 hours. With LCMS showing completion of the reaction, the reaction solution was adjusted to ∼5 in pH value by addition of 1 N hydrochloric acid, extracted with ethyl acetate (30 mL×2), dried over anhydrous sodium sulfate, and filtered. A filtrate was dried by rotary evaporation to obtain a product BB-31-5 as yellow oil (850.00 mg, yield: 81.97%). MS (ESI) m/z: 531 [M+Na]⁺.

### Step 5: synthesis of compound BB-31-6

The compound BB-31-5 (100.00 mg, 196.73 umol, 1.00 *eq*) was added to toluene (3.00 mL), and then pyridine (147.00 mg, 1.86 mmol, 150.00 uL, 9.45 *eq*), and phosphorus pentachloride (81.93 mg, 393.46 umol, 2.00 *eq*) were added. The reaction solution was allowed to react at 80 °C for 2 hours. With TLC (petroleum ether:ethyl acetate=4:1) showing completion of consumption of a reactant 1 and generation of a main product peak, the reaction solution was dried by rotary evaporation to obtain a yellow solid product BB-31-6 (105.00 mg, crude product).

### Step 6: synthesis of compound BB-31-7

The compound BB-31-6 (100.00 mg, 189.84 umol, 1.00 *eq*) was added to ethanol (3.00 mL), and cooled to 0 °C, and then 50% hydroxylamine aqueous solution (251.03 mg, 3.80 mmol, 20.02 *eq*) was added. The reaction solution was allowed to react at 0 °C for 2 hours. With LCMS showing completion of the reaction, the reaction solution was extracted with ethyl acetate (30 mL×2), dried over anhydrous sodium sulfate, and filtered. A filtrate was dried by rotary evaporation to obtain a crude product as yellow oil. The crude product was separated (ethyl acetate) through a thick preparative plate and purified to obtain a white solid product BB-31-7 (70.00 mg, yield: 64.82%, purity: 92%). MS (ESI) m/z: 546 [M+Na]⁺.

### Step 7: synthesis of compound BB-31-8

The compound BB-31-7 (80.00 mg, 152.87 umol, 1.00 *eq*) was added to tetrahydrofuran (3.00 mL), and then carbonyl diimidazole (27.27 mg, 168.16 mmol, 1.10 *eq*) was added. The reaction solution was allowed to react at 60 °C for 1 hour. With LCMS showing completion of reaction, the reaction solution was diluted by addition of ethyl acetate (50 mL), washed with a saturated saline (10 mL), dried over anhydrous sodium sulfate, and filtered. A filtrate was dried by rotary evaporation to obtain a yellow solid product BB-31-8 (80.00 mg, crude product). MS (ESI) m/z: 550 [M+H]⁺.

### Step 8: synthesis of compound BB-31

The compound BB-31-8 (80.00 mg, 145.64 umol, 1.00 eq) was added to dichloromethane (2.00 mL), and then hydrochloric acid/dioxane (4 M, 1.90 mL, 52.31 *eq*) was added. The reaction solution was allowed to react at 15 °C for 2 hours. With LCMS showing completion of reaction, the reaction solution was dried by rotary evaporation to obtain a product BB-31 as yellow oil (71.00 mg, crude product, hydrochloride). MS (ESI) m/z: 450 [M+H]⁺.

### Reference Example 32: Segment BB-32

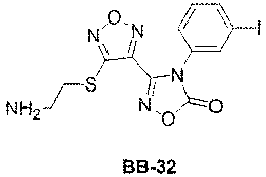

### Synthetic route:

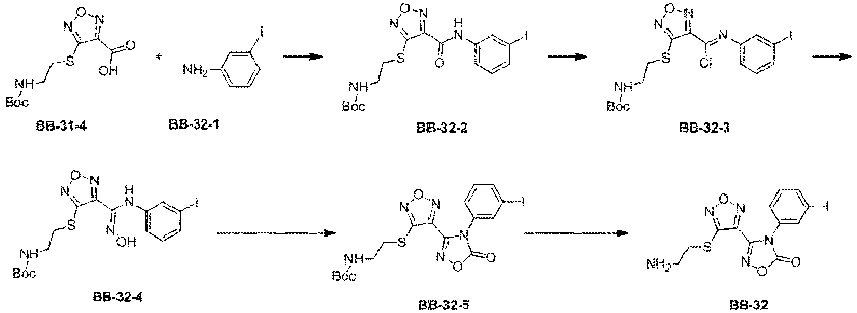

Taking the compounds BB-31-4 and BB-32-1 as raw materials, BB-32 in the reference example was synthesized according to the synthesis steps 4-8 for the segment BB-31 in Reference Example 31. MS (ESI) m/z: 432 [M+H]⁺.

### Reference Example 33: Segment BB-33

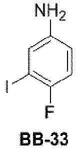

### Synthetic route:

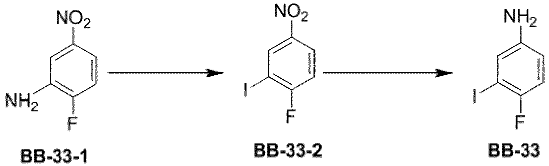

### Step 1: synthesis of compound BB-33-2

The compound BB-33-1 (1.20 g, 7.69 mmol, 1.00 *eq*) was dissolved in hydrochloric acid (4.00 mL), and cooled to 0 °C. Sodium nitrite (583.67 mg, 8.46 mmol, 459.58 uL, 1.10 *eq*) was dissolved in 2.6 mL of water, and added dropwise to the reaction solution. After being stirred for 15 minutes, the mixed solution was slowly added to an aqueous solution (16 mL) of potassium iodide (4.47 g, 26.92 mmol, 3.50 *eq*), and heated to 10 °C and stirred for 16 hours. With TLC (petroleum ether:ethyl acetate=10:1) monitoring showing completion of reaction of raw materials, a target compound was generated. The resulting compound was diluted by addition of ethyl acetate (30 mL), followed by washing with 10% sodium hydroxide (25 mL*2), washing with 5% sodium sulfite (25 mL*2), drying over anhydrous sodium sulfate, and filtration. A filtrate was dried by rotary evaporation under reduced-pressure distillation. Purification was performed by flash silica gel column chromatography (petroleum ether:ethyl acetate=10:1). The reaction succeeded, and a yellow solid product BB-33-2 (550.00 mg, yield: 21.22%, purity: 79.22%) was obtained. ¹H NMR (400 MHz, CDCl₃) δ 8.60 (dd, *J*=5.3, 2.8Hz, 1H), 8.19 (ddd, *J*=9.0, 4.3, 2.8Hz, 1H), 7.09-7.17 (m, 1H).

### Step 2: synthesis of compound BB-33

The compound BB-33-2 (1.45 g, 5.43 mmol, 1.00 *eq*) was dissolved in acetic acid (10.00 mL) and ethanol (10.00 mL), and an iron powder (1.52 g, 27.15 mmol, 5.00 eq) was added. The reaction solution was allowed to react at 60 °C for 20 minutes. Complete reaction of raw materials was observed by LCMS monitoring, and a target compound was generated. The reaction solution was filtered. A filtrate was dried by rotary evaporation under reduced-pressure distillation, dissolved in 40 mL of ethyl acetate, washed with saturated sodium bicarbonate (30 mL*3), dried over anhydrous sodium sulfate, and filtered. A filtrate was dried by rotary evaporation under reduced-pressure distillation. Purification was performed by flash silica gel column chromatography (petroleum ether:ethyl acetate=4:1). The reaction succeeded, and a brown liquid product BB-33 (900.00 mg, yield: 53.92%, purity: 77.1%) was obtained. MS (ESI) m/z: 238 [M+H]⁺.

### Reference Example 34: Segment BB-34

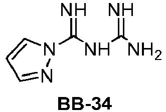

### Synthetic route:

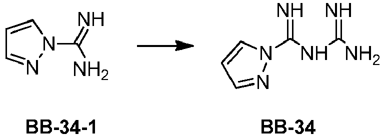

### Step 1: synthesis of compound BB-34

The compound BB-34-1 (293.00 mg, 2.00 mmol, 1.00 *eq,* HCI) was dissolved in DMF (500.00 uL), and DIEA (258.49 mg, 2.00 mmol, 349.31 uL, 1.00 *eq*) was added. The reaction solution was allowed to react at 25 °C for 64 hr, and the reaction solution gradually turned into a white turbid solution to obtain a DMF solution (0.5 ml) of segment BB-34 (200.00 mg, crude, HCI).

### Example 1: Compound 0052

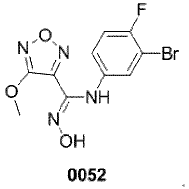

### Synthetic route:

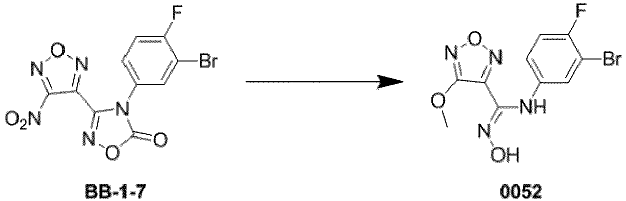

### Step 1: synthesis of compound 0052

A compound BB-1-7 (300.00 mg, 806.32 umol, 1.00 *eq*) was dissolved in methanol (4.00 mL). Sodium hydroxide (129.01 mg, 3.23 mmol, 4.00 *eq*) was dissolved in water (1.00 mL) and added to the reaction solution. After the addition of sodium hydroxide, the reaction solution turned yellow from colorless, and a solid precipitated. Upon continuous stirring, the solid gradually disappeared. The reaction was carried out at a room temperature for 16 hours. Complete reaction of raw materials was observed by LCMS monitoring, and a desired compound was generated. The resulting compound mixture was adjusted to pH=5 with 1 M hydrochloric acid, followed by rotary evaporation under reduced-pressure distillation to remove methanol, extraction with ethyl acetate (10 mL*3), drying over anhydrous sodium sulfate, and filtration. A filtrate was dried by rotary evaporation under reduced-pressure distillation, and dissolved in methanol, and filtered. A filtrate was separated by high performance liquid chromatography (Phenomenex Synergi C18 150*30 mm*4 um water (0.05% HCI)-ACN), to obtain a product 0052 (167.72 mg, yield: 62.82%, purity: 100%). MS (ESI) m/z: 331, 333 [M+H]⁺. ¹H NMR (400 MHz, CD₃OD) *δ* 7.10-7.15 (m, 1H), 7.06 (t, 1H), 6.77-6.81 (m, 1H), 3.95 (s, 3H).

### Example 2: Compound 0103

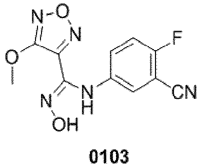

### Synthetic route:

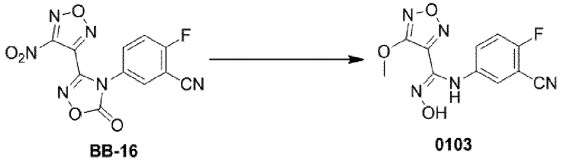

Taking a compound BB-16 as raw material, a compound 0103 was synthesized according to the synthesis step 1 for the compound 0052 in Example 1. MS (ESI) m/z: 278 [M+H]⁺. ¹H NMR (400 MHz, DMSO-*d*₆) *δ* 11.69 (s, 1H), 9.14 (s, 1H), 7.34 (t, 1H), 7.23 (dd, 1H), 7.07-7.17 (m, 1H), 4.00 (s, 2H).

### Example 3: Compound 0124

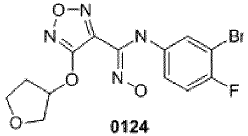

### Synthetic route:

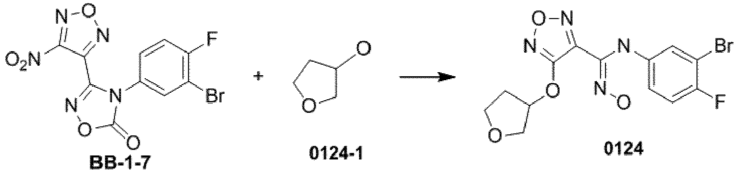

### Step 1: synthesis of compound 0124

The compound BB-1-7 (50.00 mg, 134.39 umol, 1.00 *eq*) was added to water (100.00 uL) and tetrahydrofuran (4.00 mL), and then the compound 0124-1 (23.68 mg, 268.78 umol, 21.73 uL, 2.00 *eq*) and sodium hydroxide (21.50 mg, 537.56 umol, 4.00 *eq*) were added. The reaction solution was stirred at 25 °C for 16 hours. With LCMS showing completion of consumption of a reactant 1 and generation of a main product peak, the reaction solution was adjusted to pH=5 with 6 M hydrochloric acid, and filtered. A filtrate was purified by preparative high performance liquid chromatography (column: Boston Green ODS 150*30 5u; mobile phase: [water (0.05% HCl)-ACN]; B%:40%-70%, 10 min) to obtain a product 0124 (35.00 mg, yield: 61.48%, purity: 100%, hydrochloride). MS (ESI) m/z: 387, 389 [M+H]⁺. ¹H NMR (400 MHz, CD₃OD):*δ*=7.19-7.11 (m, 1H), 7.10-7.01 (m, 1H), 6.82-6.72 (m, 1H), 5.19-5.09 (m, 1H), 3.88-3.63 (m, 4H), 2.25-2.13 (m, 1H), 1.92-1.81 (m, 1H).

Various examples in the following table were synthesized according to the synthetic method of step 1 in Example 3 (compound 0124):

| **Example** | **Structure** | **Segment 1** | **Segment 2** | **MS m/z** | **Compound** |
|---|---|---|---|---|---|
| **4** | 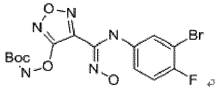 | 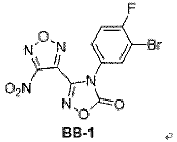 | 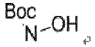 | 432 | **0078** |
| | | | | 434 | |
| | | | | [M+H]⁺ | |
| **5** | 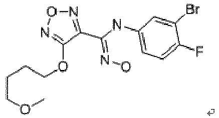 | 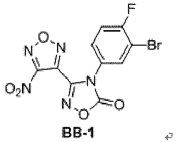 | 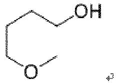 | 403 | **0128** |
| | | | | 405 | |
| | | | | [M+H]⁺ | |
| **6** | 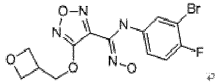 | 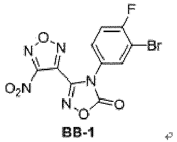 | 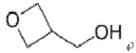 | 387 | **0129** |
| | | | | 389 | |
| | | | | [M+H]⁺ | |
| **7** | 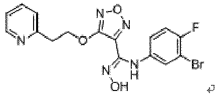 | 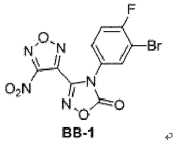 | 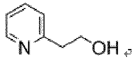 | 422 | **0131** |
| | | | | 424 | |
| | | | | [M+H]⁺ | |
| **8** | 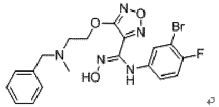 | 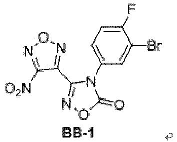 | 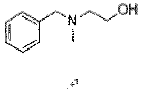 | 464 | **0133** |
| | | | | 466 | |
| | | | | [M+H]⁺ | |
| **9** | 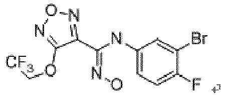 | 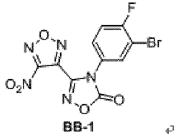 | 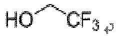 | 400 | **0135** |
| | | | | 402 | |
| | | | | [M+H]⁺ | |
| **10** | 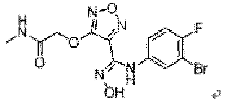 | 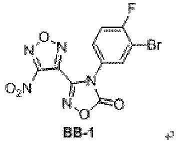 | 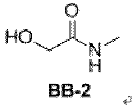 | 389 | **0139** |
| | | | | 491 | |
| | | | | [M+H]⁺ | |
| **11** | 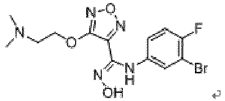 | 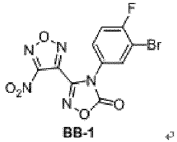 | 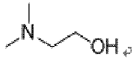 | 389 | **0140** |
| | | | | 491 | |
| | | | | [M+H]⁺ | |
| **12** | 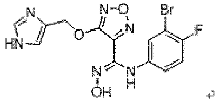 | 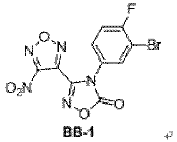 | 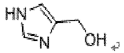 | 397 | **0143** |
| | | | | 399 | |
| | | | | [M+H]⁺ | |
| **13** | 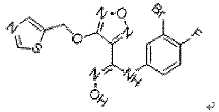 | 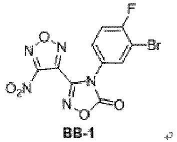 | 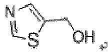 | 414 | **0144** |
| | | | | 416 | |
| | | | | [M+H]⁺ | |
| **14** | 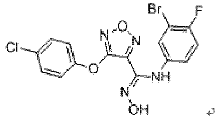 | 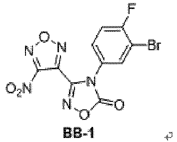 | 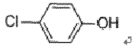 | 427 | **0150** |
| | | | | 429 | |
| | | | | [M+H]⁺ | |
| **15** | 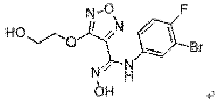 | 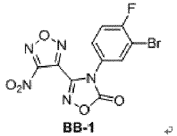 | 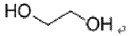 | 361 | **0177** |
| | | | | 363 | |
| | | | | [M+H]⁺ | |
| **16** | 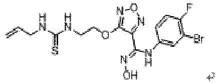 | 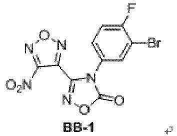 | 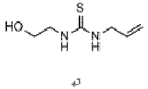 | 459 | **0178** |
| | | | | 461 | |
| | | | | [M+H]⁺ | |
| **17** | 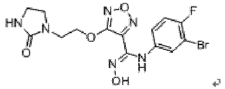 | 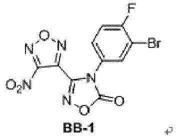 | 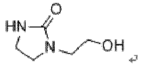 | 429 | **0179** |
| | | | | 431 | |
| | | | | [M+H]⁺ | |
| **18** | 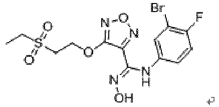 | 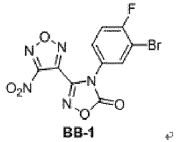 | 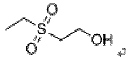 | 437 | **0180** |
| | | | | 439 | |
| | | | | [M+H]⁺ | |
| **19** | 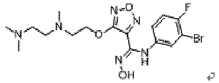 | 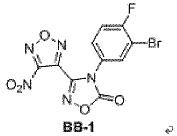 | 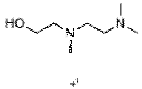 | 445 | **0182** |
| | | | | 447 | |
| | | | | [M+H]⁺ | |
| **20** | 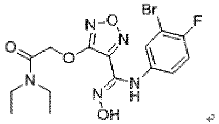 | 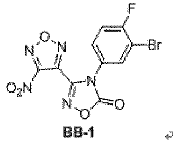 | 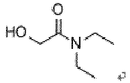 | 430 | **0183** |
| | | | | 432 | |
| | | | | [M+H]⁺ | |
| **21** | 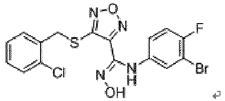 | 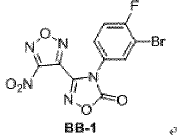 | 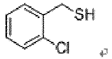 | 457 | **0134** |
| | | | | 459 | |
| | | | | [M+H]⁺ | |
| **22** | 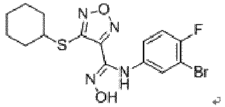 | 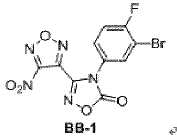 | 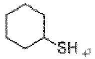 | 415 | **0137** |
| | | | | 417 | |
| | | | | [M+H]⁺ | |
| **23** | 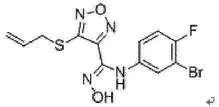 | 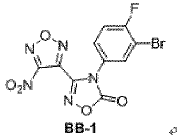 | 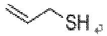 | 373 | **0138** |
| | | | | 375 | |
| | | | | [M+H]⁺ | |
| **24** | 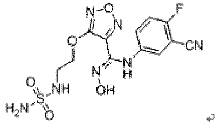 | 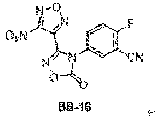 | 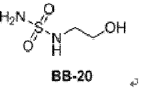 | 386 | **0120** |
| | | | | [M+H]⁺ | |
| **25** | 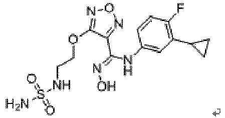 | 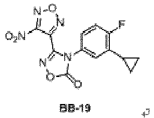 | 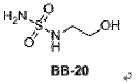 | 401 | **0122** |
| | | | | [M+H]⁺ | |
| **26** | 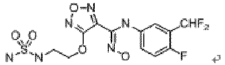 | 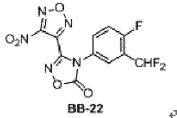 | 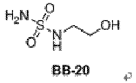 | 411 | **0151** |
| | | | | [M+H]⁺ | |
| **27** | 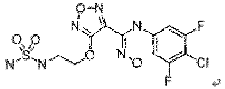 | 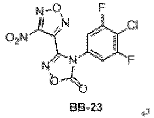 | 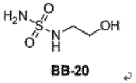 | 413 | **0156** |
| | | | | [M+H]⁺ | |
| **28** | 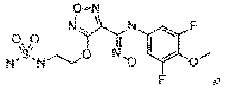 | 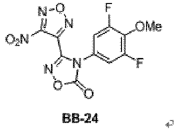 | 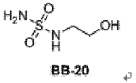 | 409 | **0158** |
| | | | | [M+H]⁺ | |
| **29** | 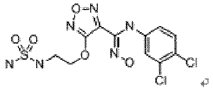 | 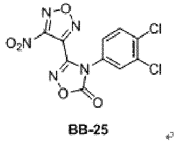 | 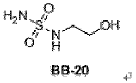 | 411 | **0160** |
| | | | | [M+H]⁺ | |
| **30** | 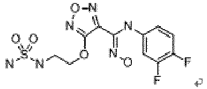 | 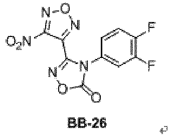 | 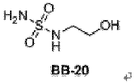 | 379 | **0162** |
| | | | | [M+H]⁺ | |
| **31** | 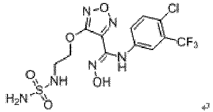 | 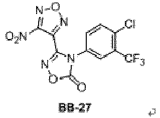 | 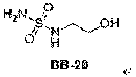 | 445 | **0184** |
| | | | | [M+H]⁺ | |
| **32** | 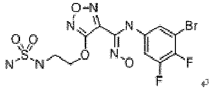 | 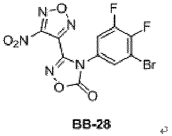 | 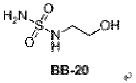 | 457 | **0189** |
| | | | | 459 | |
| | | | | [M+H]⁺ | |
| **33** | 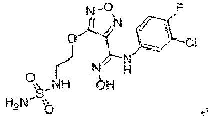 | 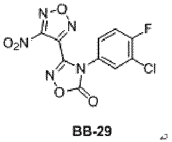 | 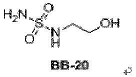 | 395 | **0190** |
| | | | | [M+H]⁺ | |

### Examples 34: 0026

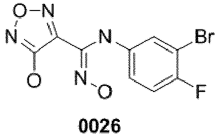

### Synthetic route:

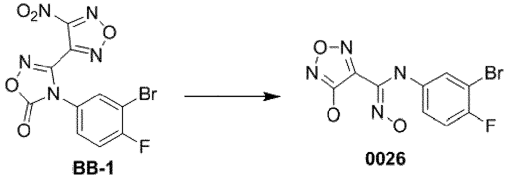

### Step 1: synthesis of compound 0026

The compound BB-1 (100.00 mg, 268.77 umol,1.00 eq) was dissolved in tetrahydrofuran (2.00 mL) and water (1.00 mL), then sodium hydroxide (43.00 mg, 1.08 mmol, 4.00 eq) was added. The resultant mixture was stirred at 25 °C for 2 hours. The reaction solution was dried by rotary evaporation to obtain a crude product as yellow oil. The crude product was purified by preparative high performance liquid chromatography (column: Boston Green ODS 150*30 5u, condition: water (0.05% HCl)-ACN) to obtain a product 0026 (15.00 mg, yield: 17.60%, purity: 100%). MS (ESI) m/z: 317, 319 [M+H]⁺. ¹H NMR (400 MHz, CD₃OD): δ 7.25-7.13 (m, 1H), 7.12-6.99 (m, 1H), 6.94-6.77 (m, 1H).

### Examples 35: 0077

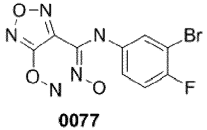

### Synthetic route:

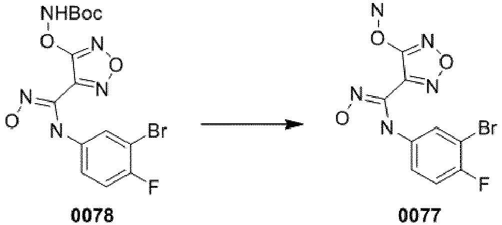

### Step 1: synthesis of compound 0077

The compound 0078 (20.00 mg, 46.27 umol, 1.00 *eq*) was dissolved in dichloromethane (500.00 uL), then trifluoroacetic acid (256.69 mg, 2.25 mmol, 166.68 uL, 48.65 *eq*) was added, and then the resultant mixture was stirred at 25 °C for 1 hour. With LCMS showing completion of most of reaction, the reaction solution was adjusted to pH=7 by addition of a saturated sodium bicarbonate solution, and filtered. A filtrate was dried by rotary evaporation to obtain a crude product. The crude product was purified by preparative high performance liquid chromatography (water (0.05% ammonia hydroxide v/v)-ACN, column: DuraShell 150*25 mm*5 um) to obtain a product 0077 (8.00 mg, yield: 51.16%, purity: 98.27%). MS (ESI) m/z: 332, 334 [M+H]⁺. ¹H NMR (400 MHz, CDCl₃): *δ* 7.21-7.15 (m, 1H), 7.08-6.97 (m, 2H), 6.85-6.79 (m, 1H), 6.76-6.67 (m, 2H).

### Examples 36: 0147

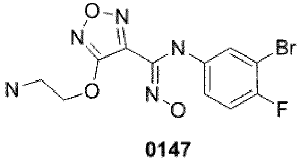

### Synthetic route:

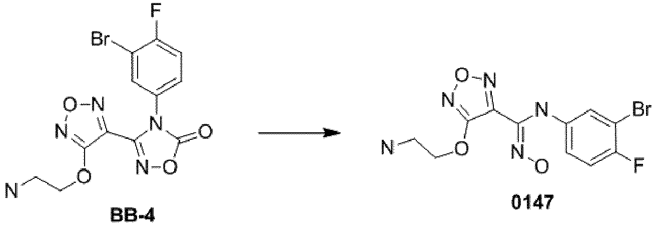

### Step 1: synthesis of compound 0147

The compound BB-4 (560.00 mg, 1.33 mmol, 1.00 *eq,* hydrochloric acid) was added to methanol (6.00 mL) and water (2.00 mL), and then sodium hydroxide (4 M, 1.33 mL, 4.00 *eq*) was added. The reaction solution was stirred at 25 °C for 16 hours. With LCMS showing completion of reaction, the reaction solution was diluted by addition of 60 mL of ethyl acetate, washed with saline (20 mL×2), dried over anhydrous sodium sulfate, filtered, and dried by rotary evaporation to obtain a crude product. 80.00 mg of the crude product was subjected to preparative high performance liquid chromatography (column: Boston Green ODS 150*30 5u; mobile phase:[water (0.05% HCl)-ACN]; B%:13%-43%, 10 min) to obtain the product 0147 (60.00 mg, yield: 67.76%, purity: 99.5%, hydrochloride). MS (ESI) m/z: 360, 362 [M+H]⁺. ¹H NMR (400 MHz, CD₃OD): *δ* 7.23-7.14 (m, 1H), 7.11-7.03 (m, 1H), 6.88-6.78 (m, 1H), 4.60-4.49 (m, 2H), 3.42-3.36 (m, 2H).

### Examples 37: 0108

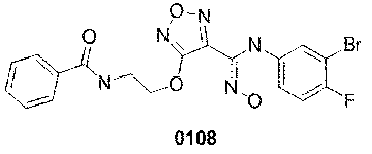

### Synthetic route:

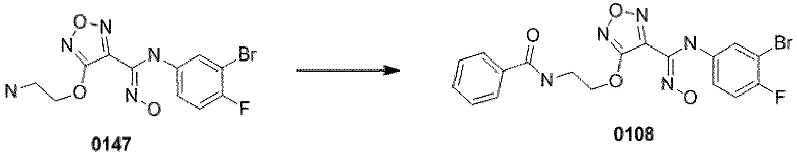

### Step 1: synthesis of compound 0108

The compound 0147 (80.00 mg, 222.14 umol, 1.00 eq) was added to dichloromethane (2.00 mL), and then diisopropylethylamine (86.13 mg, 666.42 umol, 116.39 uL, 3.00 *eq*) and benzoyl chloride (37.47 mg, 266.57 umol, 30.97 uL, 1.20 *eq*) were added. The reaction solution was stirred at 25 °C for 16 hours. Though LCMS showed completion of reactants, there were some dibenzoyl byproducts. The reaction solution was dried by rotary evaporation to obtain a crude product as yellow oil (120.00 mg, crude product). The crude product (110.00 mg, 193.54 umol, 1.00 *eq*) was added to methanol (2.00 mL) and water (1.00 mL), and then sodium hydroxide (23.23 mg, 580.63 umol, 3.00 *eq*) was added. The reaction solution was stirred at 25 °C for 1 hour. With LCMS showing completion of reaction, the reaction solution was filtered. A filtrate was subjected to preparative high performance liquid chromatography (Boston Green ODS 150*30 5u, water (0.1% TFA)-ACN) for separation to obtain a product 0108 (20.00 mg, yield: 22.26%). MS (ESI) m/z: 464, 466 [M+H]⁺. ¹H NMR (400 MHz, CD₃OD): *δ* 7.89-7.80 (m, 2H), 7.61-7.53 (m, 1H), 7.52-7.45 (m, 2H), 7.14-7.09 (m, 1H), 6.94-6.88 (m, 1H), 6.83-6.74 (m, 1H), 4.48-4.33 (m, 2H), 3.79-3.65 (m, 2H).

### Examples 38: 0015

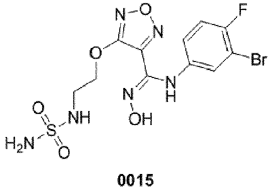

### Synthetic route:

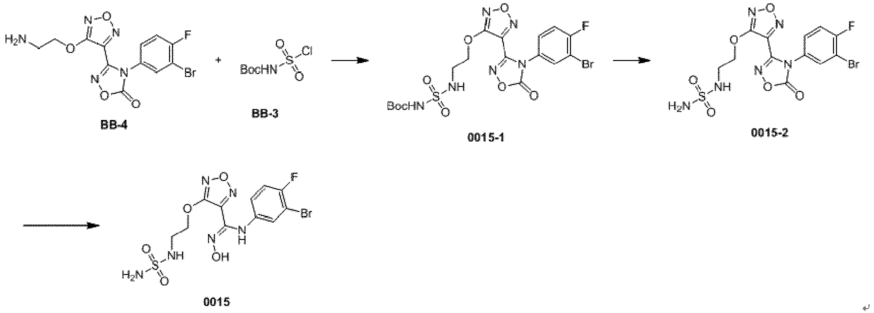

### Step 1: synthesis of compound 0015-1

The compound BB-4 (1.48 g, 3.50 mmol, 1.00 *eq*, hydrochloric acid) was added to dichloromethane (15 mL), and diisopropylethylamine (452.63 mg, 3.50 mmol, 611.66 uL, 1.00 *eq*) was added. The reaction solution turned into brown clear liquid from a turbid state. Then a dichloromethane (13 mL) solution of the compound BB-3 (830.25 mg, 3.85 mmol, 1.10 *eq*) was added dropwise, and the reaction solution gradually turned yellow. After being stirred at 0 °C for 2 hours, the reaction solution turned into a white turbid state. With LCMS monitoring showing about 40% of raw materials remaining, diisopropylethylamine (1.36 g, 10.50 mmol, 1.83 mL, 3.00 *eq*) was further added, the reaction solution turned into a brown clear state, and then the compound BB-3 (830.25 mg, 3.85 mmol, 1.10 *eq*) was added dropwise. The reaction was allowed to continue at 25 °C for 16 hours. Complete reaction of raw materials was observed by TLC (petroleum ether:ethyl acetate=1:1) monitoring. The reaction solution was then dried by rotary evaporation to obtain a liquid product 0015-1 as yellow oil (2.14 g, crude product). MS (ESI) m/z: 565, 567 [M+H]⁺.

### Step 2: synthesis of compound 0015-2

The compound 0015-1 (2.14 g, 3.79 mmol, 1.00 *eq*) was dissolved in dichloromethane (10.00 mL), and hydrochloric acid/dioxane (4 M, 10.00 mL, 10.55 *eq*) was added, followed by reacting at 25 °C for 1 hour. With LCMS monitoring showing completion of reaction of raw materials, the reaction solution was dried by rotary evaporation under reduced-pressure distillation to obtain a yellow liquid product 0015-2 (1.78 g, crude product). MS (ESI) m/z: 465, 467 [M+H]⁺.

### Step 3: synthesis of compound 0015

The compound 0015-2 (1.78 g, 3.83 mmol, 1.00 *eq*) was dissolved in methanol (8.00 mL) and water (4.00 mL), and sodium hydroxide (612.20 mg, 15.30 mmol, 4.00 *eq*) was added. The mixed solution was allowed to react at 25 °C for 20 hours. With LCMS showing completion of reaction, the mixed solution was adjusted to have pH=5 with 1 M hydrochloric acid, and extracted with ethyl acetate (15 mL*3). Organic phases were combined, dried over anhydrous sodium sulfate, and filtered. A filtrate was dried by rotary evaporation under reduced-pressure distillation, dissolved in methanol, and filtered. A filtrate was subjected to high performance liquid chromatography (Phenomenex Synergi C18 150*30 mm*4 um water (0.05% HCl)-ACN) for separation to obtain a product 0015 (532.65 mg, yield: 31.66%, purity: 100%). MS (ESI) m/z: 439, 441 [M+H]⁺. ¹H NMR (400 MHz, CD₃OD) *δ* 7.12-7.18 (m, 1H), 7.05-7.11 (m, 1H), 6.85 (ddd, 1H), 4.36 (t, 2H), 3.38 (t, 2H).

Various examples in the following table were synthesized according to the synthetic method of steps 1-3 in Example 38 (compound 0015):

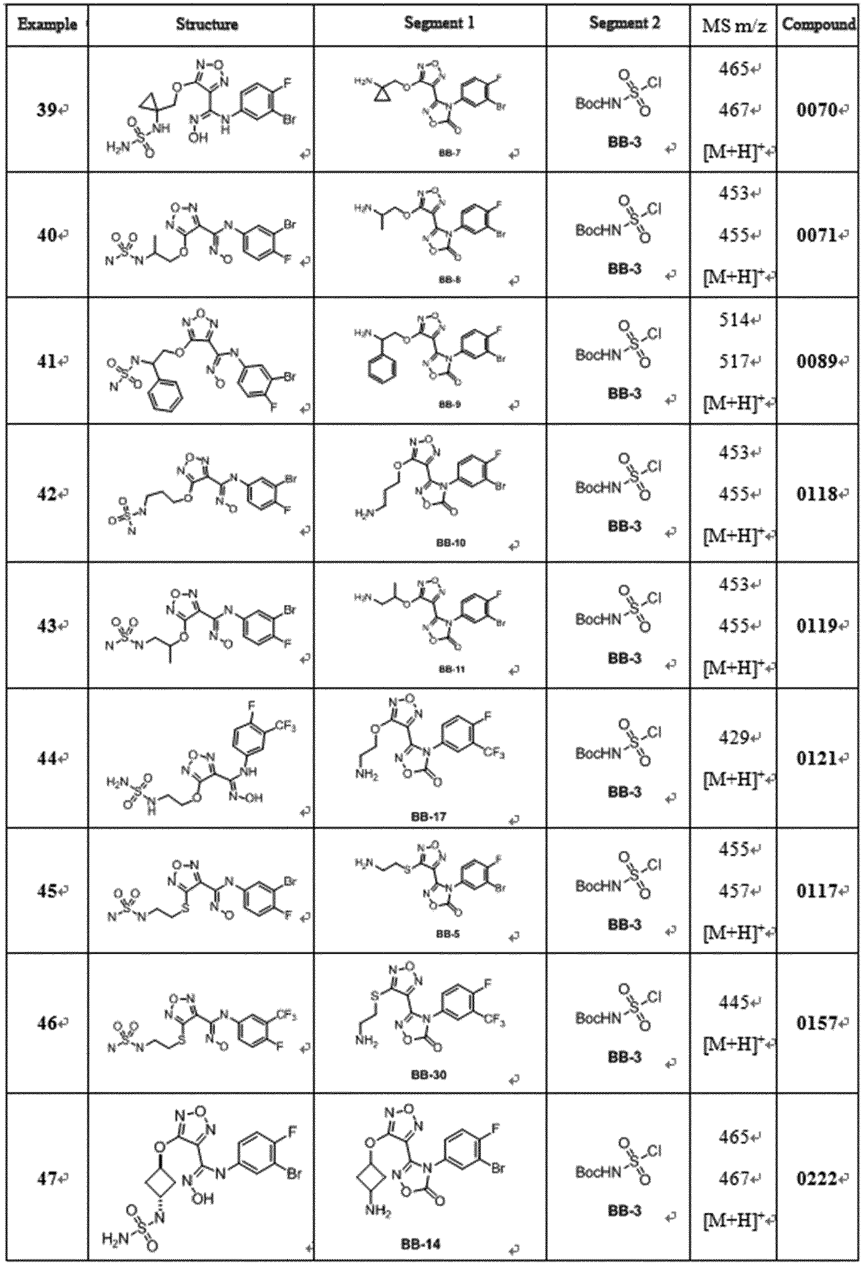

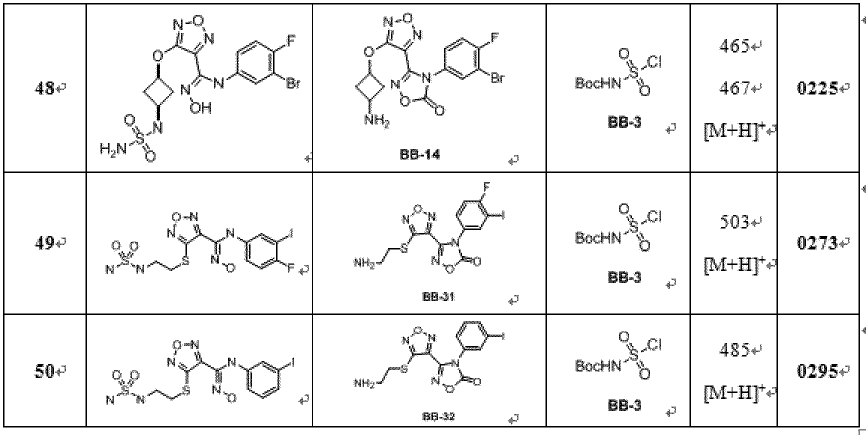

### Examples 51: 0068

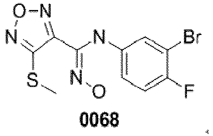

### Synthetic route:

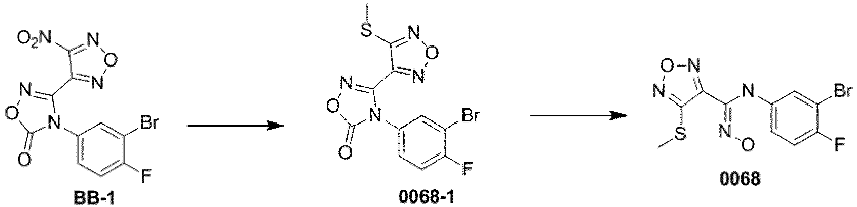

### Step 1: synthesis of compound 0068-1

A compound BB-1 (50.00 mg, 134.39 umol,1.00 *eq*) was dissolved in water (100.00 uL) and tetrahydrofuran (5.00 mL), then sodium thiomethoxide (18.84 mg, 268.78 umol, 17.13 uL, 2.00 *eq*) was added, and then the resultant mixture was stirred at 25 °C for 16 hours. With LCMS showing completion of reaction, the reaction solution was dried by rotary evaporation to obtain a product 0068-1 as yellow oil (51.00 mg, crude product) which was directly used for reaction in a next step. MS (ESI) m/z: 373, 375 [M+H]⁺.

### Step 2: synthesis of compound 0068

The compound 0068-1 (50.00 mg, 133.99 umol, 1.00 eq) was dissolved in water (300.00 uL) and tetrahydrofuran (2.00 mL), then sodium hydroxide (18.76 mg, 468.97 umol, 17.13 uL, 3.50 eq) was added, and then the resultant mixture was stirred at 25 °C for 3 hours. With LCMS showing completion of most of reaction, the reaction solution was adjusted to have a pH value of ∼7 by addition of 1 M hydrochloric acid, and filtered. A filtrate was subjected to high performance liquid chromatography (water (0.05% HCI)-ACN, column: Boston Green ODS 150*30 5u) to obtain a product 0068 (15.00 mg, yield: 32.25%, purity: 100%). MS (ESI) m/z: 347, 349 [M+H]⁺. ¹H NMR (400 MHz, CD₃OD): *δ* 7.08-7.04 (m, 2H), 6.78-6.77 (m, 1H), 2.62 (s, 3H).

### Examples 52: 0148

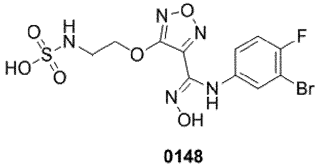

### Synthetic route:

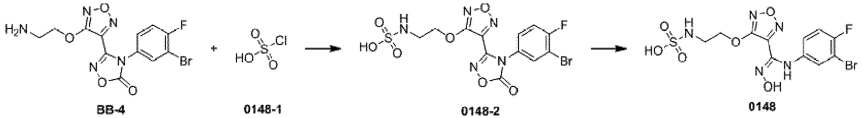

### Step 1: synthesis of compound 0148-2

The compound BB-4 (50.00 mg, 118.32 umol, 1.00 *eq,* hydrochloric acid) was dissolved in dichloromethane (1.00 mL), and diisopropylethylamine (45.88 mg, 354.96 umol, 61.99 uL, 3.00 eq) and a compound 0148-1 (15.17 mg, 130.15 umol, 8.67 uL, 1.10 *eq*) were added. The mixed solution was allowed to react at 23 °C for 2 hours. With LCMS monitoring showing completion of reaction of raw materials, the reaction solution was directly dried by rotary evaporation under reduced-pressure distillation to obtain a colorless liquid product 0148-2 (56.00 mg, crude product).

### Step 2: synthesis of compound 0148

The compound 0148-2 (56.00 mg, 120.12 umol, 1.00 *eq*) was dissolved in methanol (1.00 mL) and water (120.00 uL), and sodium hydroxide (28.83 mg, 720.72 umol, 6.00 *eq*) was added. The reaction solution gradually turned into a brown solution. The mixed solution was allowed to react at 21 °C for 16 hours. With LCMS monitoring showing completion of reaction of raw materials, the reaction solution was subjected to rotary evaporation under reduced-pressure distillation to remove methanol, followed by addition of 3 mL of water, and adjusted to pH=2 with 1 M hydrochloric acid, and extracted with ethyl acetate (5 mL*3). Organic phases were combined, dried over anhydrous sodium sulfate, and filtered. A filtrate was dried by rotary evaporation under reduced-pressure distillation, dissolved in 3 mL of methanol, and filtered. The filtrate was separated by high performance liquid chromatography (Phenomenex Synergi C18 150*30 mm*4 um water (0.05% HCl)-ACN) to obtain a product 0148 (15.73 mg, yield: 27.47%, purity: 100%, hydrochloride). MS (ESI) m/z: 438, 440 [M-H]⁻. ¹H NMR (400 MHz, CD₃OD) *δ* 7.18 (dd, 1H), 7.08 (t, 1H), 6.81-6.89 (m, 1H), 4.56-4.63 (m, 2H), 3.55-3.66 (m, 2H).

Various examples in the following table were synthesized according to the synthetic method of steps 1-2 in Example 52 (compound 0148):

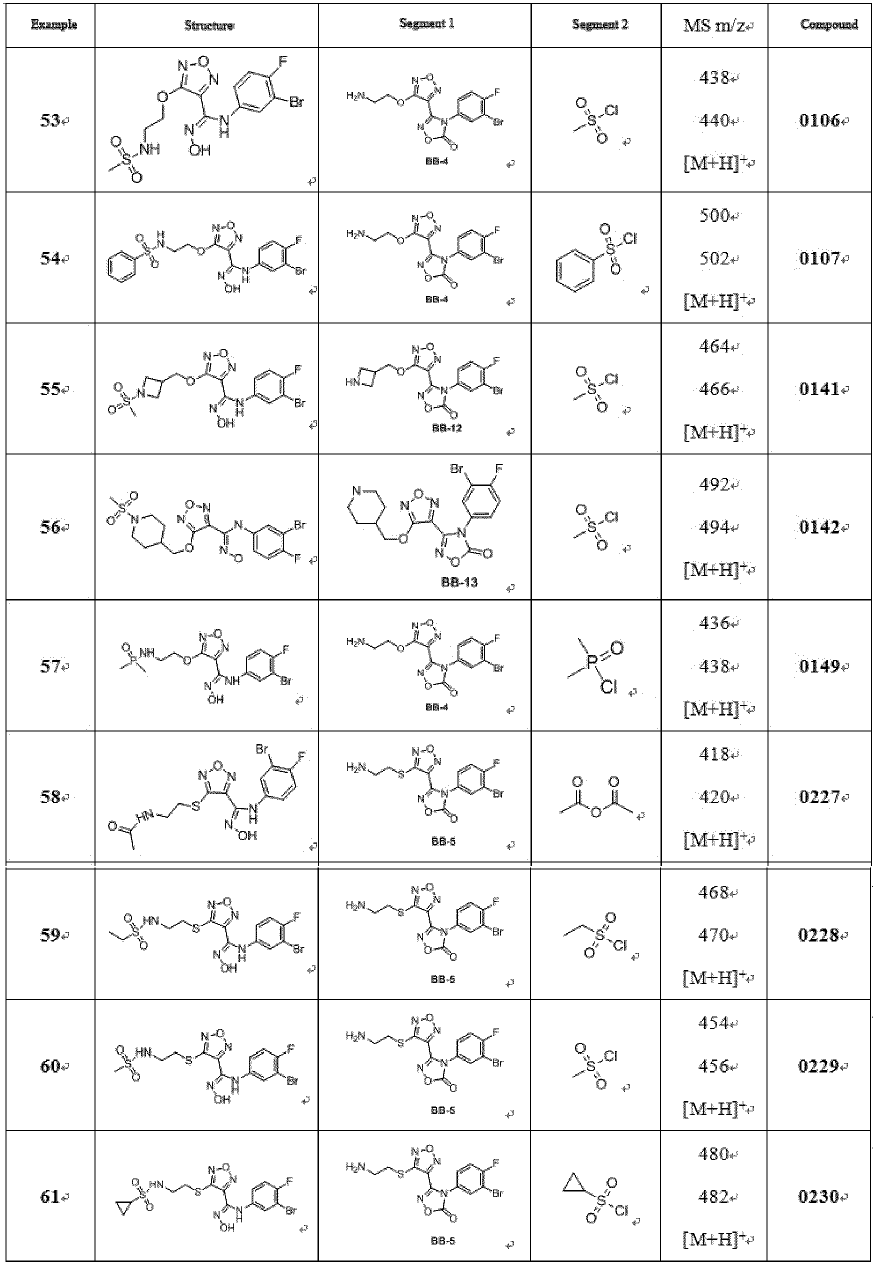

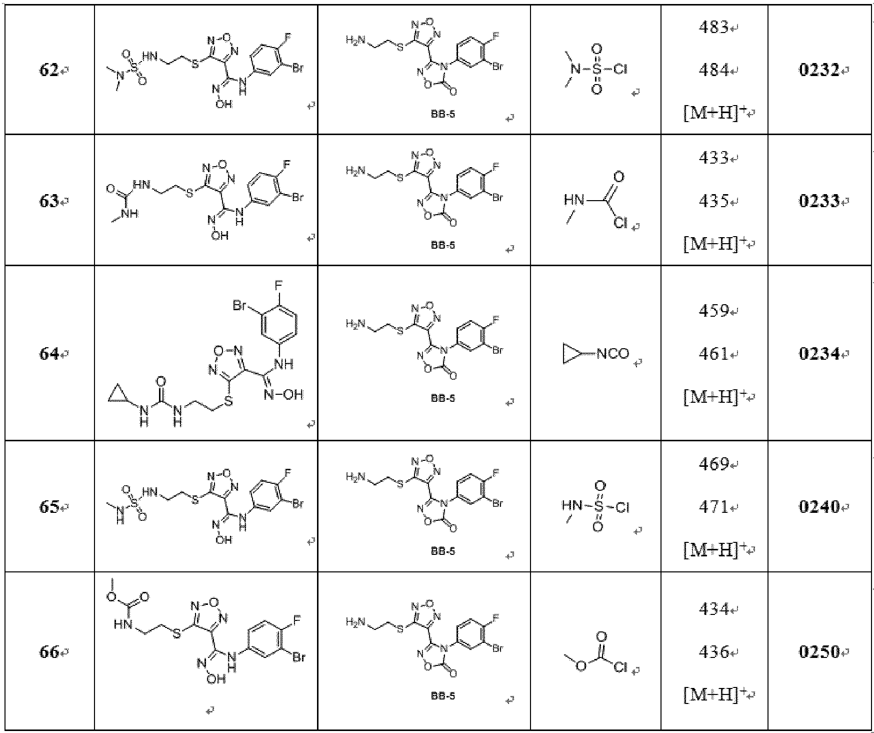

### Examples 67: 0153

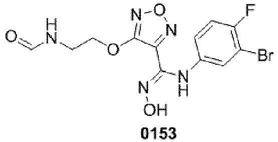

### Synthetic route:

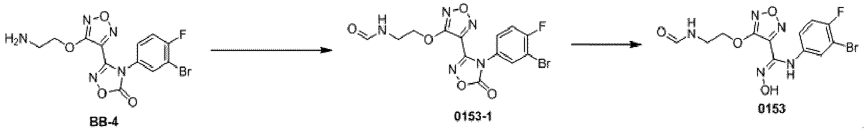

### Step 1: synthesis of compound 0153-1

The compound BB-4 (50.00 mg, 129.49 umol, 1.00 *eq*) and formic acid (8.94 mg, 194.24 umol, 7.33 uL, 1.50 *eq*) were dissolved in *N,N*-dimethylformamide (1.00 mL), and diisopropylethylamine (66.94 mg, 517.96 umol, 90.46 uL, 4.00 *eq*) and HATU (59.08 mg, 155.39 umol, 1.20 *eq*) were added. The reaction mixture was allowed to react at 20 °C for 16 hours. Complete reaction of raw materials was observed by LCMS monitoring, and a target compound was generated. Five mL of water was added, followed by extraction with ethyl acetate (5 mL*3). Organic phases were combined, dried over anhydrous sodium sulfate, and filtered. A filtrate was dried by rotary evaporation under reduced-pressure distillation to obtain a light yellow liquid product 0153-1 (55.00 mg, crude product). MS (ESI) m/z: 414, 416 [M+H]⁺.

### Step 2: synthesis of compound 0153

The compound 0153-1 (55.00 mg, 132.81 umol, 1.00 *eq*) was dissolved in methanol (1.00 mL) and water (200.00 uL), and sodium hydroxide (21.25 mg, 531.22 umol, 4.00 *eq*) was added. The mixed solution was allowed to react at 21 °C for 16 hours. Complete reaction of raw materials was observed by LCMS monitoring, and a target compound was generated. The resulting compound is subjected to rotary evaporation under reduced-pressure distillation to remove methanol, followed by adjustment to pH=2 with 1 M hydrochloric acid, and extraction with ethyl acetate (5 mL*3). Organic phases were combined, dried over anhydrous sodium sulfate, and filtered. A filtrate was dried by rotary evaporation under reduced-pressure distillation, dissolved in 3 mL of methanol, and filtered. A filtrate was separated by high performance liquid chromatography (Phenomenex Synergi C18 150*30 mm*4 um water (0.05% HCl)-ACN) to obtain a product 0153 (40.09 mg, yield: 71.09%, purity: 100%, hydrochloride). MS (ESI) m/z: 388, 390 [M+H]⁺. ¹H NMR (400 MHz, CD₃OD) *δ* 8.12 (s, 1H), 7.34 (dd, 1H), 7.09-7.19 (m, 1H), 6.86-7.02 (m, 1H), 4.27 (t, 2H), 3.54 (t, 2H).

Various examples in the following table were synthesized according to the synthetic method of steps 1-2 in Example 67 (compound 0153):

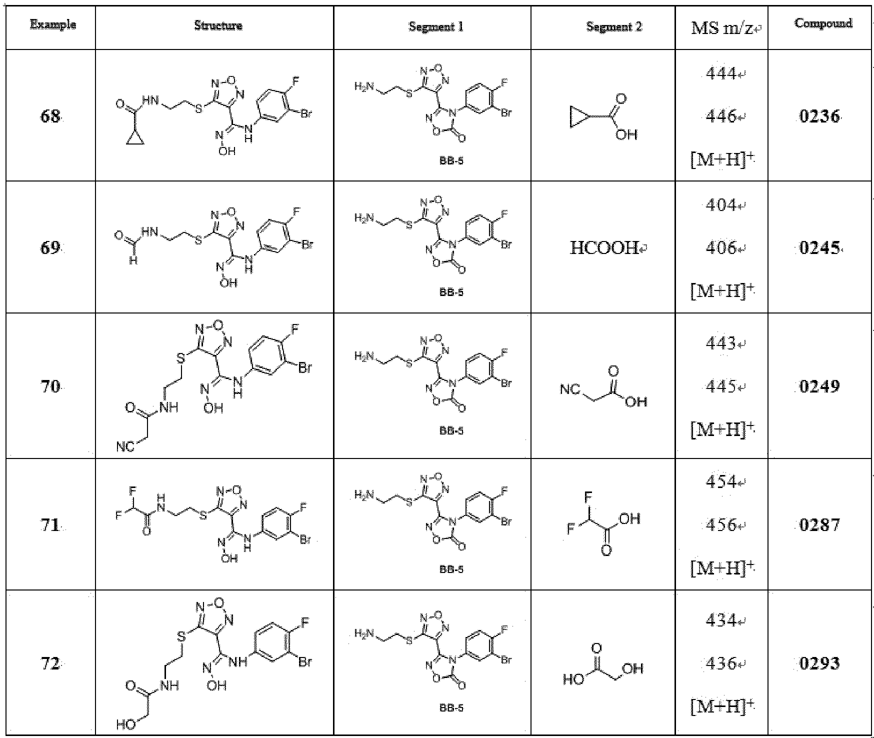

Various examples in the following table were synthesized according to the synthetic method of step 1 in Example 67 (compound 0153):

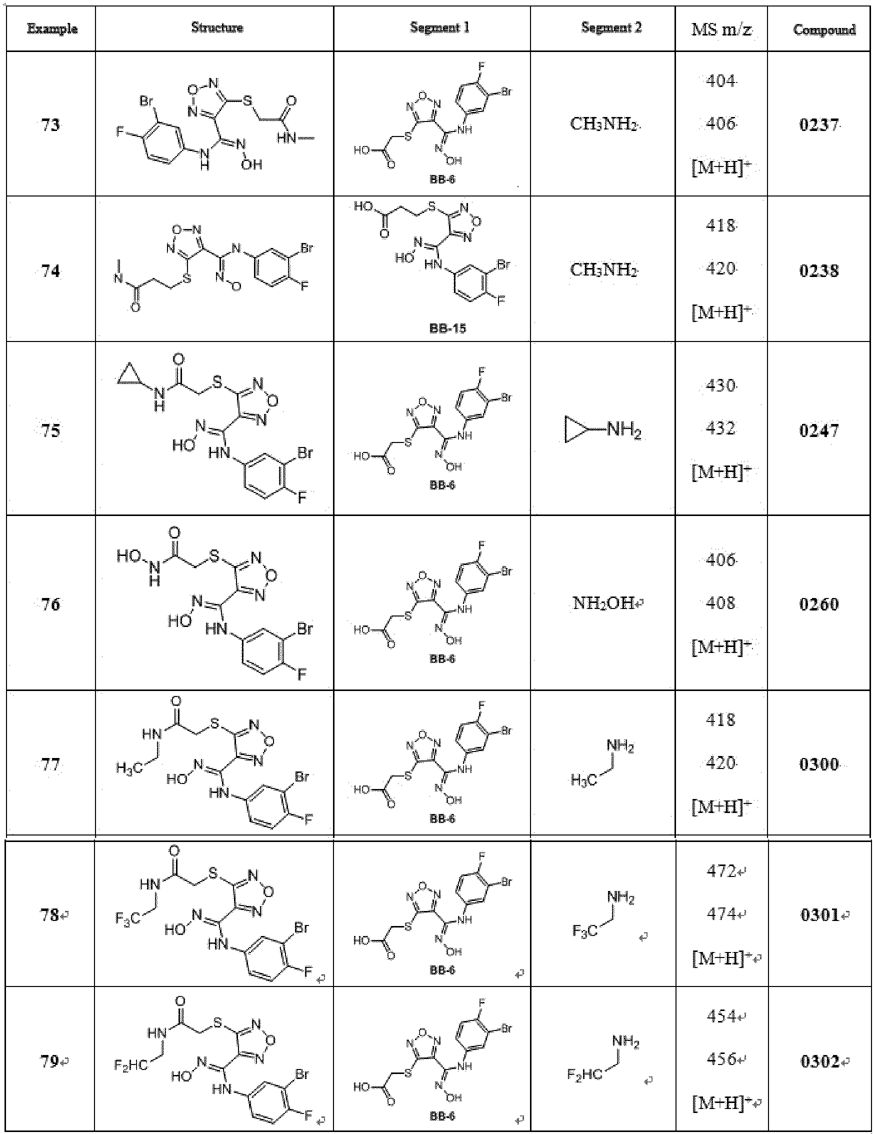

### Examples 80: 0251

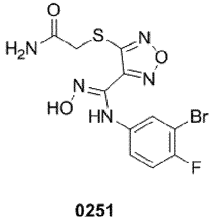

### Synthetic route:

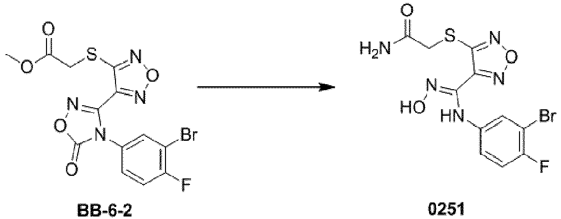

### Step 1: synthesis of compound 0251

A methanol/ammonia solution (5.00 mL, 4 M) was added to a round-bottom flask containing the compound BB-6-2 (50.00 mg, 115.96 umol, 1.00 *eq*), followed by stirring and reaction at 25 °C for 3 hours. Methanol (2 mL) was added thereto, followed by filtration. Upon high performance liquid chromatography (Kromasil 150*25 mm*10 um, water (0.05% ammonia hydroxide v/v)-ACN) separation, a product 0251 (24.50 mg, yield: 54.15%, purity: 100%) was obtained. MS (ESI) m/z: 390, 392 [M+H]⁺. ¹H NMR (400 MHz, CD₃OD): *δ* 7.13 (dd, *J*=2.6, 5.9 Hz, 1H), 7.05 (t, *J*=8.7 Hz, 1H), 6.81 (ddd, *J*=2.6, 4.0, 8.9 Hz, 1H), 3.96 (s, 2H).

### Examples 81: 0262

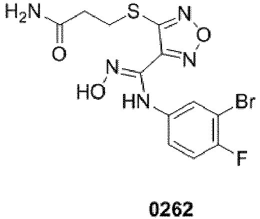

### Synthetic route:

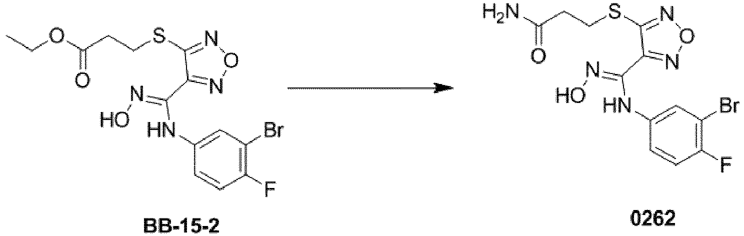

### Step 1: synthesis of compound 0262

To a round-bottom flask containing a compound BB-15-2 (180.00 mg, 392.79 umol, 1.00 *eq),* a methanol/ammonia solution (13.00 mL) (4 M) was added. Upon stirring and reaction at 15 °C for 6 hours, no product was generated. Upon continuous stirring and reaction for 20 hours, a product was generated, and the reaction was continued for 20 hours. After half of the solvent was removed upon rotary evaporation at 20 °C, the remaining was filtered, and subjected to high performance liquid chromatography (DuraShell 150*25 mm*5 um, water (0.05% HCl)-ACN) for separation, to obtain a product 0262 (15.85 mg, yield: 9.98%, purity: 100%). MS (ESI) m/z: 404, 406 [M+H]⁺. ¹H NMR (400 MHz, CD₃OD): *δ* 7.10 (dd, *J*=2.6, 5.9Hz, 1H), 7.05 (t, *J*=8.7Hz, 1H), 6.77 (ddd, *J*=2.8, 4.0, 8.8Hz, 1H), 3.40 (t, *J*=6.9Hz, 2H), 2.74 (t, *J*=6.9Hz, 2H).

### Examples 82: 0231

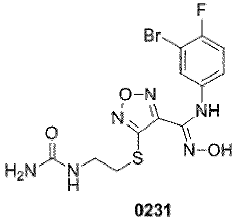

### Synthetic route:

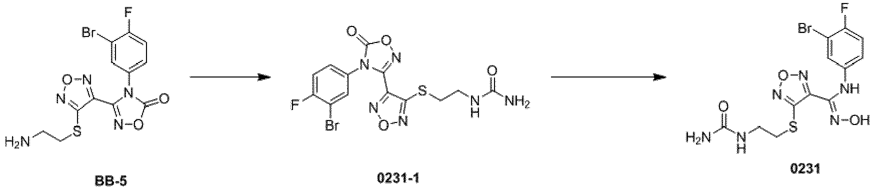

### Step 1: synthesis of compound 0231-1

A compound BB-5 (50.00 mg, 113.98 umol, 1.00 *eq*, hydrochloride) and potassium cyanate (9.25 mg, 113.98 umol, 1.00 *eq*) were dissolved in water (2.00 mL), and heated to 100 °C and reacted for 2 hours. Water (5 mL) was added to the reaction solution, and the resultant mixture was extracted with ethyl acetate (5 mL*3). Organic phases were combined and then dried over anhydrous sodium sulfate, and dried by rotary evaporation to obtain a product 0231-1 as light yellow oil (50.00 mg, crude product). MS (ESI) m/z: 445, 447 [M+H]⁺.

### Step 2: synthesis of compound 0231

The compound 0231-1 (50.00 mg, 112.30 umol, 1.00 *eq*) was dissolved in methanol (1.50 mL) and water (1.00 mL), and sodium hydroxide (17.97 mg, 449.20 umol, 4.00 *eq*) was added, followed by stirring and reaction at 20 °C for 1.5 hours. The reaction solution was adjusted to have pH of 6∼7 by addition of hydrochloric acid (6 M), followed by addition of methanol (2 mL) and filtration. A filtrate was subjected to high performance liquid chromatography (Phenomenex Synergi C18 150*30 mm*4 um, water (0.05% HCl)-ACN) for separation, to obtain a product 0231 (23.00 mg, yield: 48.51 %, purity: 99.3%). MS (ESI) m/z: 419, 421 [M+H]⁺. ¹H NMR (400 MHz, CD₃OD): *δ* 7.11 (dd, *J*=2.8, 6.0Hz, 1H), 7.06 (t, *J*=8.7Hz, 1H), 6.79 (ddd, *J*=2.8, 4.0, 8.8Hz, 1H), 3.59 (t, *J*=6.4Hz, 3H), 3.36 (br.s., 1H).

### Examples 83: 0309

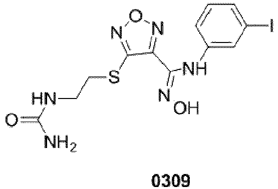

### Synthetic route:

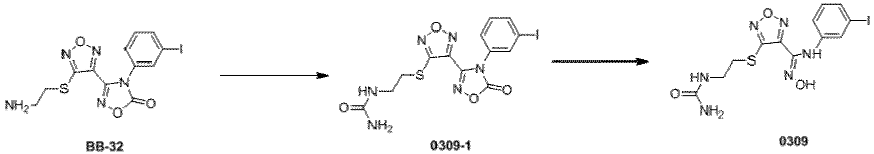

Taking a compound BB-32 as raw material, a compound 0309 was synthesized according to the synthesis steps 1-2 for the compound 0231 in Example 82. MS (ESI) m/z: 471 [M+Na]⁺. ¹H NMR (400 MHz, CD₃OD) *δ* 7.33 (d, 1H), 7.18 (t, 1H), 6.95 (t, 1H), 6.75 (dd, 1H), 3.51 (t, 2H), 3.26-3.31 (t, 2H).

### Examples 84: 0239

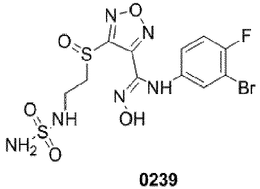

### Synthetic route:

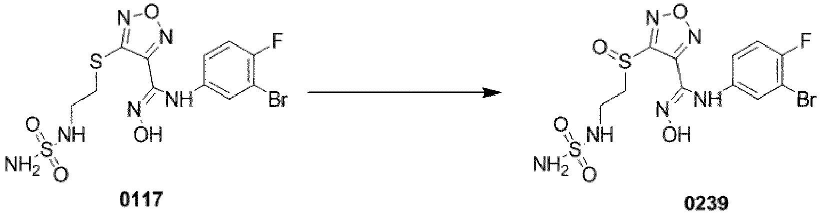

### Step 1: synthesis of compound 0239

A compound 0117 (40.00 mg, 87.86 umol, 1.00 *eq*) was dissolved in dichloromethane (1.00 mL), and m-chloroperbenzoic acid (27.57 mg, 87.86 umol, purity: 55%, 1.00 *eq*) was added. The reaction solution was allowed to react at 10 °C for 1 hour. Complete reaction of raw materials was observed by LCMS monitoring, and a target compound was generated. Three mL of methanol was added to the reaction solution, followed by filtration. A filtrate was subjected to high performance liquid chromatography (Phenomenex Synergi C18 150*30 mm*4 um water (0.05% HCl)-ACN) for separation, to obtain a product 0239 (11.57 mg, yield: 25.94%, purity: 100%, hydrochloride). MS (ESI) m/z: 471, 473 [M+H]⁺. ¹H NMR (400 MHz, CD₃OD) *δ* 7.27 (d, 1H), 7.09 (t, 1H), 6.96 (d, 1H), 3.73-3.85 (m, 1H), 3.53-3.71 (m, 3H).

### Examples 85: 0069

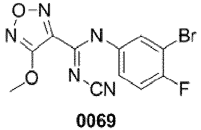

### Synthetic route:

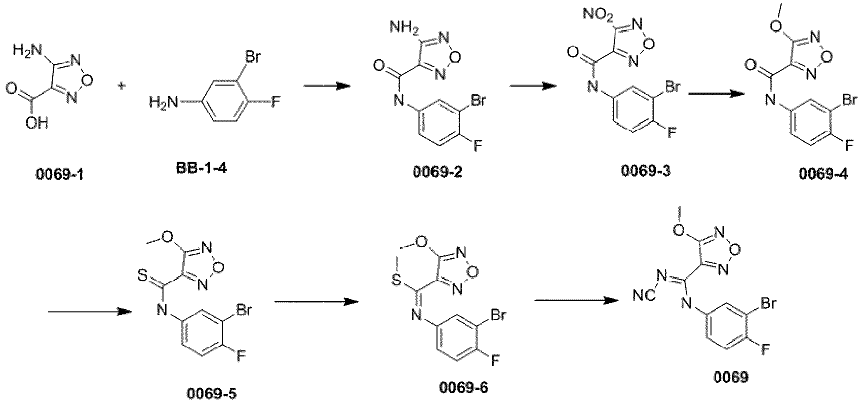

### Step 1: synthesis of compound 0069-2

The compound 0069-1 (2.50 g, 19.37 mmol, 1.00 *eq*) was dissolved in *N,N*-dimethylformamide (60.00 mL), then a compound BB-1-4 (3.68 g, 19.37 mmol, 1.00 *eq*), HATU (8.84 g, 23.24 mmol, 1.20 *eq*), and diisopropylethylamine (5.01 g, 38.74 mmol, 6.77 mL, 2.00 *eq*) were sequentially added. The reaction solution was allowed to react at 25 °C for 1 hour. The reaction solution turned yellow. With LCMS showing completion of reaction, 240 mL of water was added to the resulting mixture, followed by filtration. A filter cake was washed with water (20 mL*3), and naturally dried to obtain a gray solid product 0069-2 (5.50 g, yield: 92.43%, purity: 98%) which was directly used for reaction in a next step. MS (ESI) m/z: 301, 303 [M+H]⁺.

### Step 2: synthesis of compound 0069-3

At 0 °C, sulfuric acid (9.00 mL) was slowly added to a hydrogen peroxide (10.62 g, 93.64 mmol, 9.00 mL, purity: 30%, 56.41 *eq*), then sodium tungstate (487.96 mg, 1.66 mmol, 1.00 *eq*) was added, following by the compound 0069-2 (500.00 mg, 1.66 mmol, 1.00 eq). The resultant mixture was heated and stirred at 25 °C for 16 hours. With LCMS showing completion of reaction, the reaction solution was diluted by addition of 20 mL of water, and filtered. A filter cake was dried by rotary evaporation to obtain a white solid product 0069-3 (520.00 mg, yield: 89.89%, purity: 95%) which was directly used for reaction in a next step. MS (ESI) m/z: 331, 333 [M+H]⁺.

### Step 3: synthesis of compound 0069-4

The compound 0069-3 (500.00 mg, 1.51 mmol, 1.00 eq) was dissolved in tetrahydrofuran (5.00 mL), water (100.00 uL) and methanol (1.00 mL), then sodium hydroxide (84.58 mg, 2.11 mmol, 1.40 *eq*) was added, and then the resultant mixture was stirred at 25 °C for 16 hours. With LCMS showing completion of most of the reaction, the reaction solution was adjusted to ∼7 in pH value by addition of 1 N hydrochloric acid. The reaction solution was diluted by addition of 50 mL of ethyl acetate, washed with a saturated saline (10 mL× 3), dried over anhydrous sodium sulfate, and filtered. A filtrate was dried by rotary evaporation to obtain a yellow solid product 0069-4 (430.00 mg, crude product) which was directly used for reaction in a next step. MS (ESI) m/z: 316, 318 [M+H]⁺.

### Step 4: synthesis of compound 0069-5

The compound 0069-4 (200.00 mg, 632.75 umol, 1.00 *eq*) was dissolved in toluene (2.00 mL), then Lawesson's reagent (511.86 mg, 1.27 mmol, 2.00 *eq*) was added, and then the resultant mixture was stirred at 90 °C for 16 hours. With LCMS showing completion of most of reaction, the reaction solution was dried by rotary evaporation to obtain a crude product. The crude product was purified by a flash column chromatography (0-40% ethyl acetate in petroleum ether) to obtain a red solid product 0069-5 (120.00 mg, yield: 51.96%, purity: 91%). MS (ESI) m/z: 332, 334 [M+H]⁺.

### Step 5: synthesis of compound 0069-6

The compound 0069-5 (150.00 mg, 451.60 umol, 1.00 *eq*) was dissolved in dichloromethane (3.00 mL), then diisopropylethylamine (175.09 mg, 1.35 mmol, 236.61 uL, 3.00 *eq*) was added, then methyl trifluoromethansulfonate (111.16 mg, 677.40 umol, 74.11 uL, 1.50 *eq*) was added dropwise, and then the resultant mixture was stirred at 25 °C for 3 hours. With TLC (petroleum ether:ethyl acetate=3:1) showing completion of reaction, the reaction solution was dried by rotary evaporation to obtain a crude product. The crude product was purified by flash column chromatography (0-40% ethyl acetate in petroleum ether) to obtain a product 0069-6 as yellow oil (130.00 mg, yield: 82.82%, purity: 99.6%). MS (ESI) m/z: 346, 348 [M+H]⁺.

### Step 6: synthesis of compound 0069

The compound 0069-6 (100.00 mg, 288.87 umol, 1.00 *eq*) was dissolved in ethanol (3.00 mL), then diisopropylethylamine (112.00 mg, 866.60 mmol, 151.35 uL, 3.00 *eq*) was added, then cyanamide (36.43 mg, 866.60 umol, 36.43 uL, 3.00 *eq*) was added, and the resultant mixture was transferred into a microwave tube, to be subjected to microwave reaction at 100 °C for 1 hour. With LCMS monitoring showing completion of reaction, a main product peak was generated. Filtration was performed, and a filtrate was dried by rotary evaporation to obtain a crude product. The crude product was purified by high performance liquid chromatography (water (0.05% HCl)-ACN, Boston Green ODS 150*30 5u) to obtain a product 0069 (18.00 mg, yield: 18.32%, purity: 100%). MS (ESI) m/z: 340, 342 [M+H]⁺. ¹H NMR (400 MHz, CD₃OD): *δ*=8.22-7.98 (m, 1H), 7.82-7.62 (m, 1H), 7.43-7.26 (m, 1H), 4.25 (br.s., 3H).

### Example 85: Compound 0310

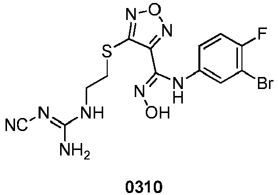

### Synthetic route:

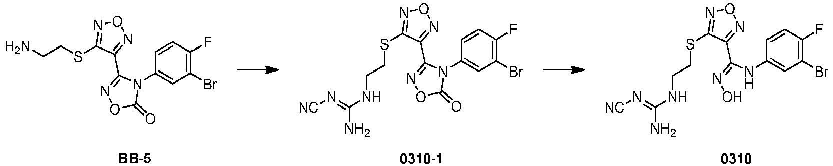

### Step 1: synthesis of compound 0310-1

The segment BB-5 (100.00 mg, 227.97 umol, 1.00 *eq*, HCl) and sodium dicyanamide (60.89 mg, 683.91 umol, 3.00 *eq*) were dissolved in DMF (2.00 mL), and HCI (2 M, 115.12 uL, 1.01 *eq*) was added. The reaction solution was heated to 110 °C and reacted for 2 hr. Eight mL of water was added to the reaction solution, followed by extraction with ethyl acetate (8 mL*3). Organic phases were combined, washed with a saline (20 ml*3), dried over anhydrous sodium sulfate, and filtered. A filtrate was dried by rotary evaporation under reduced-pressure distillation to obtain a compound 0310-1. MS (ESI) m/z: 469.0, 471.0 [M+H]⁺.

### Step 2: synthesis of compound 0310

The compound 0310-1 (106.00 mg, 225.89 umol, 1.00 *eq*) was dissolved in MeOH (1.00 mL) and H₂O (500.00 uL), and NaOH (36.14 mg, 903.56 umol, 4.00 *eq*) was added. The reaction solution was allowed to react at 22 °C for 2 hr. The reaction solution was filtered. A filtrate was purified by Pre-HPLC (Kromasil 150*25 mm*10 um water (0.05% ammonia hydroxide v/v)-ACN) for separation to obtain a compound 0310. MS (ESI) m/z: 464.9, 466.9 [M+Na]⁺. ¹H NMR (400 MHz, MeOD): *δ*=7.01-7.14 (m, 2H), 6.77 (ddd, 1H), 3.51-3.66 (m, 2H), 3.28-3.32 (m, 2H).

### Example 86: Compound 0383

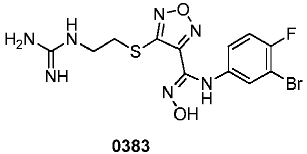

### Synthetic route:

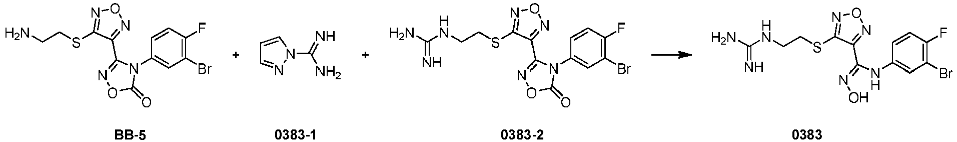

### Step 1: synthesis of compound 0383-2

The segment BB-5 (50.00 mg, 113.98 umol, 1.00 eq, HCI) was dissolved in DMF (1.00 mL), DIEA (103.12 mg, 797.86 umol, 139.35 uL, 7.00 eq) was added, and then the compound 0383-1 (33.41 mg, 227.96 umol, 2.00 eq) was added. The reaction solution was allowed to react at 23 °C for 2 hr. Five mL of water was added, followed by extraction with ethyl acetate (5 mL*3). Organic phases were combined, dried over anhydrous sodium sulfate, and filtered. A filtrate was dried by rotary evaporation under reduced-pressure distillation to obtain a compound 0383-2. MS (ESI) m/z: 444.0, 446.0 [M+H]⁺.

### Step 2: synthesis of compound 0383

The compound 0383-2 (100.00 mg, 225.10 umol, 1.00 eq) was dissolved in THF (1.00 mL) and H₂O (500.00 uL), and NaOH (18.01 mg, 450.20 umol, 2.00 eq) was added. The reaction solution was allowed to react at 25 °C for 1 hr. The reaction solution was adjusted to pH=5 with concentrated hydrochloric acid, followed by addition of 3 ml of methanol and filtration. A filtrate was separated and purified by pre-HPLC (YMC-Actus Triart C18 150*30 5u water (0.05% HCl)-ACN) to obtain a compound 0383. MS (ESI) m/z: 417.9, 419.9 [M+H]+ ¹H NMR (400 MHz, MeOD): *δ*=7.13-7.03 (m, 2H), 6.85-6.73 (m,1H), 3.70-3.54 (m, 2H), 3.45-3.35 (m, 2H).

### Example 87: Compound 0384

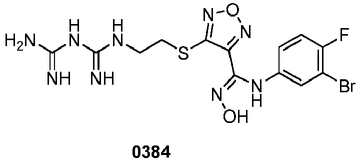

### Synthetic route:

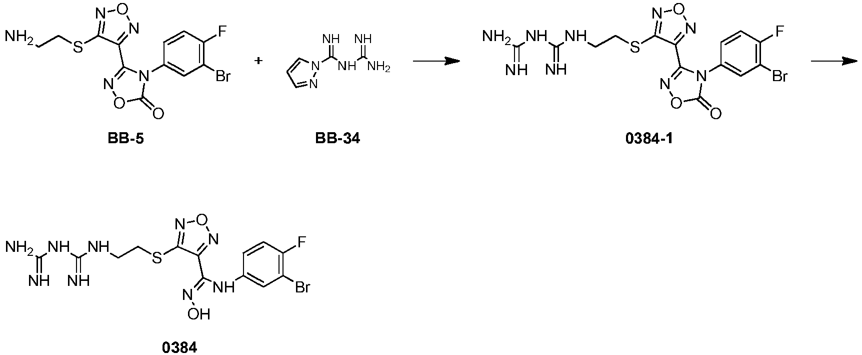

### Step 1: synthesis of compound 0384-1

The segment BB-5 (50.00 mg, 113.98 umol, 1.00 *eq*, HCl) was dissolved in CH₃CN (1.00 mL), DIEA (58.92 mg, 455.92 umol, 79.63 uL, 4.00 *eq*) was added, and then a DMF solution (0.25 ml) of the segment BB-34 (107.49 mg, 569.90 umol, 5.00 *eq*, HCl) was added. The reaction solution was allowed to react at 20 °C for 64 hr. The reaction solution was directly dried by rotary evaporation under reduced-pressure distillation to obtain a compound 0384-1. MS (ESI) m/z: 485.9, 487.9 [M+H]⁺.

### Step 2: synthesis of compound 0384

The compound 0384-1 (55.00 mg, 113.10 umol, 1.00 *eq*) was dissolved in THF (1.00 mL) and H₂O (500.00 uL), and NaOH (36.19 mg, 904.80 umol, 8.00 *eq*) was added. The reaction solution was allowed to react at 20 °C for 1 hr. The reaction solution was adjusted to pH=6 with concentrated hydrochloric acid, followed by addition of 3 ml of methanol and filtration. A filtrate was separated and purified by pre-HPLC (YMC-Actus Triart C18 150*30 5u water (0.05% HCl)-ACN) to obtain a compound 0384. MS (ESI) m/z: 460.0, 462.0 [M+H]^{+ 1}H NMR (400 MHz, MeOD): *δ*=7.13-7.04 (m, 2H), 6.85-6.77 (m, 1H), 3.80 (t, *J*=6.8 Hz, 2H), 3.49 (br t, *J*=6.5 Hz, 2H).

### Test Example 1: Bioactivity Test

### I. Test of hIDO1 for in vitro activity

### 1. Test of hIDO1 for enzymatic activity in vitro

### 1.1 Purpose of experiment:

Changes in production of NFK, an IDO1 enzymatic metabolite were detected with NFK green™ fluorescent molecules. With an IC50 value of a compound as an index, an inhibitory effect of the compound on recombinant human IDO1 enzyme was evaluated.

### 1.2 Experimental materials:

NFK green™ reagent, Netherlands Translational research center
IDO1 enzymatic activity detection reagent kit, NTRC#NTRC-hIDO-10K
384-well enzymatic reaction plate, PerkinElmer#6007279
384-well compound plate, Greiner#781280
plate sealing film, PerkinElmer#6050185
Envision multi-functional plate reader, PerkinElmer
Bravo automatic liquid processing platform, Agilent

### 1.3 Experimental steps and method:

### 1.3.1 Addition of compound sample:

The compound was diluted with dimethyl sulfoxide (DMSO) to 1 mM, with 10 gradients, each 3-fold dilution, in duplicate. 48 µL of 50 mM phosphate buffer, having pH of 6.5, was transferred by the Bravo automatic liquid processing platform to the compound plate. Then 2 µL of an already diluted compound/DMSO solution was added. After even mixing, 10 µL of the resultant mixture was transferred to the enzymatic reaction plate.

### 1.3.2 Experiment for detecting IDO1 enzymatic activity:

IDO1 enzyme was diluted to 20 nM in a reaction buffer (50 mM phosphate buffer with pH of 6.5, 0.1% of Tween-20, 2% of glycerol, 20 mM ascorbic acid, 20 µg/ml catalase, and 20 µM methylene blue), 20 µL of the diluted IDO1 enzyme was transferred to the enzymatic reaction plate, and incubated at 23 °C for 30 minutes. 10 µL of 400 µM L-type tryptophan substrate was added to start reaction. The incubation lasted at 23 °C for 90 minutes. 10 µL of NFK green™ fluorescent dye was added. The resultant mixture was sealed with the plate sealing film. After incubation at 37 °C for 4 hours, an Envision multi-functional plate reader was used for reading (Ex 400 nm/Em 510 nm).

### 1.3.3 Data analysis:

A reference well to which the IDO1 enzyme was added but no compound was added was set to have an inhibition rate of 0%, and a reference well to which no IDO1 enzyme was added was set to have an inhibition rate of 100%. Data was analyzed with XLFit 5 to calculate an IC50 value of the compound. Test results thereof are as shown in Table 1.

### 2. Test of hIDO1 for cytological activity

### 2.1 Purpose of experiment:

Changes in Hela cell kynurenine were detected by an LCMS method. With an IC50 value of a compound as an index, an inhibitory effect of the compound on IDO1 enzyme was evaluated.

### 2.2 Experimental materials:

Cell lines: Hela cells
Culture medium:
   RPMI 1640 phenol red free, Invitrogen#11835030
   10% fetal bovine serum, Gibco#10099141
   1XPenicillin-Streptomycin, Gibco#15140-122
Precipitation agent: 4 µM L-kynurenine-d4 dissolved in 100% acetonitrile, CacheSyn#CSTK008002
Trypsin, Invitrogen#25200-072
DPBS, Hyclone#SH30028.01B
Recombinant human interferon-γ, Invitrogen#PHC4033
5%(w/v) trichloroacetic acid, Alfa Aesar#A11156
96-well cell plate, Corning#3357
96-well compound plate, Greiner#781280
96-well V-bottom plate, Axygen#WIPP02280
CO₂ incubator, Thermo#371
Centrifuge, Eppendorf#5810R
Vi-cell cell counter, Beckman Coulter

### 2.3 Experimental steps and method:

### 2.3.1 Hela cell inoculation:

The culture medium, trypsin, and DPBS were preheated in water bath at 37 °C. The culture medium was removed by sucking from cell culture, followed by cleaning the cell culture with 10 mL of DPBS; the preheated trypsin was added to the culture flask, which was revolved to make the culture flask covered by trypsin uniformly, and placed into an incubator at 37 °C with 5% CO₂ for digestion for 1-2 minutes; 10-15 mL of the culture medium was used to suspend cells each T150, followed by centrifugation at 800 rpm for 5 minutes. 10 mL of the culture medium was used to resuspend the cells. 1 mL of the cell resuspension was pipetted, and counted with Vi-cell; the Hela cells were diluted to 5×10⁵/mL with the culture medium, 80 µL of the diluted Hela cells were added to the 96-well cell plate, and incubated in the an incubator at 37 °C with 5% CO₂ for 5-6 hours.

### 2.3.2 Addition of compound sample:

The compound was diluted with DMSO to 1 mM, with 9 gradients, each 3-fold dilution, in duplicate. 5 µL of the already diluted compound DMSO solution was added to the compound plate containing 95 µL of the culture medium. After even mixing, 10 µL of the mixture was transferred to the cell plate.

### 1) Test for cytological activity:

10 µL of recombinant human interferon-γ was added to result in a final concentration of 100 ng/ml, to induce expression of IDO1. Incubation was carried out in the 5% CO₂ incubator at 37 °C for 20 hours. 4 µL of 5%(w/v) trichloroacetic acid was added, followed by even mixing and incubation at 50 °C for 30 minutes. After centrifugation at 2400 rpm for 10 minutes, 40 µL of a supernatant was added to a 96-well V-bottom plate, followed by addition of a precipitant. Centrifugation was carried out at 4000 rpm for 10 minutes after even mixing. 100 µL of the supernatant was transferred to a new 96-well V-bottom plate. A content of kynurenine was detected through LCMS.

### 2) Data analysis:

A reference well to which the interferon-γ was added but no compound was added was set to have an inhibition rate of 0%, and a reference well to which no Hela cell was added was set to have an inhibition rate of 100%. Data was analyzed with XLFit 5 to calculate an IC50 value of the compound. Test results thereof are as shown in Table 1.

**Table 1: Test Results of in vitro Screening of Compounds of the Present Disclosure**

| Compound | Enzyme IC50(nM) | Hela Cell IC50(nM) | Compound | Enzyme IC50(nM) | Hela Cell IC50(nM) |
|---|---|---|---|---|---|
| 0015 | 115.37 | 14.01 | 0158 | 4358.44 | / |
| 0026 | >10000 | / | 0160 | 3830.14 | / |
| 0052 | 109.41 | 24.87 | 0162 | 928.12 | / |
| 0068 | 112.62 | 28.65 | 0177 | 278.81 | 54.25 |
| 0069 | >10000 | / | 0178 | 3077.61 | / |
| 0070 | 1376.52 | / | 0179 | 593.82 | / |
| 0071 | 798.52 | / | 0180 | >10000 | / |
| 0077 | >10000 | / | 0182 | 672.53 | / |
| 0078 | >10000 | / | 0183 | 1124.05 | / |
| 0089 | 981.00 | / | 0184 | 4833.48 | / |
| 0103 | 537.32 | / | 0189 | 590.30 | / |
| 0106 | 105.56 | 36.95 | 0190 | 944.74 | / |
| 0107 | 1298.73 | / | 0222 | 466.47 | / |
| 0108 | 387.42 | / | 0225 | 273.95 | 18.71 |
| 0117 | 26.19 | 5.85 | 0227 | 59.27 | 3.10 |
| 0118 | 55.26 | 64.51 | 0228 | 101.37 | 3.97 |
| 0119 | 2067.84 | / | 0229 | 80.11 | 11.21 |
| 0120 | 615.82 | / | 0230 | 203.23 | / |
| 0121 | 187.84 | / | 0231 | 42.20 | 4.35 |
| 0122 | 564.64 | / | 0232 | 372.66 | / |
| 0124 | 1026.54 | / | 0233 | 108.56 | 19.19 |
| 0128 | 601.05 | / | 0234 | 76.95 | 9.58 |
| 0129 | 426.13 | / | 0236 | 128.70 | 55.42 |
| 0131 | 701.55 | / | 0237 | 42.27 | 8.51 |
| 0133 | 1028.69 | / | 0238 | 85.38 | 13.88 |
| 0134 | 7150.29 | / | 0239 | 845.76 | / |
| 0135 | 3291.28 | / | 0240 | 128.22 | 26.24 |
| 0137 | 5714.89 | / | 0245 | 74.40 | 14.84 |
| 0138 | 364.38 | / | 0247 | 95.72 | 17.07 |
| 0139 | 265.41 | 100.1 | 0249 | 164.2 | 30.34 |
| 140 | 197.23 | / | 0250 | 137.59 | 27.02 |
| 0141 | 452.46 | / | 0251 | 44.33 | 7.15 |
| 0142 | 1310.71 | / | 0260 | 74.64 | 31.54 |
| 0143 | >10000 | / | 0262 | 89.56 | 70.80 |
| 0144 | >10000 | / | 0273 | 144.02 | 34.40 |
| 0147 | 71.51 | 123 | 0287 | 69.36 | 12.89 |
| 0148 | 147.21 | 158.7 | 0293 | 60.13 | 29.45 |
| 0149 | 290.21 | / | 0295 | 122.26 | 47.22 |
| 0150 | >10000 | / | 0300 | 72.57 | / |
| 0151 | 149.09 | 25.22 | 0301 | 125.28 | / |
| 0153 | 153.46 | 82.37 | 0302 | 88.03 | / |
| 0156 | 5702.94 | / | 0309 | 54.48 | 23.48 |
| 0157 | 159.86 | 13.39 | 0310 | 97.3 | 12.8 |
| 0383 | 100 | 104.7 | 0384 | 173 | 152 |

It is concluded that the compounds of the present disclosure have good *in vitro* activity.

### II. Measurement of Thermodynamic Solubility

### 1. Solutions for thermodynamic solubility

Buffer A (pH 2.0): 50 mM phosphate buffer, with a pH value of 2.0.

Buffer B (pH 7.4): 50 mM phosphate buffer, with a pH value of 7.4.

### 2. Preparation of standard solution

50% acetonitrile solution and 50% buffer (A, B) were mixed together to obtain a diluent. 10 mM (20 µL/compound) stock solution was added to acetonitrile (480 µL/compound), and mixed with the buffer (A, B) (500 µL/compound) to obtain 200 µM UV detection standard solution. The 200 µM UV detection standard solution was diluted with the diluent 10 times or 200 times the amount of the standard solution, so as to acquire 20 µM or 1 µM UV standard solution. 1, 20, and 200 µM UV standard solutions were taken as standard samples for thermodynamic solubility test.

### 3. Method

### 3.1 Sample preparation, shaking, and filtering

No less than 2 mg of a sample powder was weighed and placed in a Whatman miniuniprep vial. If it was required to test the thermodynamic solubility of the sample in multiple buffers (A, B), an independent vial was demanded for each test. 450 µL of the buffer (A, B) was respectively added to each Whatman miniuniprep vial. After the buffer was added, a piston cover of the Whatman miniuniprep functioning to filter was put on and pressed above a liquid surface, such that a filter screen was in contact with the buffer (A, B) during the shaking. The solubility sample was shaken in a vortex manner for 1 minute, and phenomenon of the solution was recorded. The sample was shaken at 600 rpm at a room temperature (approximately 22∼25 °C) for 24 hours. The filtering cover of Whatman Miniunipreps was pressed to a bottom, to obtain a filtrate of the sample solubility solution. All sample vials should be recorded for insoluble substances before and after the filtration and leakage phenomenon thereof. The buffer (A, B) was 50-fold diluted to obtain a sample diluent.

### 3.2 Analysis and detection

3 UV standard solutions, from low concentration to high concentration, were injected into HPLC, and then the diluent and the supernatant of the compound to be tested were injected. The sample to be tested was in duplicate. Integration was carried out for UV chromatographic peaks. A standard curve was established by simulation and the thermodynamic solubility of the sample was calculated. See Table 2 for results. HPLC conditions were as follows.
Test method: HPLC-UV detection
Instrument: Agilent 1200
Mobile phase: A: water+0.69% TFA; B: acetonitrile+0.62% TFA
Chromatographic column: Agilent TC C18 (2.1×50 mm, 4.6 µm)

**Proportions:**

| Time (min) | B% | Flow Velocity (mL/min) |
|---|---|---|
| 0.00 | 5 | 1 |
| 2.00 | 90 | 1 |
| 2.50 | 90 | 1 |
| 2.60 | 5 | 1 |
| 4.00 | 5 | 1 |

**Table 2: Solubility of Compounds of the Present Disclosure**

| Compound | thermodynamic solubility (pH: 2.0) | thermodynamic solubility (pH: 7.4) |
|---|---|---|
| 360 | 65 | 160 |
| 0015 | 138 | 298 |
| 0117 | 2616 | 2607 |
| 0231 | 155 | 125 |

It is concluded that the compounds of the present disclosure have relatively good water solubility.

### III. Permeability Test

### 1. Test for Permeability across MDR1

### 1.1 Preparation of stock solution

A sample for test was dissolved in dimethyl sulfoxide (DMSO) or other suitable solvents, to be prepared into 10 mM stock solution. A suitable internal standard (IS) was dissolved in acetonitrile (ACN) or other organic solvents as a stop solution. Specific information would be described in a research report.

Fenoterol, propranolol, and digoxin acted as low-permeability control, high-permeability control, and P-gp substrate in the present research. The stock solutions of these compounds were prepared with DMSO, stocked at 2-8 °C, and valid for use within 3 months.

### 1.2 Preparation of donor solution and receiver solution

In the present project, a Hank's balanced salt buffer containing 10 mM HEPES was used as a transport buffer. Preparation methods for the donor solution and the receiver solution are as shown in Table 3.

**Table 3: Preparation Methods for Donor Solution and Receiver Solution**

| Solution Name | Components | pH | Final DMSO Concentration (v/v) |
|---|---|---|---|
| Apical and basolateral donor solutions | Prepare a control or sample for test with a concentration of 2µM from the transport buffer | ND | 0.4% |
| Apical and basolateral receiver solutions | Transport buffer | ND | 0% |

| | | | |
|---|---|---|---|
| Notes: ND represents "undetected". | | | |

### 1.3 Cell culturing

MDR1-MDCK II cells were cultivated using α-MEM culture media (a-Minimum Essential Media), with a culture condition of 37±1 °C, 5% CO₂, and saturated relative humidity. Afterwards, the cells were inoculated into a BD Transwell-96-well plate (BD Gentest), with an inoculation density of 2.3×10⁵ cells/cm², then the cells were placed in a carbon dioxide incubator to be incubated for 4-7 days, and then used for a transport experiment.

### 1.4 Transport experiment

The sample for test and digoxin had a donor concentration of 2 µM, and were administered in two directions (direction A-B and direction B-A), each in duplicate. Fenoterol and propranolol each had a test concentration of 2 µM, and were administered in a single direction (direction A-B), each in duplicate.

A solution to be used was placed in a 37±1 °C water bath kettle to be pre-incubated for 30 minutes. The donor solution and the receiver solution were respectively added to corresponding sides of wells of cell plate (to an apical side and a basolateral side, 75 µL and 250 µL of samples were added respectively), and experiment of bidirectional transport was started. After the addition of the samples, the cell plate was placed in a 37±1 °C, 5% CO₂ incubator with saturated relative humidity to be incubated for 150 minutes. Information of sample collection is as shown in Table 4.

**Table 4: Information of Sample Collection**

| Sample Type | Volume of Sample Received in Each Well (µL) | Volume of Stop Solution (µL) | Volume of Transport Buffer (µL) |
|---|---|---|---|
| A-B Donor side | 50 | 250 | 100 |
| A-B Receiver side | 150 | 250 | 0 |
| B-A Donor side | 50 | 250 | 100 |
| B-A Receiver side | 50 | 250 | 100 |
| T0 | 50 | 250 | 100 |

The samples were all centrifuged at 3220 g for 10 minutes after being shaken in a vortex manner. A suitable volume of a supernatant was transferred to a sample analyzing plate. If analysis was not carried out immediately after the plate was sealed, the plate was stored at 2-8 °C. The analysis was carried out through a method of LC/MS/MS.

### 1.5 Test for cell membrane integrity

After the transport experiment was ended, integrity of MDR1-MDCK II cells was tested through Lucifer Yellow Rejection Assay. After a Lucifer Yellow solution was incubated for 30 minutes, a Lucifer Yellow sample was collected, and relative fluorescence intensity (the relative fluorescence unit, RFU) of the Lucifer Yellow in the sample was detected at 425/528 nm (excitation/emission) with a 2^{e} plate reader.

### 1.6 Sample analysis

The sample for test, fenoterol control, propranolol control, and digoxin were analyzed in a semiquantitative manner, and specific values of analytes to a peak area of the internal standard were taken as concentration of the controls.

**Table 5: Permeability of Compounds of the Present Disclosure across MDR1:**

| Compounds | A to B (10⁻⁶ cm/s) | B to A (10⁻⁶ cm/s) |
|---|---|---|
| 360 | 1.61 | 13.32 |
| 0015 | 1.76 | 13.59 |
| 0117 | 3.2 | 11.3 |
| 0227 | 12.53 | 18.03 |
| 0228 | 6.78 | 10.46 |
| 0231 | 3.79 | 17.85 |

### 2. Test for Permeability across Caco2

### 2.1 Preparation of stock solution

A sample for test was dissolved in dimethyl sulfoxide (DMSO) or other suitable solvents, to be prepared into 10 mM stock solution. A suitable internal standard (IS) was dissolved in acetonitrile (ACN) or other organic solvents as a stop solution. Specific information would be described in a research report.

Fenoterol, propranolol, and digoxin acted as low-permeability control, high-permeability control, and P-gp (P-glycoprotein) substrate, respectively, in the present research. The stock solutions of these compounds were prepared with DMSO, stocked at 2-8 °C, and valid for use within 3 months.

### 2.2 Preparation of donor solution and receiver solution

In the present project, a Hank's balanced salt buffer containing 10 mM HEPES was used as a transport buffer. Preparation methods for the donor solution and the receiver solution are as shown in Table 6.

HEPES: 2-[4-(2-Hydroxyethyl)-1-piperazinyl]ethanesulfonic acid, supplier: gibco, article number: 15630-080

Hank's balanced salt buffer: Hank's balanced salt solution, referred to as HBSS for short, purchased from gibco, with an article number 14025-076

**Table 6: Preparation Methods for Donor Solution and Receiver Solution**

| Solution Name | Components | pH | Final DMSO Concentration (v/v) |
|---|---|---|---|
| Apical and basolateral donor solutions | Prepare a control or a sample for test with a concentration of 2 µM from the transport buffer | ND | 0.5% |
| Apical and basolateral receiver solutions | Transport buffer | ND | 0% |

| | | | |
|---|---|---|---|
| Notes: ND represents "undetected". | | | |

### 2.3 Cell culturing

Caco-2 cells were cultivated using MEM culture media (Minimum Essential Media), with a culture condition of 37±1 °C, 5%CO₂, and saturated relative humidity. Afterwards, the cells were inoculated into a BD Transwell-96-well plate, with an inoculation density of 1×10⁵ cells/cm², then the cells were placed in a carbon dioxide incubator to be incubated for 21-28 days, and then used for a transport experiment.

### 2.4 transport experiment

The sample for test and digoxin with donation concentration of 2 µM were administered in two directions (direction A-B and direction B-A), each in duplicate. Fenoterol and propranolol each with test concentration of 2 µM were administered in a single direction (direction A-B), each in duplicate.

A solution to be used was placed in a 37±1 °C water bath kettle to be pre-incubated for 30 minutes. The donor solution and the receiver solution were respectively added to corresponding sides of well of cell plate (to each apical sides and each basolateral sides, 75 µL and 250 µL of samples were added respectively), and experiment of bidirectional transport was started. After addition of the samples, the cell plate was placed in a 37±1 °C, 5% CO₂ incubator with saturated relative humidity to be incubated for 120 minutes. Information of sample collection is as shown in Table 7.

**Table 7: Information of Sample Collection**

| Sample Type | Volume of Sample Received in Each Well (µL) | Volume of Stop Solution (µL) | Volume of Transport Buffer (µL) |
|---|---|---|---|
| A-B Donor side | 50 | 250 | 100 |
| A-B Receiver side | 150 | 250 | 0 |
| B-A Donor side | 50 | 250 | 100 |
| B-A Receiver side | 50 | 250 | 100 |
| T0 | 50 | 250 | 100 |

The samples were all centrifuged at 3220 g for 10 minutes after being shaken in a vortex manner. A suitable volume of a supernatant was transferred to a sample analyzing plate. If analysis was not carried out immediately after the plate was sealed, the plate was stored at 2-8 °C. The analysis was carried out through a method of LC/MS/MS.

### 2.5 Test for cell membrane integrity

After the transport experiment was ended, integrity of Caco-2 cells was tested through Lucifer Yellow Rejection Assay. After a Lucifer Yellow solution was incubated for 30 minutes, a Lucifer Yellow sample was collected, and relative fluorescence intensity (the relative fluorescence unit, RFU) of the Lucifer Yellow in the sample was detected at 425/528 nm (excitation/emission) with a 2^{e} plate reader.

### 2.6 Sample analysis

The sample for test, the fenoterol control, propranolol control, and digoxin were analyzed in a semiquantitative manner, and specific values of analytes to a peak area of the internal standard were taken as concentration of the controls.

**Table 8: Permeability of Compounds of the Present Disclosure across Caco2:**

| Compounds | A to B (10⁻⁶ cm/s) | B to A (10⁶ cm/s) |
|---|---|---|
| 360 | 0.39 | 19.63 |
| 0117 | 2.97 | 12.19 |
| 0231 | 2.58 | 20.13 |

It is concluded that the compounds of the present disclosure have good permeability.

### Test Example 2: Research on Efficacy of Anti-mice Colon Cancer CT26

### 1. Purpose of research

In the present research, colon cancer CT26 models were employed to compare differences between compound 231 and compounds 360 and 117 in antitumor effects.

### 2. Experimental materials

### 2.1 Cells

**Table 9: Basic Information of Cell Line**

| Cell Line Name | CT26.WT (ATCC CRL-2638) |
|---|---|
| Tumor Type | Mouse colorectal cancer cell |
| Conditions of Culture | RPMI 1640 culture medium, 10% fetal bovine serum, 37 °C, 5% CO₂ |
| Culturing Process | Replacing the medium every other day, passage every 2-3 at a ratio of 1:2-1:5 |
| Cell Source | Type Culture Collection, Chinese Academy of Sciences |

### 2.2 Experimental animals

**Table 10: Basic Information of Experimental Animals**

| Animal Breed/Species | BALB/c mice |
|---|---|
| Level | SPF Level |
| Sex | Male |
| Number of Animals | 200 |
| Weeks of Age | 4-5 w |
| Weight | 16-20 g |
| Quarantine/adaptation | At least 1 week of pre-adaptation before start of experiment |
| Feeding Condition | Feed: common feed for growth and reproduction of mice (Beijing Keao Xieli), water: deionized purifed water temperature: 21±2 °C, humidity: 30-70%, lighting: in the alternation of day and night of 12 hr |
| Animal Source | Beijing Vital River Laboratory Animal Technology Co., Ltd; animal certificate number: SCXK (JING) 2012-0001 |

### 2.3 Samples for test

**Table 11: Basic Information of Different IDO Inhibitors**

| Compounds | Molecular Weight | Supplier | Batch Number |
|---|---|---|---|
| 360 | 438.2 | Shanghai Send Pharmaceutical Technology Co., Ltd | SEND20160920-37-3 |
| 117 | 455.3 | | |
| 231 | 419.2 | | |

### 3. Dose design and administration route and frequency

Preliminary experimental research showed that via gavage administration by 100 mg/kg bid, the compound 360 had a tumor inhibitory rate of 30-50% for CT26, substantially reaching a maximum efficacy plateau. Therefore, the dose of the two compounds, compound 360 and compound 117, in the present experiment was set as 100 mg/kg. In order to observe dose-efficacy relevance of 231, a dose of the compound 231 was set as 25, 50, 100, 200 mg/kg in the present experiment. All drugs were administered by gavage, twice a day.

### 4. Selection and preparation of model

According to document reports, mice colon cancer CT26 is a commonly used tumor model for evaluating immunological drugs. The IDO inhibitor compound 360 can effectively inhibit growth of tumors in the above model. Therefore, CT26-bearng BALB/c models were chosen in the present experiment for research on efficacy and tissue distribution.

Tumor cells in logarithmic phase were collected, and re-suspended in a serum-free medium, and adjusted in cell concentration to be 5×10⁵/mL, followed by addition of Matrigel of an equal volume to the cell suspension, such that the cells had a final concentration of 5×10⁵/mL. In a super-clean bench, each mouse was subcutaneously inoculated with 0.2 mL of the tumor cell suspension at shoulder, with an inoculation amount of 1×10⁵ per mouse. When a tumor block grew to be 500-1000 mm³, the tumor block was taken out, cut into pieces with scissors, and subcutaneously inoculated at shoulder at the mouse's back using a subcutaneous embedding implantation puncture needle (with a diameter of 1.2 mm).

### 5. Grouping and administration

The day of tumor inoculation was defined as Day 0, and the inoculated animals were grouped randomly on the day of inoculation. One day after the inoculation (Day 1), the administration was started. The experiment had 7 groups in total, with 12 animals in each group. See details in Table 12 for information on animal grouping and administration.

**Table 12: Grouping of Animals and Administration (CT26 Models) for Efficacy analysis**

| Groups | Compounds | Dose of Administration (mg/kg) | Drug Concentration (mg/mL) | Administration Route and Frequency | Administration Volume (mL/10g) | Quantity of Animals |
|---|---|---|---|---|---|---|
| Control | 20% solutol | -- | -- | gavage, BID | 0.1 | 12 |
| 360 100 mg/kg | 360 | 100 | 10 | gavage, BID | 0.1 | 12 |
| 117 100 mg/kg | 117 | 100 | 10 | gavage, BID | 0.1 | 12 |
| 231 25 mg/kg | 231 | 40 | 4 | gavage, BID | 0.1 | 12 |
| 231 50 mg/kg | 231 | 100 | 10 | gavage, BID | 0.1 | 12 |
| 231 100 mg/kg | 231 | 100 | 10 | gavage, BID | 0.1 | 12 |
| 231 200 mg/kg | 231 | 250 | 25 | gavage, BID | 0.1 | 12 |

### 6. Observation of indexes

It was an experimental cycle when the tumor in the control group grew to have a volume of 3000 mm³. The following indexes were observed during the experiment.
(1) As for tumor growth curve, when the tumor was measurable, a maximum diameter (a) and a minimum diameter (b) of the tumor were measured once every other day, to calculate the tumor volume (a calculation formula is V=1/2×a×b²), and anti-tumor effect of the tested drug was observed dynamically.
(2) As for outcome rate, when the experiment was ended, the outcome rate of each group of animals was observed.
(3) As for animal weight, body weight of the mouse was weighed each time when the tumor diameter was measured or before the administration, and death of the animals was observed once a day.
(4) As for tumor inhibitory rate, when the experiment was ended, the mice were killed by cervical dislocation, the tumor blocks were taken out and weighed, to calculate the tumor inhibitory rate, where tumor inhibitory rate=(mean tumor weight of the control group-mean tumor weight of the treatment group)/mean tumor weight of the control groupx100%), and the tumor blocks were photographed with a digital camera for recording.

### 7. Experimental results

### 7.1 Death and body weight of tumor-bearing mice

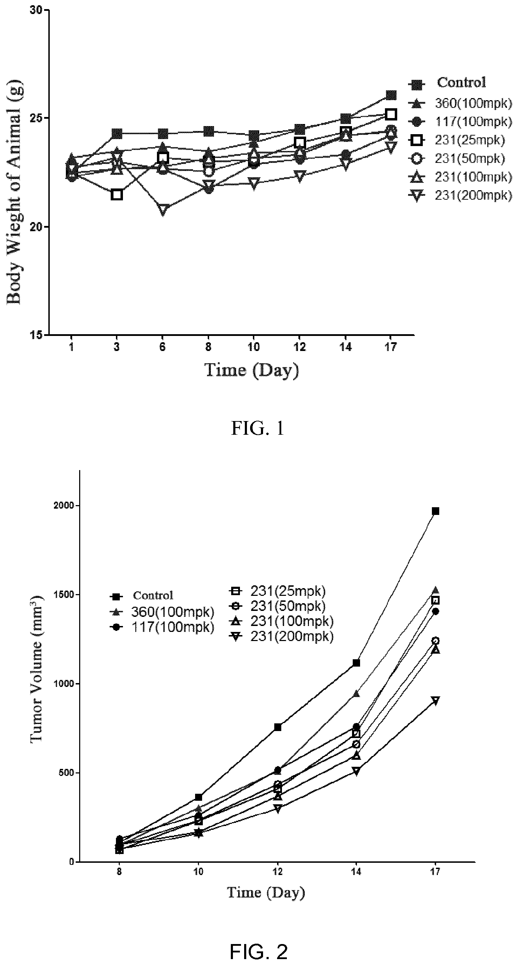

During the whole test, no animal died, surviving animals in each group had an increased body weight compared with those before administration. When the test was ended, the animals in the control group were increased by 16.0% in body weight, while the magnitude of increase in body weight of the animals in each administration group was reduced (4.4%-12.0%). Results were shown in FIG. 1 and Table 12. When the experiment was ended, the animals in group 117 and group 231 50 mg/kg, group 231 100 mg/kg, and group 231 200 mg/kg had remarkably decreased weight compared to those in the control group (*P*<0.05), and the body weight of the animals in group 360 and group 231 25 mg/kg had no significant difference than the control group (*P*>0.05). Therefore, with a relatively high dose of the compound 231, the increase magnitude of the animal weight could be modestly reduced, but the animal weight was still increased.

**Table 12: Effect of Tested Drug on Body Weight of Tumor-bearing Mice (unit: g, χ̅ ± s)**

| | Day1 | Day3 | Day6 | Day8 | Day10 | D12 | D14 | D17 | Rate of Weight Change of D17 vs D1 (%) |
|---|---|---|---|---|---|---|---|---|---|
| Control | 22.5±1.3 | 23.3±1.3 | 24.3±1.1 | 24.4±1.1 | 243±1.1 | 24.5±1.3 | 25.0±1.2 | 26.1±1.6 | 16.0 |
| 360 | 23.2±0.8 | 23.5±0.8 | 23.7±1.0 | 23.5±1.2 | 23.9±1.4 | 24.5±1.2 | 25.0±1.5 | 25.3±1.3 | 9.4 |
| 117 | 22.3±1.2 | 22.7±1.4 | 22.7±1.5 | 21.8±1.7 | 22.9±2.6 | 23.1±1.4 | 23.3±1.4 | 24.2±1.1* | 8.5 |
| 231 25 mg/kg | 22.5±1.1 | 21.5±1.0 | 23.3±1.4 | 23.0±1.3 | 23.1±1.0 | 23.9±1.1 | 24.4±1.3 | 25.2±1.5 | 12.0 |
| 231 50mg/kg | 22.8±1.5 | 23.0±1.5 | 22.7±1.2 | 22.6±1.3 | 23.211.4 | 23.4±1.3 | 24.2±1.6 | 24.5±1.6* | 7,5 |
| 231 100 mg/kg | 22.5±1.3 | 22.7±1.1 | 22.8±1.5 | 23.2±1.4 | 23.4±1.4 | 23.5±1.1 | 24.3±0.9 | 24.4±0.9* | 8.4 |
| 231 200 mg/kg | 22.7±0.9 | 23.2±0.8 | 20.8±1.8 | 21.9±1.9 | 22.0±1.9 | 22.3±1.4 | 22.9±1.5 | 23.7±1.3* | 4.4 |

| | | | | | | | | | |
|---|---|---|---|---|---|---|---|---|---|
| Notes: **P*<0.05, compared with the body weight in the control group. | | | | | | | | | |

### 7.2 Tumor volume

During the test, mean tumor volumes of group 360 and group 117 were always less than that of the control group, but without statistical difference (*P*>0.05). The tumor volume of the group 231 25 mg/kg (low dose), was remarkably less than that of the control group at Day 10 and Day 12 (*P*<0.05); the tumor volumes of 231 groups with a dose equal to or greater than 50 mg/kg were always remarkably less than that of the control group (*P*<0.05) from Day 10 to the end of the test. See details in FIG. 2 and Table 13 for results and statistical analysis.

**Table 13: Mean Tumor Volumes (unit: mm³, χ̅ ± s) of Various Groups of Tumor-bearing Models at Various Time Points**

| | Day8 | Day10 | D12 | D14 | D17 |
|---|---|---|---|---|---|
| Control | 107.5±55.9 | 362.9±160.6 | 755.7±338.2 | 1116.8±455.9 | 1973.9 ±851.1 |
| 360 | 95.2±36.6 | 303.5±233.3 | 512.0±240.2 | 949.2±773.3 | 1531.0±606.0 |
| 117 | 130.1±31.1 | 262.3±131.2 | 517.3±261.9 | 760.9±335.7 | 1410.5±734.0 |
| 231 25 mg/kg | 69.5±21.9 | 229.1±76.7* | 411.7±275.4* | 721.9±575.3 | 1471.2±1269.4 |
| 231 50mg/kg | 94.5±52.5 | 234.6±117.6* | 436.8±204.9* | 661.5±255.6* | 1242.2±500.9* |
| 231100 mg/kg | 97.9±86.9 | 168.2±145.5* | 372.4±308.9* | 598.6±442.3* | 1195.4±779.8* |
| 231 200 mg/kg | 74.2±32.2 | 160.5±114.9* | 300.4±231.8* | 512.2±351.8* | 909.7±544.6* |

| | | | | | |
|---|---|---|---|---|---|
| Notes: **P*<0.05, compared with the control group. | | | | | |

### 7.3 Tumor weight

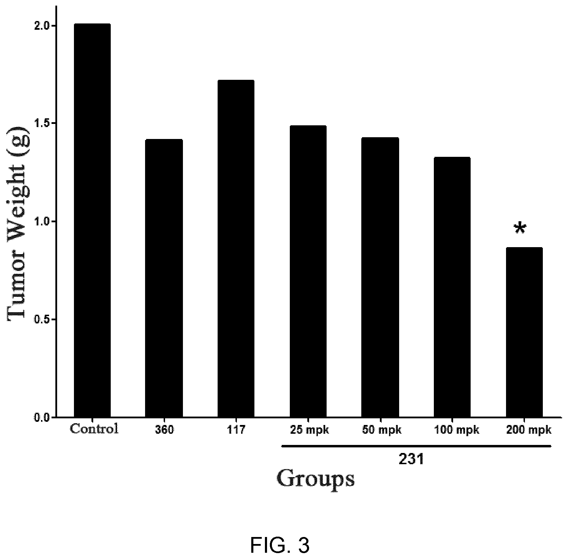

When the experiment was ended, mean tumor weight of group 360 and group 117 was less than that of the control group, but without statistical difference. The tumor inhibitory effect of the compound 231 was shown to be dose-dependent, and with 25, 50, 100 mg/kg, the mean tumor weight was less than that of the control group, but without statistical difference; and with 200 mg/kg of 231, the mean tumor weight was remarkably less than that of the control group (*P*<0.05), with a tumor inhibitory rate of 56.8%. With the same dose of 100 mg/kg, 231 and 360 had equivalent tumor inhibitory effect, superior to 117; for group 117, there was one animal on which no tumor block grew when the test was ended. See details in FIG. 3, accompanying drawings, and Table 14 for results and statistical analysis.

**Table 14: Effect of Tested Drug on Transplanted Tumor Weight (χ̅ ± s)**

| Group | Tumor Weight (g) | Inhibitory Rate for Tumor Weight (%) |
|---|---|---|
| Control | 2.01±0.84 | - |
| 360 | 1.41±0.81 | 29.5 |
| 117 | 1.71±0.76 | 14.4 |
| 23125 mg/kg | 1.49±1.16 | 25.9 |
| 231 50 mg/kg | 1.42±0.56 | 29.2 |
| 231 100 mg/kg | 1.32±1.09 | 34.0 |
| 231 200 mg/kg | 0.87±0.74 * | 56.8 |

| | | |
|---|---|---|
| Notes: **P*<0.05, compared with the control group. | | |

### 8. Conclusion

The purpose of research in the present experiment is assessing phase I metabolic stability of the sample for test in CD-1 mice, SD rats, and human liver microsomes. Under the conditions of the present experiment, the compound 231 could inhibit growth of CT26 transplanted tumor in a dose-dependent manner, with effect superior to the compound 117 and the compound 360, but could modestly decrease the increase in body weight of the tumor-bearing mice.

### Test Example 3: stability experiment for human liver microsomes

The animals and human liver microsomes used in this test system were purchased from BD Gentest, Xenotech, Corning or BioreclamationIVT, and stored in a refrigerator at -80 °C before use.

The sample for test and control were incubated together with the microsomes under a condition of 37 °C for 60 minutes, and a cold acetonitrile solution (or other organic solvents) containing an internal standard substance was added at a designated time point to terminate the reaction. After centrifugation, a resultant supernatant was assayed in a semiquantitative manner through liquid chromatography tandem mass spectrometry (LC/MS/MS). Software Analyst (AB Sciex, Framingham, Massachusetts, USA) was used for processing retention time of analyte and internal standard, and achieving chromatogram collection, and chromatogram integration

An *in vitro* elimination rate constant ke of the sample was obtained by converting a ratio of the sample to a peak area of the internal standard to remaining percentage, to calculate an *in vitro* elimination rate and half-life of the sample for test. Results are as shown in Table 15:

**Table 15**

| Test Compounds | 231 | 227 |
|---|---|---|
| Remaining% (60 min), H/R/M | 81.6/64.8/35.8 | 56.9/21.6/11.0 |
| Intrinsic clearance (mL/min/kg) | <8.6, 23.7, 134.3 | 15.3, 91.9, 274.8 |

Discussion: the compound 231 is metabolized in a relatively steady manner in human and rat liver microsomes, but metabolized quickly in mice. The compound 227 is metabolized in a moderate manner in human liver microsomes, and metabolized quickly in both rats and mice, inferior to the compound 231 in metabolic stability.

### Test Example 4: Experiment for Inhibition on Human Liver Microsome CYP

The research project aimed at evaluating inhibitory ability of a sample for test on human liver microsome cytochrome P450 isozymes (CYP1A2, CYP2C9, CYP2C19, CYP2D6, and CYP3A4) with use of a 5-in-1 probe substrate for CYP isozymes.

Mixed human liver microsomes (HLM) were purchased from Corning Inc. (Steuben, New York, USA) or XenoTech, LLC. (Lenexa, KS, USA) or other suppliers, and stored in a condition below -70 °C before use.

Diluted working solutions of the sample for test in serial concentrations were added to an incubation system containing the human liver microsomes, probe substrate, and accessory factors of a cycling system, and a control containing no sample for test but a solvent was taken as a control for enzymatic activity (100%). Concentration of metabolites generated by the probe substrate in the sample was measured with a method of liquid chromatography-tandem mass spectrometry (LC-MS/MS). Nonlinear regression analysis of mean percentage activity vs concentration of the sample for test was carried out using SigmaPlot (V.11). An IC⁵⁰ value was calculated through a three-parameter or four-parameter inflection logarithmic equation. Test results are in Table 16:

**Table 16**

| Compounds Tested | 231 | 227 |
|---|---|---|
| CYP1A2, 2C9, 2C19, 2D6, 3A4 (IC50, µM) | >50, >50, 36.4, >50, >50 | 13.0, 15.9, 3.61, 26.1, 39.2 |

Discussion: the compound 231 had relatively weak effect in inhibiting all of the five CYP isozymes. The inhibiting effects of the compound 227 on the five CYP isozymes were all superior to the compound 231, and the inhibiting effect on CYP2C19 was in an intermediate degree.

### Test Example 5: Pharmacokinetics Experiment of Mice in vivo

The present experiment aimed at researching status of pharmacokinetics of a sample for test in plasma of male CD-1 mice after single intravenous injection.

### 1. Test method:

Three animals in an intravenous group were intravenously injected with the sample for test by 1 mg/kg, and a formulation was 0.2 mg/mL settled solution of 5% DMSO/95% (10% HP-β-CD). 5 minutes, 15 minutes, 30 minutes, 1 hour, 2 hours, 4 hours, 6 hours, 8 hours, and 24 hours after the administration, blood was collected and prepared into a plasma specimen, with an anticoagulant of EDTA-K₂. Plasma concentration data was obtained by analyzing the specimen through LC-MS/MS.

### 2. Data analysis

The plasma drug concentration data was processed with a non-compartment model using WinNonlin™ Version 6.3 (Pharsight, Mountain View, CA) pharmacokinetics software. Following pharmacokinetics parameters were calculated using a log-linear trapezoidal method: elimination phase half-life (T_{1/2}), apparent volume of distribution (V_{dss}), and clearance rate (CL), mean retention time of drug in body from point 0 to end time point (MRT₀₋ₗₐₛₜ), mean retention time of drug in body from point 0 to infinite time (MRT_{0-inf}), area under a time-plasma concentration curve from point 0 to end time point (AUC₀₋ₗₐₛₜ), area under a time-plasma concentration curve from point 0 to infinite time (AUC_{0-inf}), and initial concentration (C₀). Results are shown in Table 17:

**Table 17**

| | Pharmacokinetics Parameters | 231 | 227 |
|---|---|---|---|
| Mouse PK (IV 1 mg/kg) | Cl (mL/min/kg) | 55.1 | 64.2 |
| | Vd (L/kg) | 4.85 | 2.66 |
| | AUC_{0-last/inf} (nM.h) | 852/881 | 624/628 |
| | Conc. (8h/24h,nM) | 8.16/ND | ND/ND |
| | T_{1/2} (h): | 2.44 | 0.867 |

Discussion: the two compounds both had a medium clearing rate in bodies of mice, the compound 231 had higher AUC than the compound 227, and also had apparent volume of distribution and half-life much greater than those of the compound 227.

## Claims

1. A compound represented by formula (I) or a pharmaceutically acceptable salt thereof, 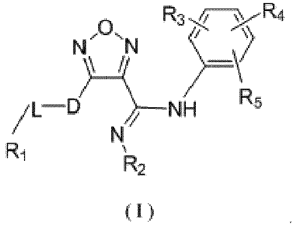 wherein
D is O, S or -S(=O)-;
L is selected from a single bond, or selected from the group consisting of -C₁₋₁₀ alkyl- group, -C₃₋₆ cycloalkyl- group, -C₃₋₆ cycloalkyl-C₁₋₃ alkyl- group, -phenyl- group, 3- to 6-membered heterocycloalkyl- group, and 3- to 6-membered heterocycloalkyl-C₁₋₃ alkyl- group, one or more of which are optionally substituted by 1, 2, or 3 R groups;
R₁ is selected from the group consisting of H, F, Cl, Br, I, OH, and NH₂, or selected from the group consisting of C₁₋₆ alkyl group, C₃₋₆ cycloalkyl group, C₁₋₆ heteroalkyl group, *N,N*-bis(C₁₋₆ alkyl)amino group, 3- to 6-membered heterocycloalkyl group, C₂₋₆ alkenyl group, phenyl group, 5-to 9-membered heteroaryl group, 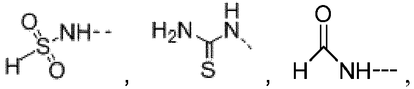 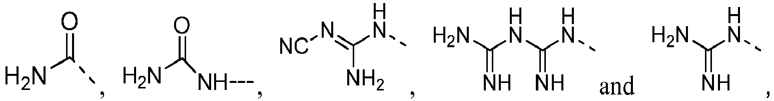 one or more of which are optionally substituted by 1, 2, or 3 R groups;
R₂ is OH or CN;
R₃, R₄, and R₅ are each independently selected from the group consisting of H, F, Cl, Br, I, OH, CN, and NH₂, or selected from the group consisting of C₁₋₆ alkyl group, C₃₋₆ cycloalkyl group, C₁₋₆ heteroalkyl group, *N,N*-bis(C₁₋₆ alkyl)amino group, and 3- to 6-membered heterocycloalkyl group, one or more of which are optionally substituted by 1, 2, or 3 R groups;
R is selected from the group consisting of H, F, Cl, Br, I, OH, CN, and NH₂, or selected from the group consisting of C₁₋₆ alkyl group, C₁₋₆ heteroalkyl group, *N,N*-bis(C₁₋₆ alkyl)amino group, C₃₋₆ cycloalkyl group, C₂₋₆ alkenyl group, phenyl group, and thienyl group, one or more of which are optionally substituted by 1, 2, or 3 R' groups;
R' is selected from the group consisting of F, Cl, Br, I, OH, CN, and NH₂; "hetero" moieties in the -3- to 6-membered heterocycloalkyl, the -3- to 6-membered heterocycloalkyl-C₁₋₃ alkyl-, the C₁₋₆ heteroalkyl, the 3- to 6-membered heterocycloalkyl, or the 5- to 9-membered heteroaryl groups are each independently selected from the group consisting of -C(=O)NH-, -NH-, -S(=O)₂NH-, -S(=O)NH-, N, -O-, -S-, =O, =S, -C(=O)O-, -C(=O)-, -C(=S)-, -S(=O)-, -S(=O)₂-, -NHC(=O)NH-, -NHC(=S)NH-, and -H₂P(=O)-NH- groups; and
in any of above cases, number of heteroatom or heteroatom group is each independently selected from 1, 2 or 3.

2. The compound or the pharmaceutically acceptable salt thereof according to claim 1, wherein R is selected from the group consisting of H, F, Cl, Br, I, OH, CN, and NH₂, or selected from the group consisting of C₁₋₆ alkyl group, C₁₋₆ alkoxy group, C₁₋₆ alkylamino group, *N,N*-bis(C₁₋₆ alkyl)amino group, C₂₋₆ alkenyl group, C₃₋₆ cycloalkyl group, phenyl group, and thienyl group, one or more of which are optionally substituted by 1, 2, or 3 R' groups.

3. The compound or the pharmaceutically acceptable salt thereof according to claim 2, wherein R is selected from the group consisting of H, F, Cl, Br, I, OH, CN, and NH₂, or selected from the group consisting of Me, Et, 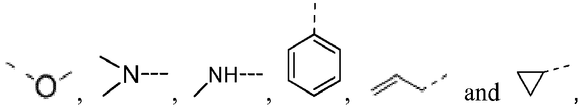 one or more of which are optionally substituted by 1, 2, or 3 R' groups.

4. The compound or the pharmaceutically acceptable salt thereof according to claim 3, wherein R is selected form the group consisting of H, F, Cl, Br, I, OH, CN, NH₂, Me, Et, CF₃, CHF₂, CH₂F,

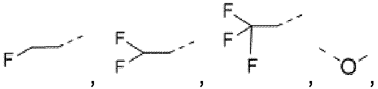

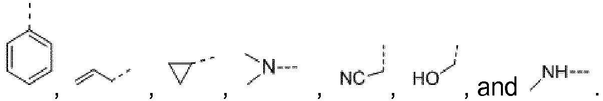

5. The compound or the pharmaceutically acceptable salt thereof according to any one of claims 1∼4, wherein L is selected from a single bond, or selected from the group consisting of -C₁₋₅ alkyl- group, -C₃₋₆ cycloalkyl-group, -C₃₋₆ cycloalkyl-C₁₋₃ alkyl- group, and -3- to 6-membered azacycloalkyl-C₁₋₃ alkyl- group, one or more of which are optionally substituted by 1, 2 or 3 R groups.

6. The compound or the pharmaceutically acceptable salt thereof according to claim 5, wherein L is selected from a single bond, or selected from the group consisting of -CH₂- group, 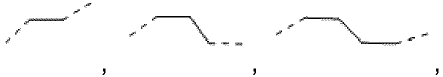 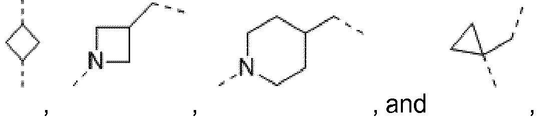 one or more of which are optionally substituted by 1, 2, or 3 R groups.

7. The compound or the pharmaceutically acceptable salt thereof according to claim 6, wherein L is selected from the group consisting of a single bond, -CH₂- group,

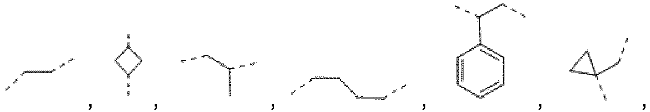

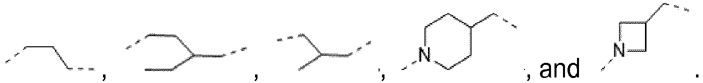

8. The compound or the pharmaceutically acceptable salt thereof according to any one of claims 1∼4, wherein R₁ is selected from the group consisting of H, F, Cl, Br, I, OH, and NH₂, or selected from the group consisting of C₁₋₆ alkyl group, C₁₋₆ alkoxy group, C₁₋₆ alkylthio group, C₁₋₆ alkylamino group, *N,N*'-bis(C₁₋₃ alkyl)amino group, C₃₋₆ cycloalkyl group, tetrahydrofuryl group, oxetanyl group, C₂₋₆ alkenyl group, phenyl group, thienyl group, pyridyl group, imidazolyl group, thiazolyl group, 2-oxo-imidazolidinyl group, NH₂C(=S)-NH- group, C₁₋₆ alkyl-S(=O)- group, C₁₋₆ alkyl-S(=O)₂- group, C₁₋₆ alkoxy-C(=O)-NH- group, NH₂-S(=O)-NH-group, -NH₂-C(=O)- group, NH₂-C(=O)-NH- group, H-C(=O)-NH- group, H-S(=O)₂-NH- group, 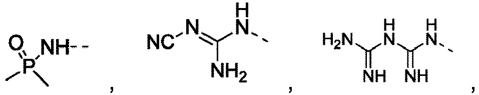 and 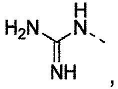 one or more of which are optionally substituted by 1, 2 or 3 R groups.

9. The compound or the pharmaceutically acceptable salt thereof according to claim 8, wherein R₁ is selected from the group consisting of H, F, Cl, Br, I, OH, and NH₂, or selected from the group consisting of Me, Et, BOC-NH-, CH₂ = CH-, 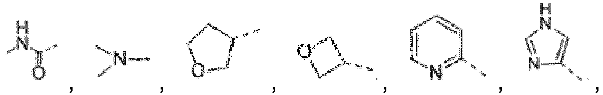 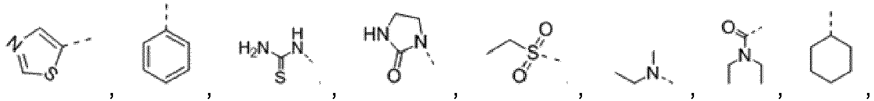 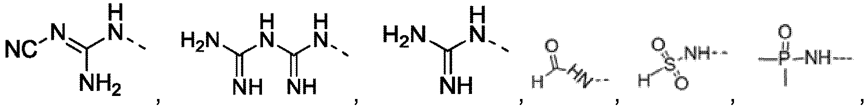 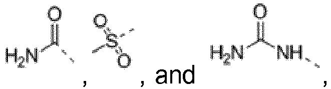 one or more of which are optionally substituted by 1, 2, or 3 R groups.

10. The compound or the pharmaceutically acceptable salt thereof according to claim 9, wherein R₁ is selected from the group consisting of H, F, Cl, Br, I, OH, NH₂, 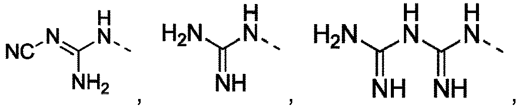 Me, CF₃, BOC-NH-, CH₂=CH-,

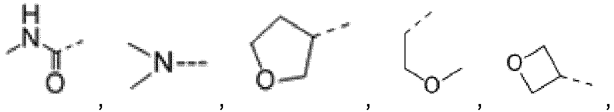

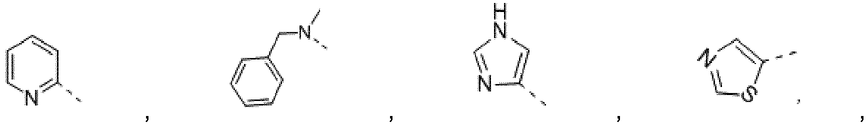

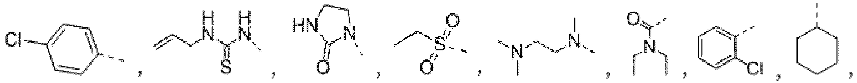

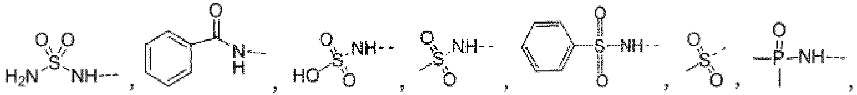

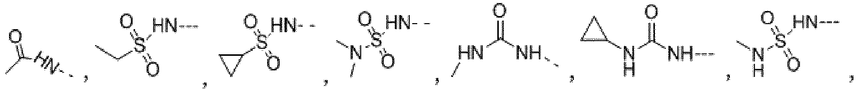

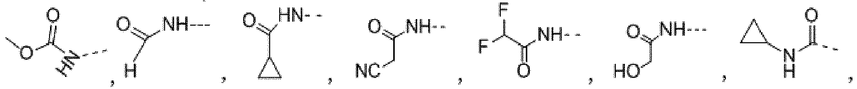

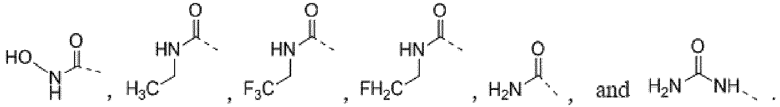

11. The compound or the pharmaceutically acceptable salt thereof according to claim 7 or 10, wherein a structural unit R₁-L- is selected from the group consisting of H, NH₂, 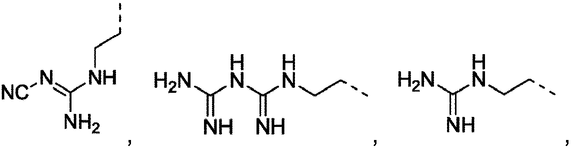 Me, BOC-NH-, 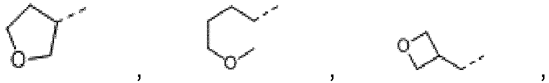 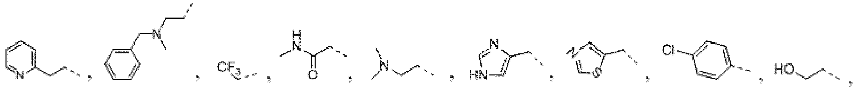 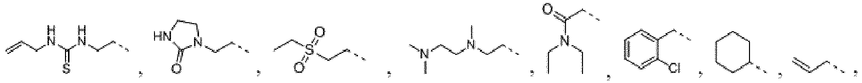 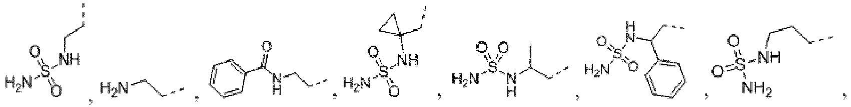 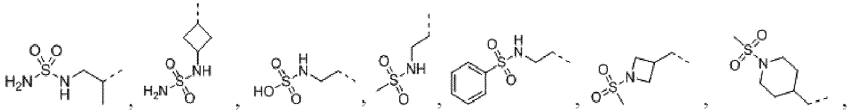 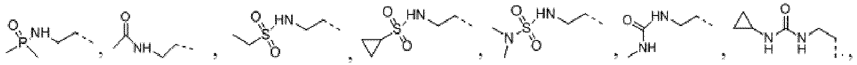 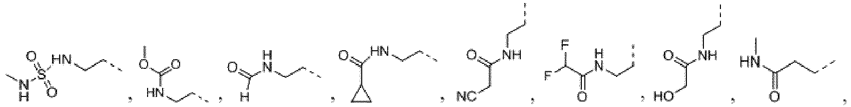 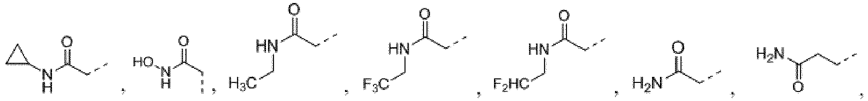 and

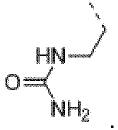

12. The compound or the pharmaceutically acceptable salt thereof according to any one of claims 1-4, wherein R₃, R₄, and R₅ are each independently selected from the group consisting of H, F, Cl, Br, I, OH, CN, and NH₂, or selected from the group consisting of Me, Et, C₃₋₆ cycloalkyl, and C₁₋₃ alkoxy, one or more of which are optionally substituted by 1, 2, or 3 R groups.

13. The compound or the pharmaceutically acceptable salt thereof according to claim 12, wherein R₃, R₄, and R₅ are each independently selected from the group consisting of H, F, Cl, Br, I, OH, CN, and NH₂, or selected from the group consisting of Me, Et, 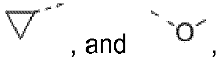 one or more of which are optionally substituted by 1, 2, or 3 R groups:.

14. The compound or the pharmaceutically acceptable salt thereof according to claim 13, wherein R₃, R₄, and R₅ are each independently selected from the group consisting of H, F, Cl, Br, I, CN, CH₂F, CHF₂, CF₃, 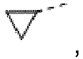 and

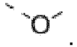

15. The compound or the pharmaceutically acceptable salt thereof according to claim 1, wherein a structural unit 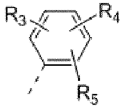 is selected from

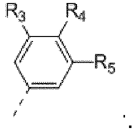

16. The compound or the pharmaceutically acceptable salt thereof according to claim 14 or 15, wherein the structural unit 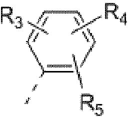 is selected from the group consisting of

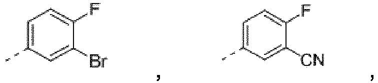

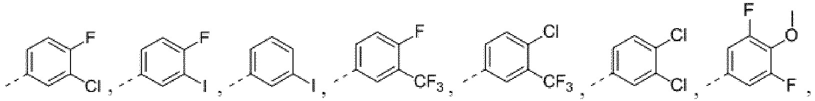

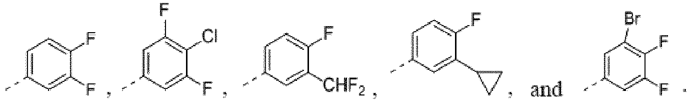

17. The compound or the pharmaceutically acceptable salt thereof according to any one of claims 1∼4 and 12∼16, represented by: 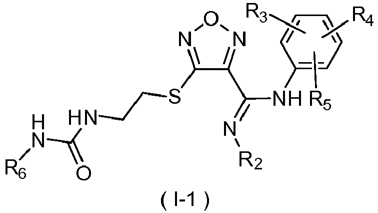 wherein
R₂, R₃, R₄, and R₅ are as defined in claims 1∼4 and 12∼16;
R₆ is selected from H, or selected from the group consisting of C₁₋₃ alkyl group and C₃₋₆ cycloalkyl group, one or more of which are optionally substituted by 1, 2, or 3 R' groups; and provided that
R₆ is not Me.

18. The compound or the pharmaceutically acceptable salt thereof according to claim 17, wherein R₆ is selected from the group consisting of H, CF₃, Et,

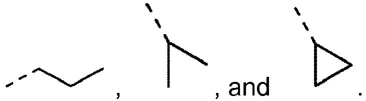

19. The compound or the pharmaceutically acceptable salt thereof according to claim 1, selected from the group consisting of 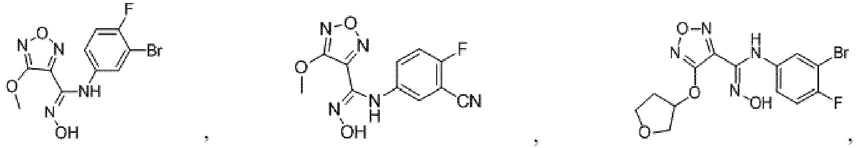 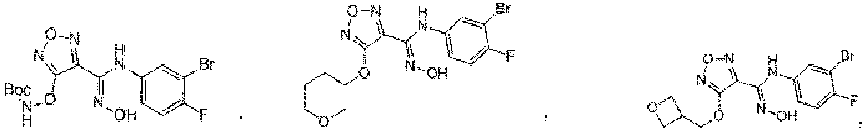 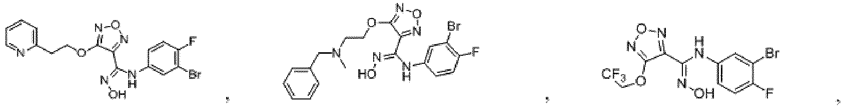 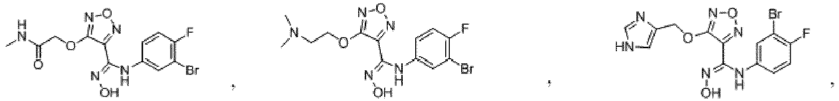 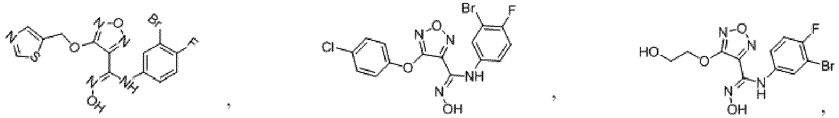 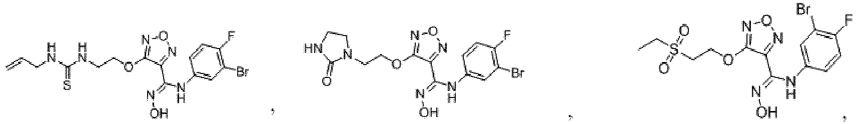 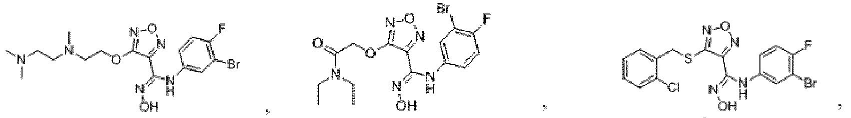 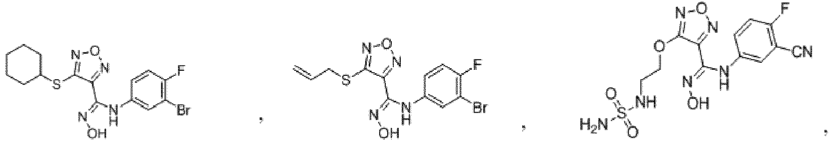 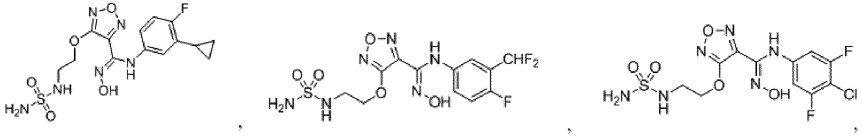 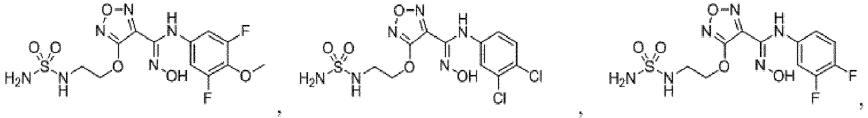 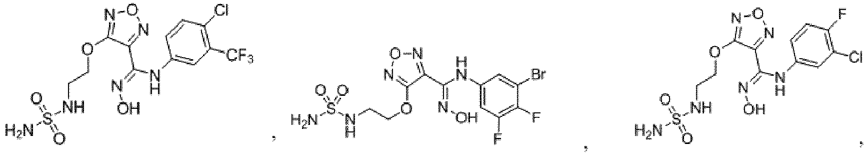 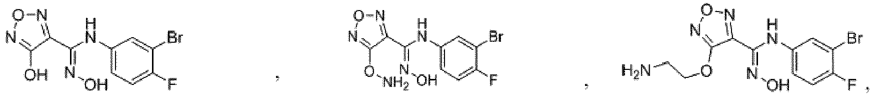 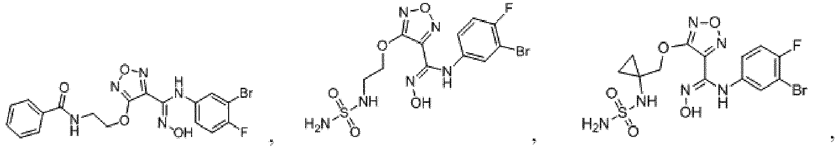 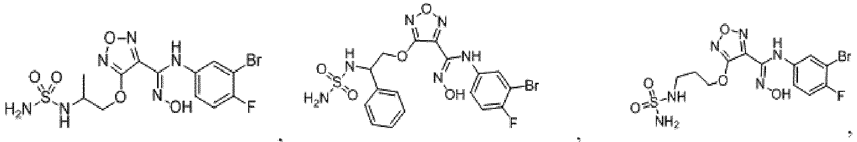 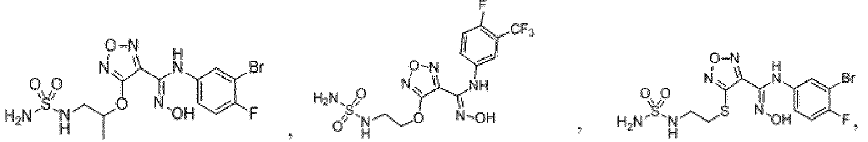 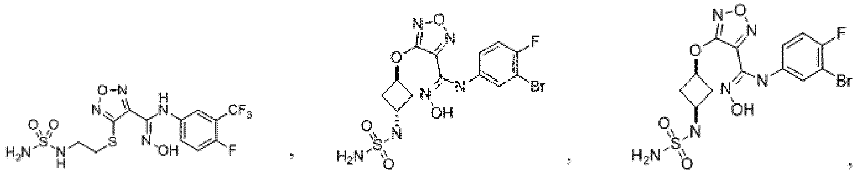 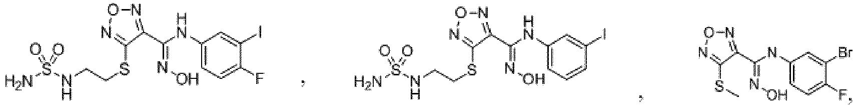 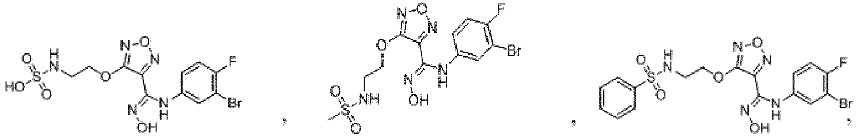 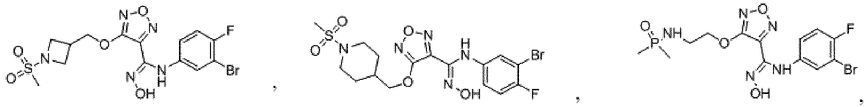 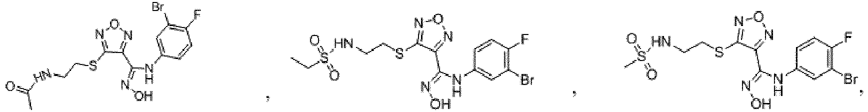 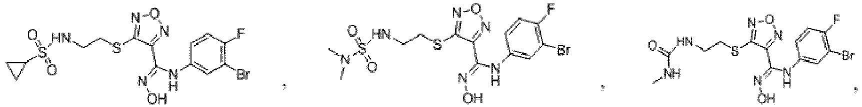 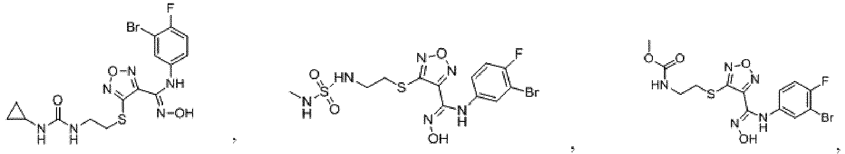 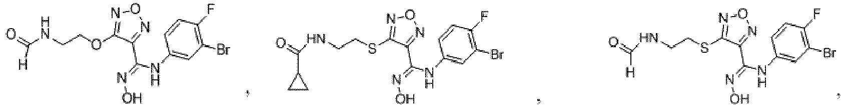 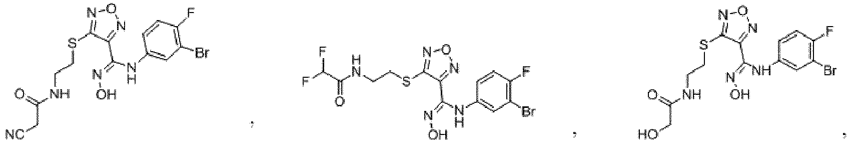 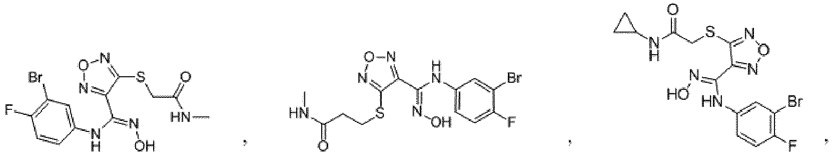 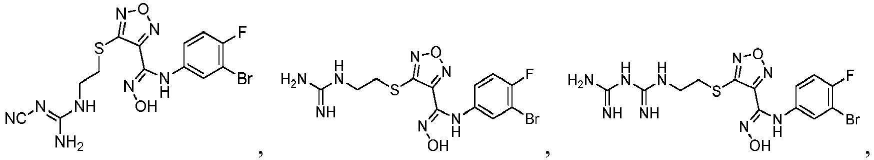 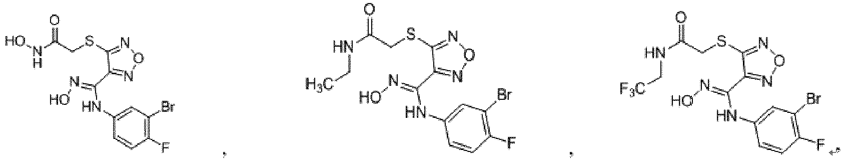 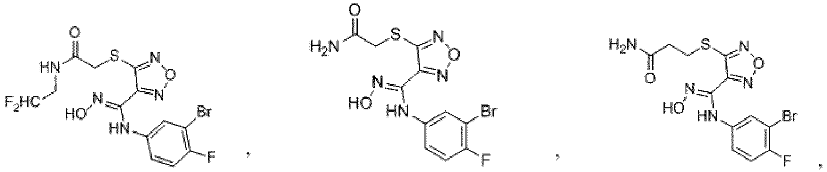 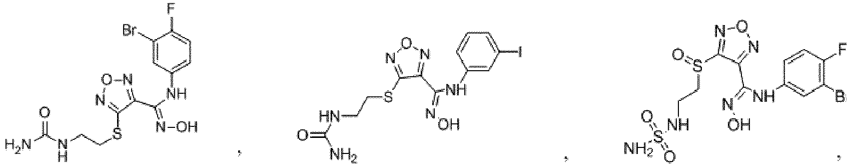 and

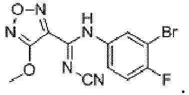

20. A pharmaceutical composition, comprising a therapeutically effective amount of the compound or the pharmaceutically acceptable salt thereof according to any one of claims 1∼19 as an active ingredient and a pharmaceutical acceptable carrier.

21. Use of the compound or the pharmaceutically acceptable salt thereof according to any one of claims 1∼19 or the composition according to claim 20 in preparation of a medicament for treatment of IDO1-related diseases.
